(19) **Europäisches Patentamt**
**European Patent Office**
**Office européen des brevets**

(11) **EP 3 779 451 A1**

(12) **EUROPEAN PATENT APPLICATION**
published in accordance with Art. 153(4) EPC

(43) Date of publication:
**17.02.2021 Bulletin 2021/07**

(21) Application number: **19774998.9**

(22) Date of filing: **15.03.2019**

(51) Int Cl.:
*G01N 33/68* (2006.01)    *A61K 31/4745* (2006.01)
*A61K 31/513* (2006.01)    *A61K 31/519* (2006.01)
*A61K 39/395* (2006.01)    *A61K 45/00* (2006.01)
*A61P 35/00* (2006.01)    *A61P 43/00* (2006.01)
*G01N 33/15* (2006.01)    *G01N 33/50* (2006.01)

(86) International application number:
**PCT/JP2019/010963**

(87) International publication number:
**WO 2019/188446 (03.10.2019 Gazette 2019/40)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **29.03.2018 JP 2018064446**
**28.09.2018 JP 2018185152**

(71) Applicants:
• **Keio University**
**Tokyo 108-8345 (JP)**
• **Kabushiki Kaisha Yakult Honsha**
**Tokyo 105-8660 (JP)**

(72) Inventors:
• **SUGIMOTO, Shinji**
**Tokyo 160-8582 (JP)**
• **SASAKI, Nobunari**
**Tokyo 160-8582 (JP)**
• **TANIGAWARA, Yusuke**
**Tokyo 160-8582 (JP)**

(74) Representative: **Schiener, Jens**
**Wächtershäuser & Hartz**
**Patentanwaltspartnerschaft mbB**
**Weinstraße 8**
**80333 München (DE)**

(54) **MARKER FOR DETERMINING SENSITIVITY OF IRINOTECAN-CONTAINING ANTI-CANCER AGENT THERAPY**

(57)    Provided is a novel marker for determining anti-cancer agent sensitivity. The present invention provides a marker for determining sensitivity to an anti-cancer agent, the anti-cancer agent including irinotecan or SN-38 or a salt thereof, fluorouracil or a salt thereof, and levofolinate or a salt thereof, the marker comprising one or more molecules selected from the group consisting of 5A4CR, ALA, ASP, CYS, CSSG, GLC3P, HIS, ILE, LEU, LYS, METSF, N6TLY, N6ALY, OCTA, SER, TUCA, THR, TRP, TYR and VAL. The present invention also provides a marker for determining sensitivity to an anti-cancer agent, the anti-cancer agent including irinotecan or SN-38 or a salt thereof, fluorouracil or a salt thereof, and levofolinate or a salt thereof, the marker comprising one or more molecules selected from the group consisting of 3IND, 4OVAL, 5A4CR, ALA, BENZA, CREAT, CSSG, DECNA, GABB, GLC3P, HYPTA, LYS, METSF, N8ASR, QUINA, SARCO, TMNO and VAL.

EP 3 779 451 A1

**Description**

Technical Field

The present invention relates to a marker for determining sensitivity to an anti-cancer agent which is used to determine whether or not a cancer of a patient of interest has a therapeutic response to the anti-cancer agent, and to use of the marker.

Background Art

Anti-cancer agents include various types of agents such as an alkylating agent, a platinum agent, an antimetabolite, an anti-cancer antibiotic, and an anti-cancer plant alkaloid. These anti-cancer agents are effective for some types of cancers but not effective for other types of cancers. However, it is known that even when an anti-cancer agent has been confirmed be effective for a certain type of cancer, the anti-cancer agent is effective for some patients and not effective for other patients, leading to interindividual differences. Whether or not a cancer of a patient has a response to an anti-cancer agent is designated as sensitivity to the anti-cancer agent.

For advanced/recurrent colorectal cancer, fluorouracil (5-FU)/levofolinate (LV) therapy, which had been employed until early 1990's, provided a survival of 10 to 12 months. With the introduction of oxaliplatin (L-OHP) and irinotecan hydrochloride (CPT-11), a survival period was increased to 21.5 months, which is almost twice the survival period of the former, by appropriately employing FOLFOX therapy, a combination of 5-FU/LV therapy and L-OHP, and FOLFIRI therapy, a combination of 5-FU/LV therapy and CPT-11. Further, it has been known that FOLFOX therapy followed by FOLFIRI therapy and FOLFIRI therapy followed by FOLFOX therapy achieve a similar survival period (Non-Patent Literature 1). It should be noted that since CPT-11 is activated by carboxyl esterase to thereby be converted to SN-38 in vivo, which has an antitumor activity 100 to several thousand higher than CPT-11, it can be said that SN-38 is an active metabolite of CPT-11.

However, even so, the response rate of FOLFOX therapy or FOLFIRI therapy against advanced/recurrent colorectal cancer in chemotherapy-untreated cases is about 55% and the response rate of the secondary therapy is 6 to 21%. In other words, the primary therapy is effective for only half of patients who have received the therapy and the secondary therapy is effective for only one in five to ten patients. Also, use of oxaliplatin may cause neutropenia as well as severe diarrhea, resulting in fatal outcome in some cases. If there is a biomarker which can predict, before starting the therapy, which patients can expect to have an efficacy and which patients cannot, and can diagnose therapeutic response in an early stage of the therapy, a chemotherapy treatment with high effectiveness and high safety can be realized.

Furthermore, since a therapy schedule of cancer chemotherapy generally takes a long period of time, monitoring over time of sensitivity to an anti-cancer agent during the therapy enables determination of whether the therapy should be continued. Not only this leads to reduction of patient's burden or adverse effects, but this is also considered useful even from the viewpoint of medical economics. In order to realize a "personalized therapy" which predicts therapeutic response in individual patients and diagnoses in an early stage to select an appropriate medicament or therapeutic regimen, it is urgently needed to establish a biomarker which enables prediction of the efficacy of an anti-cancer agent such as CPT-11 or an early diagnosis of therapeutic response, is urgent.

From such a point of view, the inventors of the present invention conducted a search for markers for determining sensitivity to an anti-cancer agent by exposing a drug to a plurality of human cancer cell lines having different drug sensitivity or cancer-bearing mice transplanted with the human cancer cell lines, comprehensively analyzing intracellular metabolic variation therein after the drug exposure using capillary electrophoresis time-of-flight mass spectrometer (CE-TOF MS), and conducting a comparative analysis of the results with drug sensitivity, and reported the obtained several markers (Patent Literatures 1 to 4). However, these markers have not yet been put into practical use. Furthermore, an antibody medicine such as a VEGF inhibitor, e.g., bevacizumab, or an EGFR receptor inhibitor, e.g., cetuximab, has been recently introduced into the treatment of colorectal cancer. However, single use of the antibody medicine in the treatment of colorectal cancer is extremely rare and the antibody medicine is generally used in combination with FOLFOX therapy or FOLFIRI therapy. Therefore, there is an increasing importance of a biomarker which enables to predict an effect of FOLFIRI therapy or to diagnose a therapeutic response in an early stage of the therapy.

Citation List

Patent Literature

Patent Literature 1: WO 2009/096189
Patent Literature 2: WO 2011/052750
Patent Literature 3: WO 2012/127984
Patent Literature 4: WO 2013/125675

Non-Patent Literature

Non-Patent Literature 1: Tournigand C. et al., FOLFIRI Followed by FOLFOX6 or the Reverse Sequence in Advanced Colorectal Cancer: A Randomized GERCOR Study, JCO 2004 22(2) : 229-37.

Summary of the Invention

Problems to be Solved by the Invention

An object of the present invention is to provide a marker for determining sensitivity to an anti-cancer agent, which can determine a therapeutic response of each individual patient, and a novel cancer therapeutic approach utilizing the marker.

Means for Solving the Problems

Accordingly, the present inventors comprehensively measured blood metabolites using CE-Q-TOF MS and CE-TOF MS before the start of FOLFIRI therapy combined with bevacizumab, after implementation of one cycle, or after implementation of two cycles targeting blood specimens of colorectal cancer patients refractory or intolerant to FOLFOX therapy, and conducted logistic analysis by using the obtained concentrations of the metabolites as explanatory variables and their clinical effects as objective variables. As a result, the present inventors found that the concentrations of particular substances differed between patients who exhibited evident tumor shrinkage (patients who exhibited partial response: PR) and patients who exhibited no evident tumor shrinkage (patients who exhibited stable disease: SD or progressive disease: PD) for mFOLFIRI therapy combined with bevacizumab, or between patients who had exacerbations without responding to mFOLFIRI therapy combined with bevacizumab (patients who exhibited PD) and patients who exhibited no evident tumor enlargement (patients who exhibited PR or SD). The present inventors also conducted proportional hazard model analysis on the blood metabolites before the start of treatment of the cancer patients and their overall survivals, the blood metabolites after implementation of one cycle and residual overall survivals, or the blood metabolites after implementation of two cycles and residual overall survivals. As a result, the present inventors found that the higher the blood concentrations of particular substances, the longer the survival period while the higher the blood concentrations of other particular substances, the shorter the survival period. On the basis of these findings, the present invention has been completed.
Specifically, the present invention provides aspects of the following [1] to [51].

[1] A marker for determining sensitivity to an anti-cancer agent, the anti-cancer agent including irinotecan or SN-38 or a salt thereof, fluorouracil or a salt thereof, and levofolinate or a salt thereof, the marker comprising one or more molecules selected from the group consisting of 5-aminoimidazole-4-carboxamide ribotide (5A4CR), alanine (ALA), aspartic acid (ASP), cysteine (CYS), cysteine-glutathione disulphide (CSSG), glycerol-3-phosphate (GLC3P), histidine (HIS), isoleucine (ILE), leucine (LEU), lysine (LYS), methionine sulfoxide (METSF), N6,N6,N6-trimethyllysine (N6TLY), N6-acetyllysine (N6ALY), octanoic acid (OCTA), serine (SER), taurocholic acid (TUCA), threonine (THR), tryptophan (TRP), tyrosine (TYR) and valine (VAL).
[2] The marker for determining anti-cancer agent sensitivity according to [1], wherein the anti-cancer agent further includes an anti-angiogenic drug.
[3] The marker for determining anti-cancer agent sensitivity according to [2], wherein the anti-angiogenic drug is bevacizumab.
[4] A method for determining sensitivity to an anti-cancer agent, the anti-cancer agent including irinotecan or SN-38 or a salt thereof, fluorouracil or a salt thereof, and levofolinate or a salt thereof, the method comprising measuring an amount of one or more molecules selected from the group consisting of 5A4CR, ALA, ASP, CYS, CSSG, GLC3P, HIS, ILE, LEU, LYS, METSF, N6TLY, N6ALY, OCTA, SER, TUCA, THR, TRP, TYR and VAL in a biological sample derived from a cancer patient.
[5] The determination method according to [4], further comprising determining the sensitivity of the cancer patient to the anti-cancer agent by comparing a measurement result with a control level.
[6] The determination method according to [4], wherein the amount of one or more molecules selected from the group consisting of ALA, CYS, CSSG, HIS, ILE, LEU, LYS, METSF, N6TLY, SER, THR, TRP, TYR and VAL is measured, and the method further comprises determining whether or not the cancer patient has PR after treatment with the anti-cancer agent, by comparing a measurement result with a cut-off value of PR, wherein the cut-off value is $9.957 \leq$ for ALA, $2.444 \times 10^{-1} \leq$ for CYS, $1.430 \times 10^{-2} \leq$ for CSSG, $2.2409 \leq$ for HIS, $2.997 \leq$ for ILE, $7.437 \leq$ for LEU, $4.945 \leq$ for LYS, $6.658 \times 10^{-2} \leq$ for METSF, $8.401 \times 10^{-2} \leq$ for N6TLY, $2.200 \leq$ for SER, $2.753 \leq$ for THR, $2.165 \leq$ for TRP, $2.084 \leq$ for TYR, and $8.317 \leq$ for VAL.

[7] The determination method according to [4], wherein the amount of one or more molecules selected from the group consisting of 5A4CR, ALA, ASP, CSSG, GLC3P, HIS, ILE, LEU, N6TLY, N6ALY, OCTA, TUCA and THR is measured, and the method further comprises determining whether or not the cancer patient has PD after treatment with the anti-cancer agent, by comparing a measurement result with a cut-off value of PD, wherein the cut-off value is $1.421 \times 10^{-2} \leq$ for 5A4CR, $< 6.494$ for ALA, $0.1433 \leq$ for ASP, $< 8.630 \times 10^{-3}$ for CSSG, $< 6.009 \times 10^{-3}$ for GLC3P, $< 2.366$ for HIS, $< 2.748$ for ILE, $< 6.413$ for LEU, $< 8.030 \times 10^{-2}$ for N6TLY, $2.915 \times 10^{-2} \leq$ for N6ALY, $< 6.767 \times 10^{-2}$ for OCTA, $2.380 \times 10^{-3} \leq$ for TUCA, and $< 2.137$ for THR.

[8] The determination method according to [4], further comprising determining whether or not the cancer patient has PR after treatment with the anti-cancer agent, by calculating a probability (p) of PR according to the following expression (1):

$$p = \frac{1}{1+e^{(4.2504-(ALA)-(CSSG))}} \qquad (1)$$

wherein ALA represents 2.5043 when a measurement result about ALA is equal to or more than a cut-off value, and represents 0 when the measurement result is less than the cut-off value; CSSG represents 2.4626 when a measurement result about CSSG is equal to or more than a cut-off value, and represents 0 when the measurement result is less than the cut-off value; and the cut-off value is 9.957 for ALA and $1.430 \times 10^{-2}$ for CSSG.

[9] The determination method according to [4], further comprising determining whether or not the cancer patient has PD after treatment with the anti-cancer agent, by calculating a probability (p) of PD according to the following expression (2):

$$p = \frac{1}{1+e^{(0.8105+(ASP)+(HIS)+(N6ALY)+(TUCA))}} \qquad (2)$$

wherein ASP represents -1.0820 when a measurement result about ASP is equal to or more than a cut-off value, and represents 1.0820 when the measurement result is less than the cut-off value; HIS represents 1.7717 when a measurement result about HIS is equal to or more than a cut-off value, and represents -1.7717 when the measurement result is less than the cut-off value; N6ALY represents -1.5499 when a measurement result about N6ALY is equal to or more than a cut-off value, and represents 1.5499 when the measurement result is less than the cut-off value; TUCA represents -0.7905 when a measurement result about TUCA is equal to or more than a cut-off value, and represents 0.7905 when the measurement result is less than the cut-off value; and the cut-off value is 0.1433 for ASP, 2.366 for HIS, $2.915 \times 10^{-2}$ for N6ALY, and $2.380 \times 10^{-3}$ for TUCA.

[10] The determination method according to any of [4] to [9], wherein the biological sample is a biological sample derived from a cancer patient to whom the anti-cancer agent has been administered.

[11] The determination method according to any of [4] to [10], wherein the anti-cancer agent further includes an anti-angiogenic drug.

[12] The determination method according to [11], wherein the anti-angiogenic drug is bevacizumab.

[13] A method for determining a total tumor size of a cancer patient, comprising measuring an amount of CSSG in a biological sample derived from the cancer patient.

[14] A marker for predicting prognosis for treatment with an anti-cancer agent, the anti-cancer agent including irinotecan or SN-38 or a salt thereof, fluorouracil or a salt thereof, and levofolinate or a salt thereof, the marker comprising one or more molecules selected from the group consisting of 3-indoxylsulfuric acid (3IND), ALA, ASP, citrulline (CITR), creatine (CREAT), CSSG, gamma-aminobutyric acid (GABA), guanidoacetic acid (GUAA), HIS, hydroxyproline (HYPRO), METSF, N6TLY, N8-acetylspermidine (N8ASR) and SER.

[15] The marker for predicting prognosis according to [14], wherein the anti-cancer agent further includes an anti-angiogenic drug.

[16] The marker for predicting prognosis according to [15], wherein the anti-angiogenic drug is bevacizumab.

[17] A method for predicting prognosis for treatment with an anti-cancer agent, the anti-cancer agent including irinotecan or SN-38 or a salt thereof, fluorouracil or a salt thereof, and levofolinate or a salt thereof, the method comprising measuring an amount of one or more molecules selected from the group consisting of 3IND, ALA, ASP, CITR, CREAT, CSSG, GABA, GUAA, HIS, HYPRO, METSF, N6TLY, N8ASR and SER in a biological sample derived from a cancer patient.

[18] The method for predicting prognosis according to [17], wherein the anti-cancer agent further includes an anti-angiogenic drug.

[19] The method for predicting prognosis according to [18], wherein the anti-angiogenic drug is bevacizumab.

[20] A kit for carrying out a determination method according to any of [4] to [13], the kit comprising a protocol for measuring an amount of one or more molecules selected from the group consisting of 5A4CR, ALA, ASP, CYS, CSSG, GLC3P, HIS, ILE, LEU, LYS, METSF, N6TLY, N6ALY, OCTA, SER, TUCA, THR, TRP, TYR and VAL in a biological sample derived from a cancer patient.

[21] A kit for carrying out a method for predicting prognosis according to any of [17] to [19], the kit comprising a protocol for measuring an amount of one or more molecules selected from the group consisting of 3IND, ALA, ASP, CITR, CREAT, CSSG, GABA, GUAA, HIS, HYPRO, METSF, N6TLY, N8ASR and SER in a biological sample derived from a cancer patient.

[22] A screening method for an anti-cancer agent sensitivity-enhancing agent, the method comprising employing, as an index, expression variation of one or more molecules selected from the group consisting of 5A4CR, ALA, ASP, CYS, CSSG, GLC3P, HIS, ILE, LEU, LYS, METSF, N6TLY, N6ALY, OCTA, SER, TUCA, THR, TRP, TYR, VAL, 3IND, CITR, CREAT, GABA, GUAA, HYPRO and N8ASR in a biological sample derived from a cancer cell line or a cancer-bearing animal in the presence of an anti-cancer agent including irinotecan or SN-38 or a salt thereof, fluorouracil or a salt thereof, and levofolinate or a salt thereof.

[23] The screening method according to [22], wherein the anti-cancer agent further includes an anti-angiogenic drug.

[24] The screening method according to [23], wherein the anti-angiogenic drug is bevacizumab.

[25] A sensitivity-enhancing agent for an anti-cancer agent including irinotecan or SN-38 or a salt thereof, fluorouracil or a salt thereof, and levofolinate or a salt thereof, which is obtained by a method according to any of [22] to [24] .

[26] A composition for cancer treatment, the composition comprising a sensitivity-enhancing agent according to [25] in combination with an anti-cancer agent including irinotecan or SN-38 or a salt thereof, fluorouracil or a salt thereof, and levofolinate or a salt thereof.

[27] The composition for cancer treatment according to [26], wherein the anti-cancer agent further includes an anti-angiogenic drug.

[28] The composition for cancer treatment according to [27], wherein the anti-angiogenic drug is bevacizumab.

[29] A marker for determining sensitivity to an anti-cancer agent, the anti-cancer agent including irinotecan or SN-38 or a salt thereof, fluorouracil or a salt thereof, and levofolinate or a salt thereof, the marker comprising one or more molecules selected from the group consisting of 3IND, 4-oxavaleric acid (4OVAL), 5A4CR, ALA, benzoic acid (BENZA), CREAT, CSSG, decanoic acid (DECNA), gamma-butyrobetaine (GABB), GLC3P, hypotaurine (HYPTA), LYS, METSF, N8ASR, quinic acid (QUINA), sarcosine (SARCO), trimethylamine N-oxide (TMNO) and VAL.

[30] The marker for determining anti-cancer agent sensitivity according to [29], wherein the anti-cancer agent further includes an anti-angiogenic drug.

[31] The marker for determining anti-cancer agent sensitivity according to [30], wherein the anti-angiogenic drug is bevacizumab.

[32] A method for determining sensitivity to an anti-cancer agent, the anti-cancer agent including irinotecan or SN-38 or a salt thereof, fluorouracil or a salt thereof, and levofolinate or a salt thereof, the method comprising measuring an amount of one or more molecules selected from the group consisting of 3IND, 4OVAL, 5A4CR, ALA, BENZA, CREAT, CSSG, DECNA, GABB, GLC3P, HYPTA, LYS, METSF, N8ASR, QUINA, SARCO, TMNO and VAL in a biological sample derived from a cancer patient who has received at least one cycle of treatment with the anti-cancer agent including irinotecan or SN-38 or a salt thereof, fluorouracil or a salt thereof, and levofolinate or a salt thereof.

[33] The determination method according to [32], further comprising determining the sensitivity of the cancer patient to the anti-cancer agent by comparing a measurement result with a control level.

[34] The determination method according to [32], wherein the cancer patient is a patient who has received one cycle of treatment with the anti-cancer agent, the amount of one or more molecules selected from the group consisting of CREAT, CSSG, METSF, QUINA and VAL is measured, and the method further comprises determining whether or not the cancer patient has PR after treatment with the anti-cancer agent, by comparing a measurement result with a cut-off value of PR, wherein the cut-off value is $1.2163 \leq$ for CREAT, $1.965 \times 10^{-2} \leq$ for CSSG, $5.060 \times 10^{-2} \leq$ for METSF, $< 1.150 \times 10^{-2}$ for QUINA, and $7.6718 \leq$ for VAL.

[35] The determination method according to [32], wherein the cancer patient is a patient who has received one cycle of treatment with the anti-cancer agent, the amount of one or more molecules selected from the group consisting of 5A4CR, CSSG, DECNA, GLC3P, HYPTA and N8ASR is measured, and the method further comprises determining whether or not the cancer patient has PD after treatment with the anti-cancer agent, by comparing a measurement result with a cut-off value of PD, wherein the cut-off value is $1.413 \times 10^{-2} \leq$ for 5A4CR, $< 1.030 \times 10^{-2}$ for CSSG, $< 1.080 \times 10^{-1}$ for DECNA, $< 4.586 \times 10^{-1}$ for GLC3P, $< 1.240 \times 10^{-2}$ for HYPTA, and $1.122 \times 10^{-2} \leq$ for N8ASR.

[36] The determination method according to [32], wherein the cancer patient is a patient who has received two cycles of treatment with the anti-cancer agent, the amount of one or more molecules selected from the group consisting of 4OVAL, ALA, BENZA, CREAT, CSSG, LYS and SARCO is measured, and the method further comprises determining whether or not the cancer patient has PR after treatment with the anti-cancer agent, by comparing a measurement result with a cut-off value of PR, wherein the cut-off value is $2.949 \times 10^{-2} \leq$ for 4OVAL, $7.9605 \leq$ for ALA, $1.367 \times 10^{-1} \leq$ for BENZA, $6.609 \times 10^{-1} \leq$ for CREAT, $1.233 \times 10^{-2} \leq$ for CSSG, $4.9765 \leq$ for LYS, and $4.548 \times 10^{-2} \leq$ for SARCO.

[37] The determination method according to [32], wherein the cancer patient is a patient who has received two cycles of treatment with the anti-cancer agent, the amount of one or more molecules selected from the group consisting of 3IND, 4OVAL, 5A4CR, ALA, CSSG, GABB and TMNO is measured, and the method further comprises determining whether or not the cancer patient has PD after treatment with the anti-cancer agent, by comparing a measurement result with a cut-off value of PD, wherein the cut-off value is $< 6.129 \times 10^{-2}$ for 3IND, $< 1.346 \times 10^{-2}$ for 4OVAL, $2.052 \times 10^{-2} \leq$ for 5A4CR, $< 7.3693$ for ALA, $< 1.273 \times 10^{-2}$ for CSSG, $5.117 \times 10^{-2} \leq$ for GABB, and $< 2.689 \times 10^{-1}$ for TMNO.

[38] The determination method according to [32], wherein the cancer patient is a patient who has received one cycle of treatment with the anti-cancer agent, and the method further comprises determining whether or not the cancer patient has PR after treatment with the anti-cancer agent, by calculating a probability (p) of PR according to the following expression (4):

$$p = \frac{1}{1+e^{(9.0171-(CREAT)-(CSSG)-(QUINA))}} \qquad (4)$$

wherein CREAT represents 1.2906 when a measurement result about CREAT is equal to or more than a cut-off value, and represents -1.2906 when the measurement result is less than the cut-off value; CSSG represents 1.7703 when a measurement result about CSSG is equal to or more than a cut-off value, and represents -1.7703 when the measurement result is less than the cut-off value; QUINA represents -8.6990 when a measurement result about QUINA is equal to or more than a cut-off value, and represents 8.6990 when the measurement result is less than the cut-off value; and the cut-off value is 1.2163 for CREAT, $1.965 \times 10^{-2}$ for CSSG, and $1.150 \times 10^{-2}$ for QUINA.

[39] The determination method according to [32], wherein the cancer patient is a patient who has received one cycle of treatment with the anti-cancer agent, and the method further comprises determining whether or not the cancer patient has PD after treatment with the anti-cancer agent, by calculating a probability (p) of PD according to the following expression (5):

$$p = \frac{1}{1+e^{(0.8232+(5A4CR)+(CSSG)+(DECNA)+(HYPTA)+(N8ASR))}} \qquad (5)$$

wherein 5A4CR represents -0.9300 when a measurement result about 5A4CR is equal to or more than a cut-off value, and represents 0.9300 when the measurement result is less than the cut-off value; CSSG represents 1.2325 when a measurement result about CSSG is equal to or more than a cut-off value, and represents -1.2325 when the measurement result is less than the cut-off value; DECNA represents 1.3052 when a measurement result about DECNA is equal to or more than a cut-off value, and represents -1.3052 when the measurement result is less than the cut-off value; HYPTA represents 0.8020 when a measurement result about HYPTA is equal to or more than a cut-off value, and represents -0.8020 when the measurement result is less than the cut-off value; N8ASR represents -1.4363 when a measurement result about N8ASR is equal to or more than a cut-off value, and represents 1.4363 when the measurement result is less than the cut-off value; and the cut-off value is $1.413 \times 10^{-2}$ for 5A4CR, $1.030 \times 10^{-2}$ for CSSG, $1.080 \times 10^{-1}$ for DECNA, $1.240 \times 10^{-2}$ for HYPTA, and $1.122 \times 10^{-2}$ for N8ASR.

[40] The determination method according to [32], wherein the cancer patient is a patient who has received two cycles of treatment with the anti-cancer agent, and the method further comprises determining whether or not the cancer patient has PR after treatment with the anti-cancer agent, by calculating a probability (p) of PR according to the following expression (6):

$$p = \frac{1}{1+e^{(1.5237-(4OVAL)-(BENZA)-(LYS))}} \qquad (6)$$

wherein 4OVAL represents 1.2359 when a measurement result about 4OVAL is equal to or more than a cut-off value, and represents -1.2359 when the measurement result is less than the cut-off value; BENZA represents 1.1105 when a measurement result about BENZA is equal to or more than a cut-off value, and represents -1.1105 when the measurement result is less than the cut-off value; LYS represents 0.8767 when a measurement result about LYS is equal to or more than a cut-off value, and represents - 0.8767 when the measurement result is less than the cut-off value; and the cut-off value is $2.949 \times 10^{-2}$ for 4OVAL, $1.367 \times 10^{-1}$ for BENZA, and 4.9765 for LYS.

[41] The determination method according to [32], wherein the cancer patient is a patient who has received two cycles of treatment with the anti-cancer agent, and the method further comprises determining whether or not the cancer patient has PD after treatment with the anti-cancer agent, by calculating a probability (p) of PD according to the following expression (7):

$$p = \frac{1}{1+e^{(2.4054+(3IND)+(5A4CR)+(CSSG)+(GABB)+(TMNO))}} \qquad (7)$$

wherein 3IND represents 1.4853 when a measurement result about 3IND is equal to or more than a cut-off value, and represents -1.4853 when the measurement result is less than the cut-off value; 5A4CR represents -1.0356 when a measurement result about 5A4CR is equal to or more than a cut-off value, and represents 1.0356 when the measurement result is less than the cut-off value; CSSG represents 1.1004 when a measurement result about CSSG is equal to or more than a cut-off value, and represents -1.1004 when the measurement result is less than the cut-off value; GABB represents -1.2343 when a measurement result about GABB is equal to or more than a cut-off value, and represents 1.2343 when the measurement result is less than the cut-off value; TMNO represents 0.9992 when a measurement result about TMNO is equal to or more than a cut-off value, and represents -0.9992 when the measurement result is less than the cut-off value; and the cut-off value is $6.129 \times 10^{-2}$ for 3IND, $2.052 \times 10^{-2}$ for 5A4CR, $1.273 \times 10^{-2}$ for CSSG, $5.117 \times 10^{-2}$ for GABB, and $2.689 \times 10^{-1}$ for TMNO.

[42] The determination method according to any of [32] to [41], wherein the anti-cancer agent further includes an anti-angiogenic drug.

[43] The determination method according to [42], wherein the anti-angiogenic drug is bevacizumab.

[44] A marker for predicting prognosis for treatment with an anti-cancer agent, the anti-cancer agent including irinotecan or SN-38 or a salt thereof, fluorouracil or a salt thereof, and levofolinate or a salt thereof, the marker comprising one or more molecules selected from the group consisting of 1-methylnicotinamide (1MNA), 2-hydroxy-4-methylpentanoic acid (2H4MP), 3IND, 3-methylhistidine (3MHIS), 5A4CR, ASP, cyclohexanecarboxylic acid (CHCA), CSSG, GABA, hippuric acid (HIPA), hypoxanthine (HYPX), mucic acid (MUCA), N8ASR and taurine (TAUR).

[45] The marker for predicting prognosis according to [44], wherein the anti-cancer agent further includes an anti-angiogenic drug.

[46] The marker for predicting prognosis according to [45], wherein the anti-angiogenic drug is bevacizumab.

[47] A method for predicting prognosis for treatment with an anti-cancer agent, the anti-cancer agent including irinotecan or SN-38 or a salt thereof, fluorouracil or a salt thereof, and levofolinate or a salt thereof, the method comprising measuring an amount of one or more molecules selected from the group consisting of 1MNA, 2H4MP, 3IND, 3MHIS, 5A4CR, ASP, CHCA, CSSG, GABA, HIPA, HYPX, MUCA, N8ASR and TAUR in a biological sample derived from a cancer patient who has received at least one cycle of treatment with the anti-cancer agent including irinotecan or SN-38 or a salt thereof, fluorouracil or a salt thereof, and levofolinate or a salt thereof.

[48] The method for predicting prognosis according to [47], wherein the anti-cancer agent further includes an anti-angiogenic drug.

[49] The method for predicting prognosis according to [48], wherein the anti-angiogenic drug is bevacizumab.

[50] A kit for carrying out a determination method according to any of [32] to [43], the kit comprising a protocol for measuring an amount of one or more molecules selected from the group consisting of 3IND, 4OVAL, 5A4CR, ALA, BENZA, CREAT, CSSG, DECNA, GABB, GLC3P, HYPTA, LYS, METSF, N8ASR, QUINA, SARCO, TMNO and VAL in a biological sample derived from a cancer patient who has received at least one cycle of treatment with the anti-cancer agent including irinotecan or SN-38 or a salt thereof, fluorouracil or a salt thereof, and levofolinate or a salt thereof.

[51] A kit for carrying out a method for predicting prognosis according to any of [47] to [49], the kit comprising a protocol for measuring an amount of one or more molecules selected from the group consisting of 1MNA, 2H4MP, 3IND, 3MHIS, 5A4CR, ASP, CHCA, CSSG, GABA, HIPA, HYPX, MUCA, N8ASR and TAUR in a biological sample derived from a cancer patient who has received at least one cycle of treatment with the anti-cancer agent including irinotecan or SN-38 or a salt thereof, fluorouracil or a salt thereof, and levofolinate or a salt thereof.

Effects of the Invention

Use of the marker of the present invention for determining anti-cancer agent sensitivity can accurately determine the anti-cancer agent sensitivity or prognosis of each individual patient before the start of treatment or in an early stage after the start of treatment, and consequently permits selection of an anti-cancer agent having high therapeutic effects. Furthermore, unnecessary adverse effects can be circumvented because use of ineffective anti-cancer agents can be circumvented. Since the schedule of treatment using an anti-cancer agent continues for a long period, sensitivity to the anti-cancer agent for the cancer can be evaluated over time by determining anti-cancer agent sensitivity on a treatment cycle basis even during the continuing treatment. Thus, whether or not to continue the treatment can be determined. As a result, the progression of cancer and increase in adverse effects associated with the continuous administration of an anti-cancer agent having no therapeutic effects can be prevented, also leading to reduction in burdens on patients and reduction in medical cost.

Moreover, use of this marker enables selection of an agent which enhances anti-cancer agent sensitivity through screening. Concomitant use of the targeted anti-cancer agent with the anti-cancer agent sensitivity-enhancing agent drastically improves the effects of cancer treatment. A reagent for measuring the marker of the present invention for determining anti-cancer agent sensitivity is useful as a reagent for determining anti-cancer agent sensitivity.

Brief Description of the Drawings

[Figure 1] Figure 1 is a diagram showing substances which differed in concentration before the start of treatment between a PR group and an SD or PD group having distinct therapeutic response to FOLFIRI therapy combined with bevacizumab.

[Figure 2] Figure 2 is a diagram showing substances which differed in concentration before the start of treatment between a PR or SD group and a PD group having distinct therapeutic response to FOLFIRI therapy combined with bevacizumab.

[Figure 3] Figure 3 is a diagram showing ROC curves of (a) a PR prediction model of the expression (1) and (b) a PD prediction model of the expression (2) among models for determining anti-cancer agent sensitivity.

[Figure 4] Figure 4(a) is a diagram showing a Kaplan-Meier curve drawn after division of patients into a PR group and an SD or PD group according to the expression (1) (PR prediction model). Figure 4(b) is a diagram showing a Kaplan-Meier curve drawn after division of patients into a PR or SD group and a PD group according to the expression (2) (PD prediction model).

[Figure 5] Figure 5 is a diagram of hazard ratios and 95% confidence intervals of metabolites which exhibited significant difference in analyzing the relationship between metabolite concentrations before the start of treatment of FOLFIRI therapy combined with bevacizumab and OS using COX proportional hazard model.

[Figure 6] Figure 6 is a diagram in which patients were grouped using cut-off values as to CSSG, HIS or N6TLY and compared for their OS.

[Figure 7] Figure 7 is a diagram showing the correlation between a total tumor size before the start of treatment of FOLFIRI therapy combined with bevacizumab and the concentration of CSSG.

[Figure 8] Figure 8 is a diagram showing substances which differed in concentration after implementation of one cycle of treatment between a PR group and an SD or PD group having distinct therapeutic response to FOLFIRI therapy combined with bevacizumab.

[Figure 9] Figure 9 is a diagram showing substances which differed in concentration after implementation of one cycle of treatment between a PR or SD group and a PD group having distinct therapeutic response to FOLFIRI therapy combined with bevacizumab.

[Figure 10] Figure 10 is a diagram showing substances which differed in concentration after implementation of two cycles of treatment between a PR group and an SD or PD group having distinct therapeutic response to FOLFIRI therapy combined with bevacizumab.

[Figure 11] Figure 11 is a diagram showing substances which differed in concentration after implementation of two cycles of treatment between a PR or SD group and a PD group having distinct therapeutic response to FOLFIRI therapy combined with bevacizumab.

[Figure 12] Figure 12 is a diagram showing ROC curves of (a) a PR prediction model of the expression (4), (b) a PD prediction model of the expression (5), (c) a PR prediction model of the expression (6) and (d) a PD prediction model of the expression (7) among models for determining anti-cancer agent sensitivity.

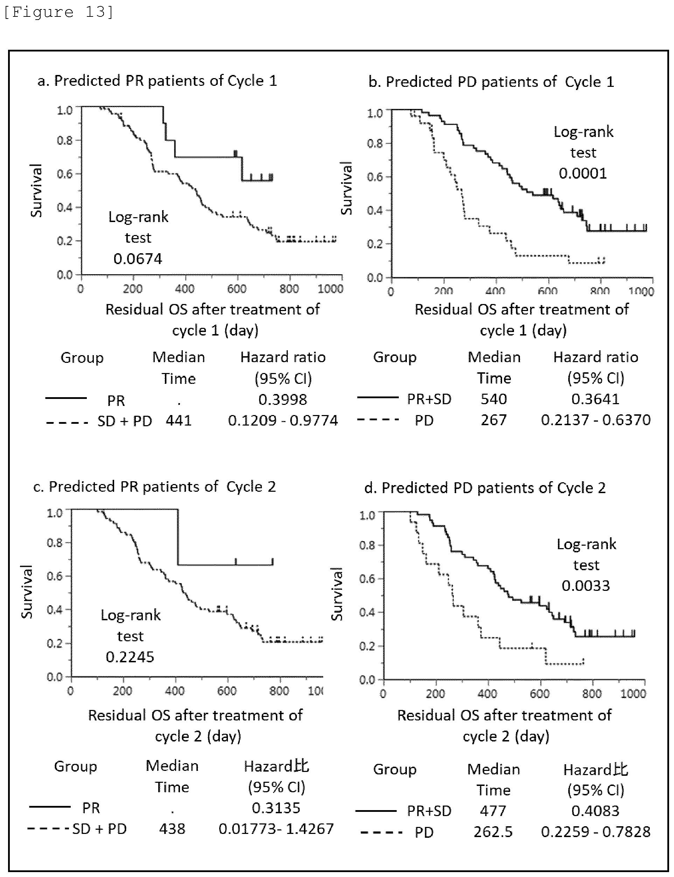

[Figure 13] Figure 13(a) is a diagram showing a Kaplan-Meier curve drawn after division of patients into a PR group and an SD or PD group according to the expression (4) (PR prediction model). Figure 13(b) is a diagram showing a Kaplan-Meier curve drawn after division of patients into a PR or SD group and a PD group according to the expression (5) (PD prediction model). Figure 13(c) is a diagram showing a Kaplan-Meier curve drawn after division of patients into a PR group and an SD or PD group according to the expression (6) (PR prediction model). Figure 13(d) is a diagram showing a Kaplan-Meier curve drawn after division of patients into a PR or SD group and a PD group according to the expression (7) (PD prediction model).

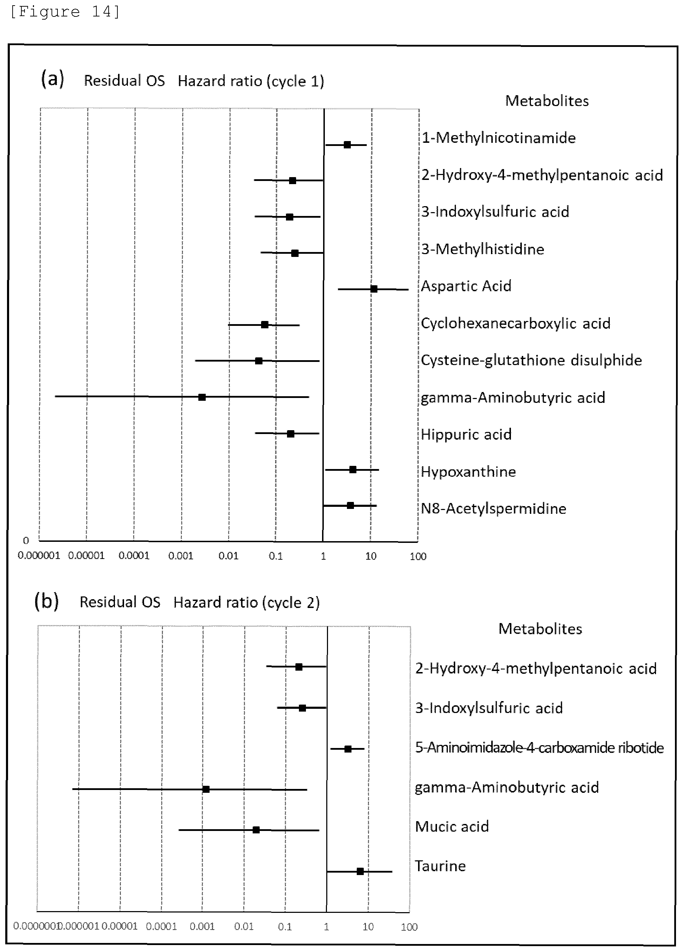

[Figure 14] Figure 14(a) is a diagram of hazard ratios and 95% confidence intervals of metabolites which exhibited significant difference in analyzing the relationship between metabolite concentrations after implementation of one cycle of treatment of FOLFIRI therapy combined with bevacizumab and residual OS using COX proportional hazard model. Figure 14(b) is a diagram of hazard ratios and 95% confidence intervals of metabolites which exhibited significant difference in analyzing the relationship between metabolite concentrations after implementation of two cycles of treatment of FOLFIRI therapy combined with bevacizumab and residual OS using COX proportional hazard model.

Modes for Carrying out the Invention

The marker for determining anti-cancer agent sensitivity according to the present invention includes 20 metabolite substances, i.e., 5-aminoimidazole-4-carboxamide ribotide (5A4CR), alanine (ALA), aspartic acid (ASP), cysteine (CYS), cysteine-glutathione disulphide (CSSG), glycerol-3-phosphate (GLC3P), histidine (HIS), isoleucine (ILE), leucine (LEU), lysine (LYS), methionine sulfoxide (METSF), N6,N6,N6-trimethyllysine (N6TLY), N6-acetyllysine (N6ALY), octanoic acid (OCTA), serine (SER), taurocholic acid (TUCA), threonine (THR), tryptophan (TRP), tyrosine (TYR) and valine (VAL). As shown in Examples mentioned later, the amounts of blood metabolites before the start of FOLFIRI therapy combined with bevacizumab were comprehensively analyzed using CE-Q-TOF MS and CE-TOF MS targeting blood specimens derived from colorectal cancer patients. As a result, among these substances, ALA, CYS, CSSG, HIS, ILE, LEU, LYS, METSF, N6TLY, SER, THR, TRP, TYR and VAL had a higher level in a group which exhibited partial response (PR) by FOLFIRI therapy combined with bevacizumab than in a group which exhibited stable disease (SD) or progressive disease (PD), and were thus found to be metabolites which permit selection of patients who have PR after the therapy. ALA, CSSG, GLC3P, HIS, ILE, LEU, N6TLY, OCTA and THR had a lower level in a group which exhibited PD after FOLFIRI therapy combined with bevacizumab than in a group which exhibited PR or SD, and were thus found to be metabolites which permit selection of patients who have PD after the therapy. 5A4CR, ASP, N6ALY and TUCA had a higher level in a group which exhibited PD after FOLFIRI therapy combined with bevacizumab than in a group which exhibited PR or SD, and were thus found to be metabolites which permit selection of patients who have PD after the therapy. Thus, these 20 substances are useful as markers for determining sensitivity to an anti-cancer agent including irinotecan or SN-38 or a salt thereof, fluorouracil or a salt thereof, and levofolinate or a salt thereof, particularly, as markers for determining sensitivity to an anti-cancer agent including irinotecan or SN-38 or a salt thereof, fluorouracil or a salt thereof, levofolinate or a salt thereof, and an anti-angiogenic drug (particularly, bevacizumab). Furthermore, these markers are useful in use for determining anti-cancer agent sensitivity targeting cancer before the start of treatment with the anti-cancer agent. Among them, 14 substances, ALA, CYS, CSSG, HIS, ILE, LEU, LYS, METSF, N6TLY, SER, THR, TRP, TYR and VAL, can be used as markers for predicting PR for determining whether or not the targeted cancer patient has PR after treatment with the anti-cancer agent. Among them, use of 2 substances, ALA and CSSG, in combination permits prediction of PR with higher accuracy. On the other hand, 13 substances, 5A4CR, ALA, ASP, CSSG, GLC3P, HIS, ILE, LEU, N6TLY, N6ALY, OCTA, TUCA and THR, can be used as markers for predicting PD for determining whether or not the targeted cancer patient has PD after treatment with the anti-cancer agent. Among them, use of 4 substances, ASP, HIS, N6ALY and TUCA, in combination permits prediction of PD with higher accuracy.

As shown in Examples mentioned later, the concentration of CSSG was found to exhibit negative correlation with a total tumor size before the start of treatment of a cancer patient refractory or intolerant to FOLFOX therapy combined with bevacizumab. Thus, CSSG is useful as a marker for determining a total tumor size of a cancer patient.

The marker for predicting prognosis for treatment with an anti-cancer agent according to the present invention includes 14 metabolite substances, i.e., 3-indoxylsulfuric acid (3IND), ALA, ASP, citrulline (CITR), creatine (CREAT), CSSG, gamma-aminobutyric acid (GABA), guanidoacetic acid (GUAA), HIS, hydroxyproline (HYPRO), METSF, N6TLY, N8-acetylspermidine (N8ASR) and SER. As shown in Examples mentioned later, the amounts of blood metabolites before the start of FOLFIRI therapy combined with bevacizumab and overall survivals were analyzed using COX proportional hazard model targeting blood specimens derived from colorectal cancer patients. As a result, among these substances, it was found that the higher the blood concentrations of 3IND, ALA, CITR, CREAT, CSSG, GABA, GUAA, HIS, HYPRO, METSF, N6TLY and SER, the longer the survival period, and the higher the blood concentrations of ASP and N8ASR, the shorter the survival period. Thus, these 14 substances are useful as markers for predicting prognosis for treatment with an anti-cancer agent including irinotecan or SN-38 or a salt thereof, fluorouracil or a salt thereof, and levofolinate or a salt thereof, particularly, an anti-cancer agent including irinotecan or SN-38 or a salt thereof, fluorouracil or a salt thereof, levofolinate or a salt thereof, and an anti-angiogenic drug (particularly, bevacizumab), particularly, as markers for predicting the length of a survival period, also including secondary treatment or subsequent treatment.

Furthermore, these markers are useful in use for predicting prognosis targeting cancer before the start of treatment with the anti-cancer agent.

The marker for determining anti-cancer agent sensitivity according to the present invention also includes 18 metabolite substances, i.e., 3IND, 4-oxavaleric acid (4OVAL), 5A4CR, ALA, benzoic acid (BENZA), CREAT, CSSG, decanoic acid (DECNA), gamma-butyrobetaine (GABB), GLC3P, hypotaurine (HYPTA), LYS, METSF, N8ASR, quinic acid (QUINA), sarcosine (SARCO), trimethylamine N-oxide (TMNO) and VAL. As shown in Examples mentioned later, the amounts of blood metabolites after implementation of one cycle of FOLFIRI therapy combined with bevacizumab were comprehensively analyzed using CE-Q-TOF MS and CE-TOF MS targeting blood specimens derived from colorectal cancer patients. As a result, among these substances, CREAT, CSSG, METSF and VAL had a higher level in a group which exhibited PR after FOLFIRI therapy combined with bevacizumab than in a group which exhibited SD or PD, and were thus found to be metabolites which permit selection of patients who have PR after the therapy. QUINA had a lower level in a group which exhibited PR after FOLFIRI therapy combined with bevacizumab than in a group which exhibited SD or PD, and was thus found to be a metabolite which permits selection of patients who have PR after the therapy. CSSG, DECNA, GLC3P and HYPTA had a lower level in a group which exhibited PD after FOLFIRI therapy combined with bevacizumab than in a group which exhibited PR or SD, and were thus found to be metabolites which permit selection of patients who have PD after the therapy. 5A4CR and N8ASR had a higher level in a group which exhibited PD after FOLFIRI therapy combined with bevacizumab than in a group which exhibited PR or SD, and were thus found to be metabolites which permit selection of patients who have PD after the therapy. As shown in Examples mentioned later, the amounts of blood metabolites after implementation of two cycles of FOLFIRI therapy combined with bevacizumab were comprehensively analyzed using CE-Q-TOF MS and CE-TOF MS targeting blood specimens derived from colorectal cancer patients. As a result, among these substances, 4OVAL, ALA, BENZA, CREAT, CSSG, LYS and SARCO had a higher level in a group which exhibited PR after FOLFIRI therapy combined with bevacizumab than in a group which exhibited SD or PD, and were thus found to be metabolites which permit selection of patients who have PR after the therapy. 3IND, 4OVAL, ALA, CSSG and TMNO had a lower level in a group which exhibited PD after FOLFIRI therapy combined with bevacizumab than in a group which exhibited PR or SD, and were thus found to be metabolites which permit selection of patients who have PD after the therapy. 5A4CR and GABB had a higher level in a group which exhibited PD after FOLFIRI therapy combined with bevacizumab than in a group which exhibited PR or SD, and were thus found to be metabolites which permit selection of patients who have PD after the therapy. Thus, these 18 substances are useful as markers for determining sensitivity to an anti-cancer agent including irinotecan or SN-38 or a salt thereof, fluorouracil or a salt thereof, and levofolinate or a salt thereof, particularly, as markers for determining sensitivity to an anti-cancer agent including irinotecan or SN-38 or a salt thereof, fluorouracil or a salt thereof, levofolinate or a salt thereof, and an anti-angiogenic drug (particularly, bevacizumab). Furthermore, these markers are useful in use for determining anti-cancer agent sensitivity targeting cancer in an early stage after the start of treatment with the anti-cancer agent. Among them, 10 substances, 4OVAL, ALA, BENZA, CREAT, CSSG, LYS, METSF, QUINA, SARCO and VAL, can be used as markers for predicting PR for determining whether or not the targeted cancer patient has PR after treatment with the anti-cancer agent. Among them, use of 3 substances, CREAT, CSSG and QUINA, in combination, or 3 substances, 4OVAL, BENZA and LYS, in combination permits prediction of PR with higher accuracy. On the other hand, 11 substances, 3IND, 4OVAL, 5A4CR, ALA, CSSG, DECNA, GABB, GLC3P, HYPTA, N8ASR and TMNO, can be used as markers for predicting PD for determining whether or not the targeted cancer patient has PD after treatment with the anti-cancer agent. Among them, use of 5 substances, 5A4CR, CSSG, DECNA, HYPTA and N8ASR, in combination, or 5 substances, 3IND, 5A4CR, CSSG, GABB and TMNO, in combination permits prediction of PD with higher accuracy.

The marker for predicting prognosis for treatment with an anti-cancer agent according to the present invention includes 14 metabolite substances, i.e., 1-methylnicotinamide (1MNA), 2-hydroxy-4-methylpentanoic acid (2H4MP), 3IND, 3-methylhistidine (3MHIS), 5A4CR, ASP, cyclohexanecarboxylic acid (CHCA), CSSG, GABA, hippuric acid (HIPA), hypoxanthine (HYPX), mucic acid (MUCA), N8ASR and taurine (TAUR). As shown in Examples mentioned later, the amounts of blood metabolites after implementation of one cycle of FOLFIRI therapy combined with bevacizumab and residual overall survivals were analyzed using COX proportional hazard model targeting blood specimens derived from colorectal cancer patients. As a result, among these substances, it was found that the higher the blood concentrations of 2H4MP, 3IND, 3MHIS, CHCA, CSSG, GABA and HIPA, the longer the survival period, and the higher the blood concentrations of 1MNA, ASP, HYPX and N8ASR, the shorter the survival period. In addition, as shown in Examples mentioned later, the amounts of blood metabolites after implementation of two cycles of FOLFIRI therapy combined with bevacizumab and residual overall survivals were analyzed using COX proportional hazard model targeting blood specimens derived from colorectal cancer patients. As a result, among these substances, it was found that the higher the blood concentrations of 2H4MP, 3IND, GABA and MUCA, the longer the survival period, and the higher the blood concentrations of 5A4CR and TAUR, the shorter the survival period. Thus, these 14 substances are useful as markers for predicting prognosis for treatment with an anti-cancer agent including irinotecan or SN-38 or a salt thereof, fluorouracil or a salt thereof, and levofolinate or a salt thereof, particularly, an anti-cancer agent including irinotecan or SN-38 or a salt thereof, fluorouracil or a salt thereof, levofolinate or a salt thereof, and an anti-angiogenic drug (particularly, bevacizumab), particularly, as markers for predicting the length of a survival period, also including secondary treatment or subsequent treatment. Furthermore, these markers are useful in use for predicting prognosis targeting cancer in an early stage after the start of treatment with the anti-cancer agent.

In the present specification, the term "partial response: PR" refers to a state confirmed to have evident tumor shrinkage by treatment with an anti-cancer agent. PR also includes complete response (CR) in which tumor has disappeared. The term "stable disease: SD" refers to a state which has successfully controlled tumor without tumor enlargement, though evident tumor shrinkage has not been seen by treatment with an anti-cancer agent. The term "progressive disease: PD" refers to a state which has failed to control tumor because treatment with an anti-cancer agent has been totally ineffective. All of these states are evaluated on the basis of Response Evaluation Criteria in Solid Tumors Guideline (RECIST) 1.0.

In the present specification, the term "before the start of treatment with an anti-cancer agent" refers to a state before receiving treatment with an anti-cancer agent including irinotecan or SN-38 or a salt thereof, fluorouracil or a salt thereof, and levofolinate or a salt thereof, i.e., before administration of the anti-cancer agent.

In the present specification, the term " early stage after the start of treatment with an anti-cancer agent" refers to a state which has received at least one cycle, preferably one or more cycles and four or less cycles, more preferably one or more cycles and three or less cycles, further preferably one cycle or two cycles, of treatment with the anti-cancer agent.

In the present specification, the term "patient who has PR (or PD) after treatment with an anti-cancer agent" refers to a patient who has PR (or PD) in terms of final clinical effects of treatment with the anti-cancer agent.

5A4CR is known as a substance in the histidine metabolic pathway, the purine metabolic pathway and the AMPK signaling pathway. However, it is totally unknown that 5A4CR can be used as a marker for determining sensitivity to an anti-cancer agent including irinotecan or SN-38 or a salt thereof, fluorouracil or a salt thereof, and levofolinate or a salt thereof, and permits determination of PD by its high concentration before the start of treatment with the anti-cancer agent or in an early stage after the start of treatment, particularly, after implementation of one cycle or two cycles of treatment, with the anti-cancer agent.

Furthermore, it is totally unknown that 5A4CR can be used as a marker for predicting prognosis for treatment with an anti-cancer agent including irinotecan or SN-38 or a salt thereof, fluorouracil or a salt thereof, and levofolinate or a salt thereof, and permits determination of a shorter survival period by its higher concentration in an early stage after the start of treatment, particularly, after implementation of two cycles of treatment, with the anti-cancer agent.

ALA, an amino acid, is known as a substance not only in the ALA, aspartate and glutamate metabolic pathways, the CYS and methionine metabolic pathways, and the taurine and hypotaurine metabolic pathways but in various metabolic pathways. ALA is also known as a biomarker for diagnosing depression or a biomarker for prostate cancer. However, it is totally unknown that ALA can be used as a marker for determining sensitivity to an anti-cancer agent including irinotecan or SN-38 or a salt thereof, fluorouracil or a salt thereof, and levofolinate or a salt thereof, and permits determination of PR by its high concentration and PD by its low concentration before the start of treatment with the anti-cancer agent or in an early stage after the start of treatment, particularly, after implementation of two cycles of treatment, with the anti-cancer agent.

Furthermore, it is totally unknown that ALA can be used as a marker for predicting prognosis for treatment with an anti-cancer agent including irinotecan or SN-38 or a salt thereof, fluorouracil or a salt thereof, and levofolinate or a salt thereof, and permits determination of a longer survival period by its higher concentration before the start of treatment with the anti-cancer agent.

ASP, a widely known amino acid, is known as a substance not only in the ALA, aspartate and glutamate metabolic pathways, the CYS and methionine metabolic pathways, and the glycine, SER and THR metabolic pathways but in various metabolic pathways. It has already been known that ASP can be used as a marker for determining sensitivity to an anti-cancer agent including oxaliplatin or a salt thereof, and fluorouracil or a salt thereof, and has a higher concentration in a highly oxaliplatin-sensitive cell line than in a low oxaliplatin-sensitive cell line (WO 2013/125675). However, it is totally unknown that ASP can be used as a marker for determining sensitivity to an anti-cancer agent including irinotecan or SN-38 or a salt thereof, fluorouracil or a salt thereof, and levofolinate or a salt thereof, and permits determination of PD by its high concentration before the start of treatment with the anti-cancer agent.

Furthermore, it is totally unknown that ASP can be used as a marker for predicting prognosis for treatment with an anti-cancer agent including irinotecan or SN-38 or a salt thereof, fluorouracil or a salt thereof, and levofolinate or a salt thereof, and permits determination of a shorter survival period by its higher concentration before the start of treatment with the anti-cancer agent or in an early stage after the start of treatment, particularly, after implementation of one cycle of treatment, with the anti-cancer agent.

CYS, an amino acid, is a substance in the CYS and methionine metabolic pathways and is also known as a substance in the glycine, SER and THR metabolic pathways as well as various metabolic pathways. CYS is also known as a biomarker for obstructive sleep apnea or a biomarker for epilepsy. However, it is totally unknown that CYS can be used as a marker for determining sensitivity to an anti-cancer agent including irinotecan or SN-38 or a salt thereof, fluorouracil or a salt thereof, and levofolinate or a salt thereof, and permits determination of PR by its high concentration before the start of treatment with the anti-cancer agent.

CSSG is a metabolite of glutathione (GSH) known as a substance involved in the detoxification of drugs. CSSG is composed of GSH and CYS bound to each other. The findings are known that: GSH in blood is rapidly oxidized into GSSG after blood collection; and more molecules of GSH bind rapidly after blood collection to CYS more abundant in blood than GSH to form CSSG in a few minutes, for example. There are no reports focused on drug efficacy and CSSG, also including reports related to cancer. It is totally unknown that CSSG can be used as a marker for determining sensitivity to an anti-cancer agent including irinotecan or SN-38 or a salt thereof, fluorouracil or a salt thereof, and levofolinate or a salt thereof, and permits determination of PR by its high concentration and PD by its low concentration before the start of treatment with the anti-cancer agent or in an early stage after the start of treatment, particularly, after implementation of one cycle or two cycles of treatment, with the anti-cancer agent.

Furthermore, it is totally unknown that CSSG can be used as a marker for predicting prognosis for treatment with an anti-cancer agent including irinotecan or SN-38 or a salt thereof, fluorouracil or a salt thereof, and levofolinate or a salt thereof, and permits determination of a longer survival period by its higher concentration before the start of treatment with the anti-cancer agent or in an early stage after the start of treatment, particularly, after implementation of one cycle of treatment, with the anti-cancer agent. In addition, it is also totally unknown that CSSG serves as a marker for predicting a total tumor size before the start of treatment of a cancer patient refractory or intolerant to FOLFOX therapy combined with bevacizumab.

GLC3P is known as a substance in the glycerolipid metabolic pathway, the glycerophospholipid metabolic pathway and the choline metabolic pathway in cancer. However, it is totally unknown that GLC3P can be used as a marker for determining sensitivity to an anti-cancer agent including irinotecan or SN-38 or a salt thereof, fluorouracil or a salt thereof, and levofolinate or a salt thereof, and permits determination of PD by its low concentration before the start of treatment with the anti-cancer agent or in an early stage after the start of treatment, particularly, after implementation of one cycle of treatment, with the anti-cancer agent.

HIS, an amino acid, is known as a substance in the HIS metabolic pathway and the beta-ALA metabolic pathway and also known as a biomarker for cancer in blood, a biomarker for inflammatory bowel disease or a biomarker for diagnosing depression. However, it is totally unknown that HIS can be used as a marker for determining sensitivity to an anti-cancer agent including irinotecan or SN-38 or a salt thereof, fluorouracil or a salt thereof, and levofolinate or a salt thereof, and permits determination of PR by its high concentration and PD by its low concentration before the start of treatment with the anti-cancer agent.

Furthermore, it is totally unknown that HIS can be used as a marker for predicting prognosis for treatment with an anti-cancer agent including irinotecan or SN-38 or a salt thereof, fluorouracil or a salt thereof, and levofolinate or a salt thereof, and permits determination of a longer survival period by its higher concentration before the start of treatment with the anti-cancer agent.

ILE, an essential amino acid, is known as a substance in the synthesis and metabolic pathways of VAL, LEU and ILE. ILE is also known as a salivary biomarker for cancer intended for breast cancer detection, a biomarker for cancer in blood or a biomarker for diagnosing depression. However, it is totally unknown that ILE can be used as a marker for determining sensitivity to an anti-cancer agent including irinotecan or SN-38 or a salt thereof, fluorouracil or a salt thereof, and levofolinate or a salt thereof, and permits determination of PR by its high concentration and PD by its low concentration before the start of treatment with the anti-cancer agent.

LEU, an essential amino acid, is known as a substance in the synthesis and metabolic pathways of VAL, LEU and ILE. LEU is also known as a biomarker for cancer in blood or a biomarker for diagnosing depression. However, it is totally unknown that LEU can be used as a marker for determining sensitivity to an anti-cancer agent including irinotecan or SN-38 or a salt thereof, fluorouracil or a salt thereof, and levofolinate or a salt thereof, and permits determination of PR by its high concentration and PD by its low concentration before the start of treatment with the anti-cancer agent.

LYS, an amino acid, is known as a substance in the LYS synthesis and metabolic pathways. LYS is also known as a salivary biomarker for cancer intended for mouth cancer detection or a biomarker for diagnosing depression. However, it is totally unknown that LYS can be used as a marker for determining sensitivity to an anti-cancer agent including irinotecan or SN-38 or a salt thereof, fluorouracil or a salt thereof, and levofolinate or a salt thereof, and permits determination of PR by its high concentration before the start of treatment with the anti-cancer agent or in an early stage after the start of treatment, particularly, after implementation of two cycles of treatment, with the anti-cancer agent.

METSF is known as a substance in the CYS and methionine metabolic pathways and also known as a biomarker for prostate cancer or oxidative stress or a biomarker for diagnosing depression. However, it is totally unknown that METSF can be used as a marker for determining sensitivity to an anti-cancer agent including irinotecan or SN-38 or a salt thereof, fluorouracil or a salt thereof, and levofolinate or a salt thereof, and permits determination of PR by its high concentration before the start of treatment with the anti-cancer agent or in an early stage after the start of treatment, particularly, after implementation of one cycle of treatment, with the anti-cancer agent.

Furthermore, it is totally unknown that METSF can be used as a marker for predicting prognosis for treatment with an anti-cancer agent including irinotecan or SN-38 or a salt thereof, fluorouracil or a salt thereof, and levofolinate or a salt thereof, and permits determination of a longer survival period by its higher concentration before the start of treatment with the anti-cancer agent.

N6TLY is known as a substance in the LYS metabolic pathway and also known as a salivary marker for cancer. However, it is totally unknown that N6TLY can be used as a marker for determining sensitivity to an anti-cancer agent including irinotecan or SN-38 or a salt thereof, fluorouracil or a salt thereof, and levofolinate or a salt thereof, and permits determination of PR by its high concentration and PD by its low concentration before the start of treatment with the anti-cancer agent.

Furthermore, it is totally unknown that N6TLY can be used as a marker for predicting prognosis for treatment with an anti-cancer agent including irinotecan or SN-38 or a salt thereof, fluorouracil or a salt thereof, and levofolinate or a salt thereof, and permits determination of a longer survival period by its higher concentration before the start of treatment with the anti-cancer agent.

N6ALY is known as a substance in the LYS metabolic pathway. However, it is totally unknown that N6ALY can be used as a marker for determining sensitivity to an anti-cancer agent including irinotecan or SN-38 or a salt thereof, fluorouracil or a salt thereof, and levofolinate or a salt thereof, and permits determination of PD by its high concentration before the start of treatment with the anti-cancer agent.

OCTA is known as a metabolite in the fatty acid synthesis pathway. However, it is totally unknown that OCTA can be used as a marker for determining sensitivity to an anti-cancer agent including irinotecan or SN-38 or a salt thereof, fluorouracil or a salt thereof, and levofolinate or a salt thereof, and permits determination of PD by its low concentration before the start of treatment with the anti-cancer agent.

SER, an amino acid, is a substance in the glycine, SER and THR metabolic pathways, and the CYS and methionine metabolic pathways as well as various metabolic pathways. SER is known as a marker for schizophrenia, amyotrophic lateral sclerosis, and acute kidney injury or a biomarker for diagnosing depression. However, it is totally unknown that SER can be used as a marker for determining sensitivity to an anti-cancer agent including irinotecan or SN-38 or a salt thereof, fluorouracil or a salt thereof, and levofolinate or a salt thereof, and permits determination of PR by its high concentration before the start of treatment with the anti-cancer agent.

Furthermore, it is totally unknown that SER can be used as a marker for predicting prognosis for treatment with an anti-cancer agent including irinotecan or SN-38 or a salt thereof, fluorouracil or a salt thereof, and levofolinate or a salt thereof, and permits determination of a longer survival period by its higher concentration before the start of treatment with the anti-cancer agent.

TUCA is a substance in the bile acid synthesis pathway and is known as a substance in the taurine and hypotaurine metabolic pathways. TUCA is further known as a marker for the examination of liver damage. However, it is totally unknown that TUCA can be used as a marker for determining sensitivity to an anti-cancer agent including irinotecan or SN-38 or a salt thereof, fluorouracil or a salt thereof, and levofolinate or a salt thereof, and permits determination of PD by its high concentration before the start of treatment with the anti-cancer agent.

THR, an amino acid, is known as a substance in the glycine, SER and THR metabolic pathways and as a substance in the VAL, LEU and ILE synthesis and metabolic pathways. THR has already been known as a biomarker for diagnosing depression. However, it is totally unknown that THR can be used as a marker for determining sensitivity to an anti-cancer agent including irinotecan or SN-38 or a salt thereof, fluorouracil or a salt thereof, and levofolinate or a salt thereof, and permits determination of PR by its high concentration and PD by its low concentration before the start of treatment with the anti-cancer agent.

TRP, an amino acid, is known as a substance in various metabolic pathways such as the TRP metabolic pathway, and the glycine, SER and THR metabolic pathways. TRP has already been known as a salivary biomarker for cancer intended for breast cancer or mouth cancer detection or a biomarker for cancer in blood, and as a biomarker indicating tryptophan hydroxylase activity by a TRP/5-hydroxytryptophan ratio or a biomarker for diagnosing depression. However, it is totally unknown that TRP can be used as a marker for determining sensitivity to an anti-cancer agent including irinotecan or SN-38 or a salt thereof, fluorouracil or a salt thereof, and levofolinate or a salt thereof, and permits determination of PR by its high concentration before the start of treatment with the anti-cancer agent.

TYR, an amino acid, is known as a substance not only in the TYR metabolic pathway but in various metabolic pathways such as the phenylalanine metabolic pathway. TYR has already been known as a biomarker for cancer in blood, and as a biomarker indicating tyrosine hydroxylase activity by a TYR/dihydroxyphenylalanine ratio or a biomarker for diagnosing depression. However, it is totally unknown that TYR can be used as a marker for determining sensitivity to an anti-cancer agent including irinotecan or SN-38 or a salt thereof, fluorouracil or a salt thereof, and levofolinate or a salt thereof, and permits determination of PR by its high concentration before the start of treatment with the anti-cancer agent.

VAL, an essential amino acid, is known as a substance in the VAL, LEU and ILE synthesis and metabolic pathways. VAL is also known as a biomarker for cancer in blood or a biomarker for diagnosing depression. However, it is totally unknown that VAL can be used as a marker for determining sensitivity to an anti-cancer agent including irinotecan or SN-38 or a salt thereof, fluorouracil or a salt thereof, and levofolinate or a salt thereof, and permits determination of PR by its high concentration before the start of treatment with the anti-cancer agent or in an early stage after the start of treatment, particularly, after implementation of one cycle of treatment, with the anti-cancer agent.

3IND is a metabolite of tryptophan and is known as a causative substance of uremia. It is known that 3IND decreases GSH concentrations and can be used as a biomarker for chronic kidney disease. However, it is totally unknown that 3IND can be used as a marker for determining sensitivity to an anti-cancer agent including irinotecan or SN-38 or a salt thereof, fluorouracil or a salt thereof, and levofolinate or a salt thereof, and permits determination of PD by its low concentration in an early stage after the start of treatment, particularly, after implementation of two cycles of treatment, with the anti-cancer agent.

Furthermore, it is totally unknown that 3IND can be used as a marker for predicting prognosis for treatment with an anti-cancer agent including irinotecan or SN-38 or a salt thereof, fluorouracil or a salt thereof, and levofolinate or a salt thereof, and permits determination of a longer survival period by its higher concentration before the start of treatment with the anti-cancer agent or in an early stage after the start of treatment, particularly, after implementation of one cycle or two cycles of treatment, with the anti-cancer agent.

CITR is known as a substance in the arginine synthesis pathway and known to serve as a biomarker for fatigue by an ornithine/CITR ratio. However, it is totally unknown that CITR can be used as a marker for predicting prognosis for treatment with an anti-cancer agent including irinotecan or SN-38 or a salt thereof, fluorouracil or a salt thereof, and levofolinate or a salt thereof, and permits determination of a longer survival period by its higher concentration before the start of treatment with the anti-cancer agent.

CREAT is an amino acid present in muscle. However, it is totally unknown that CREAT can be used as a marker for determining sensitivity to an anti-cancer agent including irinotecan or SN-38 or a salt thereof, fluorouracil or a salt thereof, and levofolinate or a salt thereof, and permits determination of PR by its high concentration in an early stage after the start of treatment, particularly, after implementation of one cycle or two cycles of treatment, with the anti-cancer agent.

Furthermore, it is totally unknown that CREAT can be used as a marker for predicting prognosis for treatment with an anti-cancer agent including irinotecan or SN-38 or a salt thereof, fluorouracil or a salt thereof, and levofolinate or a salt thereof, and permits determination of a longer survival period by its higher concentration before the start of treatment with the anti-cancer agent.

GABA is known as a substance in the ALA, aspartate and glutamate metabolic pathways and the arginine and proline metabolic pathways, and has already been known to serve as a marker for determining sensitivity to SN-38 (WO 2011/052750). However, it is totally unknown that GABA can be used as a marker for predicting prognosis for treatment with an anti-cancer agent including irinotecan or SN-38 or a salt thereof, fluorouracil or a salt thereof, and levofolinate or a salt thereof, and permits determination of a longer survival period by its higher concentration before the start of treatment with the anti-cancer agent or in an early stage after the start of treatment, particularly, after implementation of one cycle or two cycles of treatment, with the anti-cancer agent.

GUAA is known as a substance in the glycine, SER and THR metabolic pathways and the arginine and proline metabolic pathways and also known to serve as a diagnostic marker for kidney disease. However, it is totally unknown that GUAA can be used as a marker for predicting prognosis for treatment with an anti-cancer agent including irinotecan or SN-38 or a salt thereof, fluorouracil or a salt thereof, and levofolinate or a salt thereof, and permits determination of a longer survival period by its higher concentration before the start of treatment with the anti-cancer agent.

HYPRO is known as a substance in the arginine and proline metabolic pathways and also known as a biomarker for quantifying the amount of collagen or gelatin. However, it is totally unknown that HYPRO can be used as a marker for predicting prognosis for treatment with an anti-cancer agent including irinotecan or SN-38 or a salt thereof, fluorouracil or a salt thereof, and levofolinate or a salt thereof, and permits determination of a longer survival period by its higher concentration before the start of treatment with the anti-cancer agent.

N8ASR belongs to polyamine and is known as a salivary biomarker for cancer intended for pancreatic disease or mouth cancer detection. However, it is totally unknown that N8ASR can be used as a marker for determining sensitivity to an anti-cancer agent including irinotecan or SN-38 or a salt thereof, fluorouracil or a salt thereof, and levofolinate or a salt thereof, and permits determination of PD by its high concentration in an early stage after the start of treatment, particularly, after implementation of one cycle of treatment, with the anti-cancer agent.

Furthermore, it is totally unknown that N8ASR can be used as a marker for predicting prognosis for treatment with an anti-cancer agent including irinotecan or SN-38 or a salt thereof, fluorouracil or a salt thereof, and levofolinate or a salt thereof, and permits determination of a shorter survival period by its higher concentration before the start of treatment with the anti-cancer agent or in an early stage after the start of treatment, particularly, after implementation of one cycle of treatment, with the anti-cancer agent.

4OVAL belongs to the group of gamma-keto acid and its metabolites and is present in various body fluids such as blood, urine, and saliva and present in the cytoplasms within cells. However, it is totally unknown that 4OVAL can be used as a marker for determining sensitivity to an anti-cancer agent including irinotecan or SN-38 or a salt thereof, fluorouracil or a salt thereof, and levofolinate or a salt thereof, and permits determination of PR by its high concentration and PD by its low concentration in an early stage after the start of treatment, particularly, after implementation of two cycles of treatment, with the anti-cancer agent.

BENZA is a substance which is degraded into hippuric acid in the liver. However, it is totally unknown that BENZA can be used as a marker for determining sensitivity to an anti-cancer agent including irinotecan or SN-38 or a salt thereof, fluorouracil or a salt thereof, and levofolinate or a salt thereof, and permits determination of PR by its high concentration in an early stage after the start of treatment, particularly, after implementation of two cycles of treatment, with the anti-cancer agent.

DECNA is a fatty acid. However, it is totally unknown that DECNA can be used as a marker for determining sensitivity to an anti-cancer agent including irinotecan or SN-38 or a salt thereof, fluorouracil or a salt thereof, and levofolinate or a salt thereof, and permits determination of PD by its low concentration in an early stage after the start of treatment, particularly, after implementation of one cycle of treatment, with the anti-cancer agent.

GABB is a product in the lysine degradation pathway and is also known as a precursor of carnitine which plays a role in transporting fatty acids into mitochondria in muscle cells. However, it is totally unknown that GABB can be used as a marker for determining sensitivity to an anti-cancer agent including irinotecan or SN-38 or a salt thereof, fluorouracil or a salt thereof, and levofolinate or a salt thereof, and permits determination of PD by its high concentration in an early stage after the start of treatment, particularly, after implementation of two cycles of treatment, with the anti-cancer agent.

HYPTA is an intermediate in taurine biosynthesis and acts as a neurotransmitter. However, it is totally unknown that HYPTA can be used as a marker for determining sensitivity to an anti-cancer agent including irinotecan or SN-38 or a salt thereof, fluorouracil or a salt thereof, and levofolinate or a salt thereof, and permits determination of PD by its low concentration in an early stage after the start of treatment, particularly, after implementation of one cycle of treatment, with the anti-cancer agent.

QUINA is a hydroxy acid discovered from china barks. However, it is totally unknown that QUINA can be used as a marker for determining sensitivity to an anti-cancer agent including irinotecan or SN-38 or a salt thereof, fluorouracil or a salt thereof, and levofolinate or a salt thereof, and permits determination of PR by its low concentration in an early stage after the start of treatment, particularly, after implementation of one cycle of treatment, with the anti-cancer agent.

SARCO is an intermediate of metabolism of choline into glycine. However, it is totally unknown that SARCO can be used as a marker for determining sensitivity to an anti-cancer agent including irinotecan or SN-38 or a salt thereof, fluorouracil or a salt thereof, and levofolinate or a salt thereof, and permits determination of PR by its high concentration in an early stage after the start of treatment, particularly, after implementation of two cycles of treatment, with the anti-cancer agent.

TMNO is an oxidation product of trimethylamine and is an *in vivo* metabolic intermediate. However, it is totally unknown that TMNO can be used as a marker for determining sensitivity to an anti-cancer agent including irinotecan or SN-38 or a salt thereof, fluorouracil or a salt thereof, and levofolinate or a salt thereof, and permits determination of PD by its low concentration in an early stage after the start of treatment, particularly, after implementation of two cycles of treatment, with the anti-cancer agent.

1MNA is known as a substance in the nicotinic acid and nicotinamide metabolic pathways and reportedly has an anti-inflammatory effect. However, it is totally unknown that 1MNA can be used as a marker for predicting prognosis for treatment with an anti-cancer agent including irinotecan or SN-38 or a salt thereof, fluorouracil or a salt thereof, and levofolinate or a salt thereof, and permits determination of a shorter survival period by its higher concentration in an early stage after the start of treatment, particularly, after implementation of one cycle of treatment, with the anti-cancer agent.

2H4MP is known to have a high concentration in the urine of patients with dihydrolipoamide dehydrogenase deficiency. However, it is totally unknown that 2H4MP can be used as a marker for predicting prognosis for treatment with an anti-cancer agent including irinotecan or SN-38 or a salt thereof, fluorouracil or a salt thereof, and levofolinate or a salt thereof, and permits determination of a longer survival period by its higher concentration in an early stage after the start of treatment, particularly, after implementation of one cycle or two cycles of treatment, with the anti-cancer agent.

3MHIS is utilized as an index for the amount of myoprotein degraded. However, it is totally unknown that 3MHIS can be used as a marker for predicting prognosis for treatment with an anti-cancer agent including irinotecan or SN-38 or a salt thereof, fluorouracil or a salt thereof, and levofolinate or a salt thereof, and permits determination of a longer survival period by its higher concentration in an early stage after the start of treatment, particularly, after implementation of one cycle of treatment, with the anti-cancer agent.

CHCA is known as a product in the benzoic acid degradation pathway. However, it is totally unknown that CHCA can be used as a marker for predicting prognosis for treatment with an anti-cancer agent including irinotecan or SN-38 or a salt thereof, fluorouracil or a salt thereof, and levofolinate or a salt thereof, and permits determination of a longer survival period by its higher concentration in an early stage after the start of treatment, particularly, after implementation of one cycle of treatment, with the anti-cancer agent.

HIPA is a substance which is produced from an aromatic hydrocarbon compound in the liver. However, it is totally unknown that HIPA can be used as a marker for predicting prognosis for treatment with an anti-cancer agent including irinotecan or SN-38 or a salt thereof, fluorouracil or a salt thereof, and levofolinate or a salt thereof, and permits determination of a longer survival period by its higher concentration in an early stage after the start of treatment, particularly, after implementation of one cycle of treatment, with the anti-cancer agent.

HYPX is a metabolic product of the salvage pathway. However, it is totally unknown that HYPX can be used as a marker for predicting prognosis for treatment with an anti-cancer agent including irinotecan or SN-38 or a salt thereof, fluorouracil or a salt thereof, and levofolinate or a salt thereof, and permits determination of a shorter survival period by its higher concentration in an early stage after the start of treatment, particularly, after implementation of one cycle of treatment, with the anti-cancer agent.

MUCA is a substance in the ascorbic acid and aldaric acid metabolic pathways and is also a glucuronic acid-related substance. However, it is totally unknown that MUCA can be used as a marker for predicting prognosis for treatment with an anti-cancer agent including irinotecan or SN-38 or a salt thereof, fluorouracil or a salt thereof, and levofolinate or a salt thereof, and permits determination of a longer survival period by its higher concentration in an early stage after the start of treatment, particularly, after implementation of two cycles of treatment, with the anti-cancer agent.

TAUR is a substance which is biosynthesized from cysteine. However, it is totally unknown that TAUR can be used as a marker for predicting prognosis for treatment with an anti-cancer agent including irinotecan or SN-38 or a salt thereof, fluorouracil or a salt thereof, and levofolinate or a salt thereof, and permits determination of a shorter survival period by its higher concentration in an early stage after the start of treatment, particularly, after implementation of two cycles of treatment, with the anti-cancer agent.

The anti-cancer agent targeted by the marker of the present invention for determining anti-cancer agent sensitivity or the marker of the present invention for predicting prognosis for treatment with an anti-cancer agent is an anti-cancer agent including irinotecan or SN-38 or a salt thereof, fluorouracil or a salt thereof, and levofolinate or a salt thereof. An anti-cancer agent which is converted to irinotecan or SN-38, fluorouracil or levofolinate through metabolism *in vivo* is also targeted by the marker of the present invention for determining anti-cancer agent sensitivity. Specifically, it has been revealed that tegafur or capecitabine is converted to fluorouracil through metabolism *in vivo.* Therefore, tegafur or capecitabine may be used instead of fluorouracil. In this case, an anti-cancer agent including irinotecan or SN-38 or a salt thereof, tegafur or a salt thereof, and levofolinate or a salt thereof, or an anti-cancer agent including irinotecan or SN-38 or a salt thereof, capecitabine or a salt thereof, and levofolinate or a salt thereof is targeted by the marker of the present invention.

Examples of an additional anti-cancer agent for use in combination with the anti-cancer agent including irinotecan or SN-38 or a salt thereof, fluorouracil or a salt thereof, and levofolinate or a salt thereof include, but are not particularly limited to, oxaliplatin, cyclophosphamide, ifosfamide, thiotepa, melphalan, busulfan, nimustine, ranimustine, dacarbazine, procarbazine, temozolomide, cisplatin, carboplatin, nedaplatin, methotrexate, pemetrexed, tegafur/uracil, doxifluridine, tegafur/gimeracil/oteracil, capecitabine, cytarabine, enocitabine, gemcitabine, 6-mercaptopurine, fludarabine, pentostatin, cladribine, hydroxyurea, doxorubicin, epirubicin, daunorubicin, idarubicine, pirarubicin, mitoxantrone, amrubicin, actinomycin D, bleomycine, pepleomycin, mitomycin C, aclarubicin, zinostatin, vincristine, vindesine, vinblastine, vinorelbine, paclitaxel, docetaxel, nogitecan, topotecan, etoposide, prednisolone, dexamethasone, tamoxifen, toremifene, medroxyprogesterone, anastrozole, exemestane, letrozole, rituximab, imatinib, gefitinib, gemtuzumab ozogamicin, bortezomib, erlotinib, cetuximab, bevacizumab, sunitinib, sorafenib, dasatinib, panitumumab, ramucirumab, aflibercept, asparaginase, tretinoin, arsenic trioxide, and salts thereof, and active metabolites thereof. Among them, an anti-angiogenic drug such as cetuximab, bevacizumab, or panitumumab, or oxaliplatin is preferred, an anti-angiogenic drug is more preferred, and bevacizumab is particularly preferred.

A method for determining anti-cancer agent sensitivity using the marker of the present invention for determining anti-cancer agent sensitivity can be performed by measuring an amount of one or more molecules selected from the group consisting of 5A4CR, ALA, ASP, CYS, CSSG, GLC3P, HIS, ILE, LEU, LYS, METSF, N6TLY, N6ALY, OCTA, SER, TUCA, THR, TRP, TYR and VAL in a biological sample (specimen) derived from a cancer patient before the start of treatment with an anti-cancer agent including irinotecan or SN-38 or a salt thereof, fluorouracil or a salt thereof, and levofolinate or a salt thereof, and specifically, further comparing the measurement result with a control level (a standard concentration, a cut-off value of PR or PD (hereinafter, the cut-off value means a relative concentration when the concentration of an internal standard such as an internal standard solution for LC/MS is defined as 1), etc.).

Specifically, a method for determining anti-cancer agent sensitivity using one or more molecules selected from the group consisting of ALA, CYS, CSSG, HIS, ILE, LEU, LYS, METSF, N6TLY, SER, THR, TRP, TYR and VAL (hereinafter, also referred to as a marker for predicting PR) as the marker of the present invention for determining anti-cancer agent sensitivity can be performed by measuring an amount of the one or more molecules in a biological sample (specimen) derived from a cancer patient before the start of treatment with an anti-cancer agent including irinotecan or SN-38 or a salt thereof, fluorouracil or a salt thereof, and levofolinate or a salt thereof, and specifically, further comparing the measurement result with a control level (a standard concentration, a cut-off value of PR, etc.). Alternatively, this method can be performed by measuring amounts of ALA and CSSG in a biological sample (specimen) derived from a cancer patient before the start of treatment with the anti-cancer agent, and specifically, further digitizing the measurement results by comparison with a control level of each substance (e.g., a cut-off value of PR), and assigning the obtained numeric values to a particular calculation expression. Whether or not the cancer patient has PR after treatment with the anti-cancer agent can be determined by the marker for predicting PR.

A method for determining anti-cancer agent sensitivity using one or more molecules selected from the group consisting of 5A4CR, ALA, ASP, CSSG, GLC3P, HIS, ILE, LEU, N6TLY, N6ALY, OCTA, TUCA and THR (hereinafter, also referred to as a marker for predicting PD) as the marker of the present invention for determining anti-cancer agent sensitivity can be performed by measuring an amount of the one or more molecules in a biological sample (specimen) derived from a cancer patient before the start of treatment with an anti-cancer agent including irinotecan or SN-38 or a salt thereof, fluorouracil or a salt thereof, and levofolinate or a salt thereof, and specifically, further comparing the measurement result with a control level (a standard concentration, a cut-off value of PD, etc.). Alternatively, this method can be performed by measuring amounts of ASP, HIS, N6ALY and TUCA in a biological sample (specimen) derived from a cancer patient before the start of treatment with the anti-cancer agent, and specifically, further digitizing the measurement results by comparison with a control level of each substance (e.g., a cut-off value of PD), and assigning the obtained numeric values to a particular calculation expression. Whether or not the cancer patient has PD after treatment with the anti-cancer agent can be determined by the marker for predicting PD.

Alternatively, a method for determining anti-cancer agent sensitivity using the marker of the present invention for determining anti-cancer agent sensitivity can be performed by measuring an amount of one or more molecules selected from the group consisting of 3IND, 4OVAL, 5A4CR, ALA, BENZA, CREAT, CSSG, DECNA, GABB, GLC3P, HYPTA, LYS, METSF, N8ASR, QUINA, SARCO, TMNO and VAL in a biological sample (specimen) derived from a cancer patient in an early stage after the start of treatment with an anti-cancer agent including irinotecan or SN-38 or a salt thereof, fluorouracil or a salt thereof, and levofolinate or a salt thereof, and specifically, further comparing the measurement result with a control level (a standard concentration, a cut-off value of PR or PD, etc.).

Specifically, a method for determining anti-cancer agent sensitivity using one or more molecules selected from the group consisting of CREAT, CSSG, METSF, QUINA and VAL (hereinafter, also referred to as a marker for predicting PR) as the marker of the present invention for determining anti-cancer agent sensitivity can be performed by measuring an amount of the one or more molecules in a biological sample (specimen) derived from a cancer patient after implementation of one cycle of treatment with an anti-cancer agent including irinotecan or SN-38 or a salt thereof, fluorouracil or a salt thereof, and levofolinate or a salt thereof, and specifically, further comparing the measurement result with a control level (a standard concentration, a cut-off value of PR, etc.). Alternatively, this method can be performed by measuring amounts of CREAT, CSSG and QUINA in a biological sample (specimen) derived from a cancer patient after implementation of one cycle of treatment with the anti-cancer agent, and specifically, further digitizing the measurement results by comparison with a control level of each substance (e.g., a cut-off value of PR), and assigning the obtained numeric values to a particular calculation expression. Whether or not the cancer patient has PR after treatment with the anti-cancer agent can be determined by the marker for predicting PR.

A method for determining anti-cancer agent sensitivity using one or more molecules selected from the group consisting of 5A4CR, CSSG, DECNA, GLC3P, HYPTA and N8ASR (hereinafter, also referred to as a marker for predicting PD) as the marker of the present invention for determining anti-cancer agent sensitivity can be performed by measuring an amount of the one or more molecules in a biological sample (specimen) derived from a cancer patient after implementation of one cycle of treatment with an anti-cancer agent including irinotecan or SN-38 or a salt thereof, fluorouracil or a salt thereof, and levofolinate or a salt thereof, and specifically, further comparing the measurement result with a control level (a standard concentration, a cut-off value of PD, etc.). Alternatively, this method can be performed by measuring amounts of 5A4CR, CSSG, DECNA, HYPTA and N8ASR in a biological sample (specimen) derived from a cancer patient after implementation of one cycle of treatment with the anti-cancer agent, and specifically, further digitizing the measurement results by comparison with a control level of each substance (e.g., a cut-off value of PD), and assigning the obtained numeric values to a particular calculation expression. Whether or not the cancer patient has PD after treatment with the anti-cancer agent can be determined by the marker for predicting PD.

A method for determining anti-cancer agent sensitivity using one or more molecules selected from the group consisting of 4OVAL, ALA, BENZA, CREAT, CSSG, LYS and SARCO (hereinafter, also referred to as a marker for predicting PR) as the marker of the present invention for determining anti-cancer agent sensitivity can be performed by measuring an amount of the one or more molecules in a biological sample (specimen) derived from a cancer patient after implementation of two cycles of treatment with an anti-cancer agent including irinotecan or SN-38 or a salt thereof, fluorouracil or a salt thereof, and levofolinate or a salt thereof, and specifically, further comparing the measurement result with a control level (a standard concentration, a cut-off value of PR, etc.). Alternatively, this method can be performed by measuring amounts of 4OVAL, BENZA and LYS in a biological sample (specimen) derived from a cancer patient after implementation of two cycles of treatment with the anti-cancer agent, and specifically, further digitizing the measurement results by comparison with a control level of each substance (e.g., a cut-off value of PR), and assigning the obtained numeric values to a particular calculation expression. Whether or not the cancer patient has PR after treatment with the anti-cancer agent can be determined by the marker for predicting PR.

A method for determining anti-cancer agent sensitivity using one or more molecules selected from the group consisting of 3IND, 4OVAL, 5A4CR, ALA, CSSG, GABB and TMNO (hereinafter, also referred to as a marker for predicting PD) as the marker of the present invention for determining anti-cancer agent sensitivity can be performed by measuring an amount of the one or more molecules in a biological sample (specimen) derived from a cancer patient after implementation of two cycles of treatment with an anti-cancer agent including irinotecan or SN-38 or a salt thereof, fluorouracil or a salt thereof, and levofolinate or a salt thereof, and specifically, further comparing the measurement result with a control level (a standard concentration, a cut-off value of PD, etc.). Alternatively, this method can be performed by measuring amounts of 3IND, 5A4CR, CSSG, GABB and TMNO in a biological sample (specimen) derived from a cancer patient after implementation of two cycles of treatment with the anti-cancer agent, and specifically, further digitizing the measurement results by comparison with a control level of each substance (e.g., a cut-off value of PD), and assigning the obtained numeric values to a particular calculation expression. Whether or not the cancer patient has PD after treatment with the anti-cancer agent can be determined by the marker for predicting PD.

A method for predicting long-term prognosis for treatment with an anti-cancer agent using the marker of the present invention for predicting prognosis can be performed by measuring an amount of one or more molecules selected from the group consisting of 3IND, ALA, ASP, CITR, CREAT, CSSG, GABA, GUAA, HIS, HYPRO, METSF, N6TLY, N8ASR and SER in a biological sample (specimen) derived from a cancer patient before the start of treatment with an anti-cancer agent including irinotecan or SN-38 or a salt thereof, fluorouracil or a salt thereof, and levofolinate or a salt thereof, and specifically, further comparing the measurement result with a control level (a standard concentration, a cut-off value of PR or PD, a cut-off value of OS, etc.).

Alternatively, a method for predicting long-term prognosis for treatment with an anti-cancer agent using the marker of the present invention for predicting prognosis can be performed by measuring an amount of one or more molecules selected from the group consisting of 1MNA, 2H4MP, 3IND, 3MHIS, 5A4CR, ASP, CHCA, CSSG, GABA, HIPA, HYPX, MUCA, N8ASR and TAUR in a biological sample (specimen) derived from a cancer patient in an early stage after the start of treatment with an anti-cancer agent including irinotecan or SN-38 or a salt thereof, fluorouracil or a salt thereof, and levofolinate or a salt thereof, and specifically, further comparing the measurement result with a control level (a standard concentration, a cut-off value of PR or PD, a cut-off value of OS, etc.).

Specifically, a method for predicting prognosis using one or more molecules selected from the group consisting of 1MNA, 2H4MP, 3IND, 3MHIS, ASP, CHCA, CSSG, GABA, HIPA, HYPX and N8ASR as the marker of the present invention for predicting prognosis can be performed by measuring an amount of the one or more molecules in a biological sample (specimen) derived from a cancer patient after implementation of one cycle of treatment with an anti-cancer agent including irinotecan or SN-38 or a salt thereof, fluorouracil or a salt thereof, and levofolinate or a salt thereof, and specifically, further comparing the measurement result with a control level (a standard concentration, a cut-off value of PR, a cut-off value of OS, etc.).

A method for predicting prognosis using one or more molecules selected from the group consisting of 2H4MP, 3IND, 5A4CR, GABA, MUCA and TAUR as the marker of the present invention for predicting prognosis can be performed by measuring an amount of the one or more molecules in a biological sample (specimen) derived from a cancer patient after implementation of two cycles of treatment with an anti-cancer agent including irinotecan or SN-38 or a salt thereof, fluorouracil or a salt thereof, and levofolinate or a salt thereof, and specifically, further comparing the measurement result with a control level (a standard concentration, a cut-off value of PR, etc.).

In this context, the cancer patient encompasses a test subject having cancer or a test subject who had cancer. Examples of the biological sample include blood, serum, plasma, a cancer tissue biopsy specimen, a cancer extirpation specimen, feces, urine, ascitic fluid, pleural effusion, cerebrospinal fluid, and sputum. Serum is particularly preferred.

Examples of the targeted cancer of the present invention include lip, oral and pharyngeal cancers typified by pharyngeal cancer; gastrointestinal tract cancers typified by esophageal cancer, gastric cancer, colorectal cancer, etc.; respiratory and pleural organ cancers typified by lung cancer, bone and articular cartilage cancers; malignant melanoma of the skin, squamous cell cancer and other cancers of the skin; mesothelial and soft tissue cancers typified by mesothelioma; female genital cancers typified by breast cancer, uterine cancer, and ovarian cancer; male genital cancers typified by prostate cancer; urinary tract cancers typified by bladder cancer; eye, brain and central nervous system cancers typified by brain tumor; thyroid and other endocrine cancers; lymphoid tissue, hematopoietic tissue and related tissue cancers typified by non-Hodgkin's lymphoma and lymphoid leukemia; and metastatic cancers from the aforementioned cancers as primary foci. Among these, colorectal cancer (large intestine cancer) is preferred, and cancer refractory or intolerant to treatment with an anti-cancer agent including oxaliplatin or a salt thereof, fluorouracil or a salt thereof, and levofolinate or a salt thereof is particularly preferred. With respect to pancreatic cancer, chemotherapy based on concomitant treatment with an anti-cancer agent including irinotecan or SN-38 or a salt thereof, capecitabine or a salt thereof, and levofolinate or a salt thereof is performed, whereas only a limited number of patients responds to this therapy. Therefore, pancreatic cancer is also preferred.

The means for measuring the molecule selected from the group consisting of 5A4CR, ALA, ASP, CYS, CSSG, GLC3P, HIS, ILE, LEU, LYS, METSF, N6TLY, N6ALY, OCTA, SER, TUCA, THR, TRP, TYR, VAL, 3IND, CITR, CREAT, GABA, GUAA, HYPRO, N8ASR, 4OVAL, BENZA, DECNA, GABB, HYPTA, QUINA, SARCO, TMNO, 1MNA, 2H4MP, 3MHIS, CHCA, HIPA, HYPX, MUCA and TAUR in a specimen may be appropriately determined according to the substance to be measured, and may be measured by use of, for example, various mass spectrometers such as CE-Q-TOF MS, CE-TOF MS, and gas chromatography-mass spectrometry (GC-MS), HPLC, an immunological assay, or a biochemical assay.

In order to determine sensitivity to the targeted anti-cancer agent, specifically, determine whether or not to have PR after treatment with the anti-cancer agent, using one or more molecules selected from the group consisting of ALA, CYS, CSSG, HIS, ILE, LEU, LYS, METSF, N6TLY, SER, THR, TRP, TYR and VAL, the amount, for example, concentration, of one or more molecules selected from the group consisting of ALA, CYS, CSSG, HIS, ILE, LEU, LYS, METSF, N6TLY, SER, THR, TRP, TYR and VAL in a biological sample derived from a cancer patient may be measured before administration of the anti-cancer agent. When the concentration has a concentration confirmed to be higher than the predetermined control level, cancer in the cancer patient can be determined to be sensitive to the targeted anti-cancer agent, i.e., the cancer patient can be determined to have PR after treatment with the targeted anti-cancer agent. Therefore, these markers for determining anti-cancer agent sensitivity can be used as markers for predicting PR for aggressively continuing the treatment of patients who can be expected to receive therapeutic effects. On the other hand, when the concentration has a concentration confirmed to be lower than the predetermined control level, cancer in the cancer patient can be determined to be not sensitive to the targeted anti-cancer agent, i.e., the cancer patient can be determined to have no PR after treatment with the targeted anti-cancer agent. If the cancer patient has no sensitivity to the targeted anti-cancer agent, the control of tumor with the anti-cancer agent cannot be expected. In the case of performing or continuing the administration of such an anti-cancer agent which cannot be expected to have drug efficacy, there is a fear on the progression of cancer or increase in adverse effects. Thus, the marker for determining anti-cancer agent sensitivity according to the present invention can be used as a marker for aggressively continuing the treatment of patients who can be expected to receive therapeutic effects, and in addition, can also be used as a marker for circumventing the progression of cancer and increase in adverse effects associated with the continuous administration of an anti-cancer agent which cannot be expected to have drug efficacy.

Examples of the control level include cut-off values of PR. Examples of the cut-off values include $9.957 \leq$ for ALA, $2.444 \times 10^{-1} \leq$ for CYS, $1.430 \times 10^{-2} \leq$ for CSSG, $2.2409 \leq$ for HIS, $2.997 \leq$ for ILE, $7.437 \leq$ for LEU, $4.945 \leq$ for LYS, $6.658 \times 10^{-2} \leq$ for METSF, $8.401 \times 10^{-2} \leq$ for N6TLY, $2.200 \leq$ for SER, $2.753 \leq$ for THR, $2.165 \leq$ for TRP, $2.084 \leq$ for TYR, and $8.317 \leq$ for VAL.

In order to determine sensitivity to the targeted anti-cancer agent, specifically, determine whether or not to have PR after treatment with the anti-cancer agent, using ALA and CSSG, the amounts, for example, concentrations, of ALA and CSSG in a biological sample derived from a cancer patient may be measured at a stage before administration of the anti-cancer agent, and the predetermined numeric values according to the measurement results may be assigned to the expression (1):

$$p = \frac{1}{1+e^{(4.2504-(ALA)-(CSSG))}} \tag{1}$$

wherein ALA represents 2.5043 when a measurement result about ALA is equal to or more than a cut-off value, and represents 0 when the measurement result is less than the cut-off value; and CSSG represents 2.4626 when a measurement result about CSSG is equal to or more than a cut-off value, and represents 0 when the measurement result is less than the cut-off value.

The cut-off value of each substance is 9.957 for ALA and $1.430 \times 10^{-2}$ for CSSG.

p calculated according to the expression (1) represents a probability that the targeted cancer patient exhibits tumor shrinkage by treatment with the targeted anti-cancer agent and thus has PR. When p is 0.5 or more, cancer in the cancer patient can be determined to be sensitive to the targeted anti-cancer agent, i.e., the cancer patient can be determined to have PR after treatment with the targeted anti-cancer agent. Therefore, these markers for determining anti-cancer agent sensitivity can be used as markers for predicting PR for aggressively continuing the treatment of patients who can be expected to receive therapeutic effects. On the other hand, when p is less than 0.5, cancer in the cancer patient can be determined to be not sensitive to the targeted anti-cancer agent, i.e., the cancer patient can be determined to have no PR after treatment with the targeted anti-cancer agent. If the cancer patient has no sensitivity to the targeted anti-cancer agent, the control of tumor with the anti-cancer agent cannot be expected. In the case of performing or continuing the administration of such an anti-cancer agent which cannot be expected to have drug efficacy, there is a fear on the progression of cancer or increase in adverse effects. Thus, the marker for determining anti-cancer agent sensitivity according to the present invention can be used as a marker for aggressively continuing the treatment of patients who can be expected to receive therapeutic effects, and in addition, can also be used as a marker for circumventing the progression of cancer and increase in adverse effects associated with the continuous administration of an anti-cancer agent which cannot be expected to have drug efficacy.

In order to determine sensitivity to the targeted anti-cancer agent, specifically, determine whether or not to have PD after treatment with the anti-cancer agent, using one or more molecules selected from the group consisting of 5A4CR, ALA, ASP, CSSG, GLC3P, HIS, ILE, LEU, N6TLY, N6ALY, OCTA, TUCA and THR, the amount, for example, concentration, of one or more molecules selected from the group consisting of 5A4CR, ALA, ASP, CSSG, GLC3P, HIS, ILE, LEU, N6TLY, N6ALY, OCTA, TUCA and THR in a biological sample derived from a cancer patient may be measured at a stage before administration of the anti-cancer agent. When the concentration of one or more molecules selected from the group consisting of 5A4CR, ASP, N6ALY and TUCA has a concentration confirmed to be higher than the predetermined control level, cancer in the cancer patient can be determined to be not sensitive to the targeted anti-cancer agent, i.e., the cancer patient can be determined to have PD after treatment with the targeted anti-cancer agent. When the concentration of one or more molecules selected from the group consisting of ALA, CSSG, GLC3P, HIS, ILE, LEU, N6TLY, OCTA and THR has a concentration confirmed to be lower than the predetermined control level, cancer in the cancer patient can be determined to be not sensitive to the targeted anti-cancer agent, i.e., the cancer patient can be determined to have PD after treatment with the targeted anti-cancer agent. Accordingly, these markers for determining anti-cancer agent sensitivity can be used as markers for predicting PD for circumventing the continuation of the treatment of patients who cannot be expected to receive therapeutic effects, and giving priority to another treatment. On the other hand, when the concentration of one or more molecules selected from the group consisting of 5A4CR, ASP, N6ALY and TUCA has a concentration confirmed to be lower than the predetermined control level, or when the concentration of one or more molecules selected from the group consisting of ALA, CSSG, GLC3P, HIS, ILE, LEU, N6TLY, OCTA and THR has a concentration confirmed to be higher than the predetermined control level, cancer in the cancer patient can be determined to be sensitive to the targeted anti-cancer agent, i.e., the cancer patient can be determined to have no PD after treatment with the targeted anti-cancer agent. If the cancer patient has sensitivity to the targeted anti-cancer agent, the therapeutic effects of the anti-cancer agent, such as the control of tumor or the suppression of disease progression, can be expected. Thus, the marker for determining anti-cancer agent sensitivity according to the present invention can be used as a marker for circumventing the continuation of the treatment of patients who cannot be expected to receive therapeutic effects, and giving priority to another treatment, and in addition, can also be used as a marker for continuing the treatment of patients who can be expected to receive therapeutic effects.

Examples of the control level include cut-off values of PD. Examples of the cut-off values include $1.421 \times 10^{-2} \leq$ for 5A4CR, $< 6.494$ for ALA, $0.1433 \leq$ for ASP, $< 8.630 \times 10^{-3}$ for CSSG, $< 6.009 \times 10^{-3}$ for GLC3P, $< 2.366$ for HIS, $< 2.748$ for ILE, $< 6.413$ for LEU, $< 8.030 \times 10^{-2}$ for N6TLY, $2.915 \times 10^{-2} \leq$ for N6ALY, $< 6.767 \times 10^{-2}$ for OCTA, $2.380 \times 10^{-3} \leq$ for TUCA, and $< 2.137$ for THR.

In order to determine sensitivity to the targeted anti-cancer agent, specifically, determine whether or not to have PD after treatment with the anti-cancer agent, using ASP, HIS, N6ALY and TUCA, the amounts, for example, concentrations, of ASP, HIS, N6ALY and TUCA in a biological sample derived from a cancer patient may be measured at a stage before administration of the anti-cancer agent, and the predetermined numeric values according to the measurement results may be assigned to the expression (2):

$$p = \frac{1}{1+e^{(0.8105+(ASP)+(HIS)+(N6ALY)+(TUCA))}} \qquad (2)$$

wherein ASP represents -1.0820 when a measurement result about ASP is equal to or more than a cut-off value, and represents 1.0820 when the measurement result is less than the cut-off value; HIS represents 1.7717 when a measurement result about HIS is equal to or more than a cut-off value, and represents -1.7717 when the measurement result is less than the cut-off value; N6ALY represents -1.5499 when a measurement result about N6ALY is equal to or more than a cut-off value, and represents 1.5499 when the measurement result is less than the cut-off value; and TUCA represents -0.7905 when a measurement result about TUCA is equal to or more than a cut-off value, and represents 0.7905 when the measurement result is less than the cut-off value.

The cut-off value of each substance is 0.1433 for ASP, 2.366 for HIS, $2.915 \times 10^{-2}$ for N6ALY, and $2.380 \times 10^{-3}$ for TUCA.

p calculated according to the expression (2) represents a probability that the targeted cancer patient does not respond to treatment with the targeted anti-cancer agent at all and thus has PD. When p is 0.5 or more, cancer in the cancer patient can be determined to be not sensitive to the targeted anti-cancer agent, i.e., the cancer patient can be determined to have PD after treatment with the targeted anti-cancer agent. Therefore, these markers for determining anti-cancer agent sensitivity can be used as markers for predicting PD for circumventing the continuation of the treatment of patients who cannot be expected to receive therapeutic effects, and giving priority to another treatment. On the other hand, when p is less than 0.5, cancer in the cancer patient can be determined to be sensitive to the targeted anti-cancer agent, i.e., the cancer patient can be determined to have no PD aftertreatment with the targeted anti-cancer agent. If the cancer patient has sensitivity to the targeted anti-cancer agent, the therapeutic effects of the anti-cancer agent, such as the control of tumor or the suppression of disease progression, can be expected. Thus, the marker for determining anti-cancer agent sensitivity according to the present invention can be used as a marker for circumventing the continuation of the treatment of patients who cannot be expected to receive therapeutic effects, and giving priority to another treatment, and in addition, can also be used as a marker for continuing the treatment of patients who can be expected to receive therapeutic effects.

In order to determine a total tumor size before the start of treatment of a cancer patient using CSSG, the amount, for example, concentration, of CSSG in a biological sample derived from a cancer patient may be measured before administration of the anti-cancer agent, and the measurement result may be assigned to the expression (3). In this context, the cancer patient is preferably a patient refractory or intolerant to FOLFOX therapy combined with bevacizumab. In this case, a total tumor size before the start of secondary treatment of the cancer patient can be predicted.

$$\texttt{Total tumor size (mm) = 111.5 - 1864.9} \times \texttt{CSSG ... (3)}$$

wherein CSSG represents the concentration of CSSG.

One or more molecules selected from the group consisting of 3IND, ALA, ASP, CITR, CREAT, CSSG, GABA, GUAA, HIS, HYPRO, METSF, N6TLY, N8ASR and SER can be used as a marker for predicting prognosis for treatment with the targeted anti-cancer agent. Among these markers for predicting prognosis, ALA, ASP, CSSG, HIS, METSF, N6TLY and SER which also serve as markers for predicting PR and/or PD are preferred. In order to predict prognosis for treatment with the targeted anti-cancer agent using one or more molecules selected from the group consisting of 3IND, ALA, ASP, CITR, CREAT, CSSG, GABA, GUAA, HIS, HYPRO, METSF, N6TLY, N8ASR and SER, the amount, for example, concentration, of one or more molecules selected from the group consisting of 3IND, ALA, ASP, CITR, CREAT, CSSG, GABA, GUAA, HIS, HYPRO, METSF, N6TLY, N8ASR and SER in a biological sample derived from a cancer patient may be measured at a stage before administration of the anti-cancer agent. A higher concentration of one or more molecules selected from the group consisting of 3IND, ALA, CITR, CREAT, CSSG, GABA, GUAA, HIS, HYPRO, METSF, N6TLY and SER is indicative of better prognosis. For example, when the concentration has a concentration confirmed to be higher than the predetermined control level, good prognosis can be predicted as compared with when the concentration has a concentration confirmed to be lower than the predetermined control level. On the other hand, a lower concentration of one or more molecules selected from the group consisting of ASP and N8ASR is indicative of better prognosis. For example, when the concentration has a concentration confirmed to be lower than the predetermined control level, good prognosis can be predicted as compared with when the concentration has a concentration confirmed to be higher than the predetermined control level. The prediction of prognosis can be indicated by the length of a progression-free survival (PFS), an overall survival (OS), a disease-free survival (DFS), or the like, and is particularly preferably indicated by OS.

Examples of the control level include cut-off values of OS. Examples of the cut-off values include $1.050 \times 10^{-1}$ for 3IND, 5.1960 for ALA, $1.433 \times 10^{-1}$ for ASP, $4.660 \times 10^{-1}$ for CITR, 1.0399 for CREAT, $1.430 \times 10^{-2}$ for CSSG, $5.450 \times 10^{-2}$ for GABA, $5.500 \times 10^{-2}$ for GUAA, 2.2409 for HIS, $1.890 \times 10^{-1}$ for HYPRO, $9.1900 \times 10^{-2}$ for METSF, $8.401 \times 10^{-2}$ for N6TLY, $8.401 \times 10^{-2}$ for N8ASR, and 0.1890 for SER.

In order to determine sensitivity to the targeted anti-cancer agent, specifically, determine whether or not to have PR after treatment with the anti-cancer agent, using one or more molecules selected from the group consisting of CREAT, CSSG, METSF, QUINA and VAL, the amount, for example, concentration, of one or more molecules selected from the group consisting of CREAT, CSSG, METSF, QUINA and VAL in a biological sample derived from a cancer patient may be measured after implementation of one cycle of treatment with the anti-cancer agent. When the concentration of one or more molecules selected from the group consisting of CREAT, CSSG, METSF and VAL has a concentration confirmed to be higher than the predetermined control level, cancer in the cancer patient can be determined to be sensitive to the targeted anti-cancer agent, i.e., the cancer patient can be determined to have PR after treatment with the targeted anti-cancer agent. When the concentration of QUINA has a concentration confirmed to be lower than the predetermined control level, cancer in the cancer patient can be determined to be sensitive to the targeted anti-cancer agent, i.e., the cancer patient can be determined to have PR after treatment with the targeted anti-cancer agent. Accordingly, these markers for determining anti-cancer agent sensitivity can be used as markers for predicting PR for aggressively continuing the treatment of patients who can be expected to receive therapeutic effects. On the other hand, when the concentration of one or more molecules selected from the group consisting of CREAT, CSSG, METSF and VAL has a concentration confirmed to be lower than the predetermined control level, or when the concentration of QUINA has a concentration confirmed to be higher than the predetermined control level, cancer in the cancer patient can be determined to be not sensitive to the targeted anti-cancer agent, i.e., the cancer patient can be determined to have no PR after treatment with the targeted anti-cancer agent. If the cancer patient has no sensitivity to the targeted anti-cancer agent, the control of tumor with the anti-cancer agent cannot be expected. In the case of performing or continuing the administration of such an anti-cancer agent which cannot be expected to have drug efficacy, there is a fear on the progression of cancer or increase in adverse effects. Thus, the marker for determining anti-cancer agent sensitivity according to the present invention can be used as a marker for aggressively continuing the treatment of patients who can be expected to receive therapeutic effects, and in addition, can also be used as a marker for circumventing the progression of cancer and increase in adverse effects associated with the continuous administration of an anti-cancer agent which cannot be expected to have drug efficacy.

Examples of the control level include cut-off values of PR. Examples of the cut-off values include $1.2163 \leq$ for CREAT, $1.965 \times 10^{-2} \leq$ for CSSG, $5.060 \times 10^{-2} \leq$ for METSF, $< 1.150 \times 10^{-2}$ for QUINA, and $7.6718 \leq$ for VAL.

In order to determine sensitivity to the targeted anti-cancer agent, specifically, determine whether or not to have PR after treatment with the anti-cancer agent, using CREAT, CSSG and QUINA, the amounts, for example, concentrations, of CREAT, CSSG and QUINA in a biological sample derived from a cancer patient may be measured at a stage after implementation of one cycle of treatment with the anti-cancer agent, and the predetermined numeric values according to the measurement results may be assigned to the expression (4):

$$p = \frac{1}{1+e^{(9.0171-(CREAT)-(CSSG)-(QUINA))}} \qquad (4)$$

wherein CREAT represents 1.2906 when a measurement result about CREAT is equal to or more than a cut-off value, and represents -1.2906 when the measurement result is less than the cut-off value; CSSG represents 1.7703 when a measurement result about CSSG is equal to or more than a cut-off value, and represents -1.7703 when the measurement result is less than the cut-off value; and QUINA represents -8.6990 when a measurement result about QUINA is equal to or more than a cut-off value, and represents 8.6990 when the measurement result is less than the cut-off value.

The cut-off value of each substance is 1.2163 for CREAT, $1.965 \times 10^{-2}$ for CSSG, and $1.150 \times 10^{-2}$ for QUINA.

p calculated according to the expression (4) represents a probability that the targeted cancer patient exhibits tumor shrinkage by treatment with the targeted anti-cancer agent and thus has PR. When p exceeds 0.5, cancer in the cancer patient can be determined to be sensitive to the targeted anti-cancer agent, i.e., the cancer patient can be determined to have PR after treatment with the targeted anti-cancer agent. Therefore, these markers for determining anti-cancer agent sensitivity can be used as markers for predicting PR for aggressively continuing the treatment of patients who can be expected to receive therapeutic effects. On the other hand, when p is 0.5 or less, cancer in the cancer patient can be determined to be not sensitive to the targeted anti-cancer agent, i.e., the cancer patient can be determined to have no PR after treatment with the targeted anti-cancer agent. If the cancer patient has no sensitivity to the targeted anti-cancer agent, the control of tumor with the anti-cancer agent cannot be expected. In the case of performing or continuing the administration of such an anti-cancer agent which cannot be expected to have drug efficacy, there is a fear on the progression of cancer or increase in adverse effects. Thus, the marker for determining anti-cancer agent sensitivity according to the present invention can be used as a marker for aggressively continuing the treatment of patients who can be expected to receive therapeutic effects, and in addition, can also be used as a marker for circumventing the progression of cancer and increase in adverse effects associated with the continuous administration of an anti-cancer agent which cannot be expected to have drug efficacy.

In order to determine sensitivity to the targeted anti-cancer agent, specifically, determine whether or not to have PD after treatment with the anti-cancer agent, using one or more molecules selected from the group consisting of 5A4CR, CSSG, DECNA, GLC3P, HYPTA and N8ASR, the amount, for example, concentration, of one or more molecules selected from the group consisting of 5A4CR, CSSG, DECNA, GLC3P, HYPTA and N8ASR in a biological sample derived from a cancer patient may be measured at a stage after implementation of one cycle of treatment with the anti-cancer agent. When the concentration of one or more molecules selected from the group consisting of 5A4CR and N8ASR has a concentration confirmed to be higher than the predetermined control level, cancer in the cancer patient can be determined to be not sensitive to the targeted anti-cancer agent, i.e., the cancer patient can be determined to have PD after treatment with the targeted anti-cancer agent. When the concentration of one or more molecules selected from the group consisting of CSSG, DECNA, GLC3P and HYPTA has a concentration confirmed to be lower than the predetermined control level, cancer in the cancer patient can be determined to be not sensitive to the targeted anti-cancer agent, i.e., the cancer patient can be determined to have PD after treatment with the targeted anti-cancer agent. Accordingly, these markers for determining anti-cancer agent sensitivity can be used as markers for predicting PD for circumventing the continuation of the treatment of patients who cannot be expected to receive therapeutic effects, and giving priority to another treatment. On the other hand, when the concentration of one or more molecules selected from the group consisting of 5A4CR and N8ASR has a concentration confirmed to be lower than the predetermined control level, or when the concentration of one or more molecules selected from the group consisting of CSSG, DECNA, GLC3P and HYPTA has a concentration confirmed to be higher than the predetermined control level, cancer in the cancer patient can be determined to be sensitive to the targeted anti-cancer agent, i.e., the cancer patient can be determined to have no PD after treatment with the targeted anti-cancer agent. If the cancer patient has sensitivity to the targeted anti-cancer agent, the therapeutic effects of the anti-cancer agent, such as the control of tumor or the suppression of disease progression, can be expected. Thus, the marker for determining anti-cancer agent sensitivity according to the present invention can be used as a marker for circumventing the continuation of the treatment of patients who cannot be expected to receive therapeutic effects, and giving priority to another treatment, and in addition, can also be used as a marker for continuing the treatment of patients who can be expected to receive therapeutic effects.

Examples of the control level include cut-off values of PD. Examples of the cut-off values include $1.413 \times 10^{-2}$ ≤ for 5A4CR, $< 1.030 \times 10^{-2}$ for CSSG, $< 1.080 \times 10^{-1}$ for DECNA, $< 4.586 \times 10^{-1}$ for GLC3P, $< 1.240 \times 10^{-2}$ for HYPTA, and $1.122 \times 10^{-2}$ ≤ for N8ASR.

In order to determine sensitivity to the targeted anti-cancer agent, specifically, determine whether or not to have PD after treatment with the anti-cancer agent, using 5A4CR, CSSG, DECNA, HYPTA and N8ASR, the amounts, for example, concentrations, of 5A4CR, CSSG, DECNA, HYPTA and N8ASR in a biological sample derived from a cancer patient may be measured at a stage after implementation of one cycle of treatment with the anti-cancer agent, and the predetermined numeric values according to the measurement results may be assigned to the expression (5) :

$$p = \frac{1}{1+e^{(0.8232+(5A4CR)+(CSSG)+(DECNA)+(HYPTA)+(N8ASR))}} \qquad (5)$$

wherein 5A4CR represents -0.9300 when a measurement result about 5A4CR is equal to or more than a cut-off value, and represents 0.9300 when the measurement result is less than the cut-off value; CSSG represents 1.2325 when a measurement result about CSSG is equal to or more than a cut-off value, and represents -1.2325 when the measurement result is less than the cut-off value; DECNA represents 1.3052 when a measurement result about DECNA is equal to or more than a cut-off value, and represents -1.3052 when the measurement result is less than the cut-off value; HYPTA represents 0.8020 when a measurement result about HYPTA is equal to or more than a cut-off value, and represents -0.8020 when the measurement result is less than the cut-off value; and N8ASR represents -1.4363 when a measurement result about N8ASR is equal to or more than a cut-off value, and represents 1.4363 when the measurement result is less than the cut-off value.

The cut-off value of each substance is $1.413 \times 10^{-2}$ for 5A4CR, $1.030 \times 10^{-2}$ for CSSG, $1.080 \times 10^{-1}$ for DECNA, $1.240 \times 10^{-2}$ for HYPTA, and $1.122 \times 10^{-2}$ for N8ASR.

p calculated according to the expression (5) represents a probability that the targeted cancer patient does not respond to the targeted anti-cancer agent at all and thus has PD. When p exceeds 0.5, cancer in the cancer patient can be determined to be not sensitive to the targeted anti-cancer agent, i.e., the cancer patient can be determined to have PD after treatment with the targeted anti-cancer agent. Therefore, these markers for determining anti-cancer agent sensitivity can be used as markers for predicting PD for circumventing the continuation of the treatment of patients who cannot be expected to receive therapeutic effects, and giving priority to another treatment. On the other hand, when p is 0.5 or less, cancer in the cancer patient can be determined to be sensitive to the targeted anti-cancer agent, i.e., the cancer patient can be determined to have no PD after treatment with the targeted anti-cancer agent. If the cancer patient has sensitivity to the targeted anti-cancer agent, the therapeutic effects of the anti-cancer agent, such as the control of tumor or the suppression of disease progression, can be expected. Thus, the marker for determining anti-cancer agent sensitivity according to the present invention can be used as a marker for circumventing the continuation of the treatment of patients who cannot be expected to receive therapeutic effects, and giving priority to another treatment, and in addition, can also be used as a marker for continuing the treatment of patients who can be expected to receive therapeutic effects.

In order to determine sensitivity to the targeted anti-cancer agent, specifically, determine whether or not to have PR after treatment with the anti-cancer agent, using one or more molecules selected from the group consisting of 4OVAL, ALA, BENZA, CREAT, CSSG, LYS and SARCO, the amount, for example, concentration, of one or more molecules selected from the group consisting of 4OVAL, ALA, BENZA, CREAT, CSSG, LYS and SARCO in a biological sample derived from a cancer patient may be measured after implementation of two cycles of treatment with the anti-cancer agent. When the concentration has a concentration confirmed to be higher than the predetermined control level, cancer in the cancer patient can be determined to be sensitive to the targeted anti-cancer agent, i.e., the cancer patient can be determined to have PR after treatment with the targeted anti-cancer agent. Therefore, these markers for determining anti-cancer agent sensitivity can be used as markers for predicting PR for aggressively continuing the treatment of patients who can be expected to receive therapeutic effects. On the other hand, when the concentration has a concentration confirmed to be lower than the predetermined control level, cancer in the cancer patient can be determined to be not sensitive to the targeted anti-cancer agent, i.e., the cancer patient can be determined to have no PR after treatment with the targeted anti-cancer agent. If the cancer patient has no sensitivity to the targeted anti-cancer agent, the control of tumor with the anti-cancer agent cannot be expected. In the case of performing or continuing the administration of such an anti-cancer agent which cannot be expected to have drug efficacy, there is a fear on the progression of cancer or increase in adverse effects. Thus, the marker for determining anti-cancer agent sensitivity according to the present invention can be used as a marker for aggressively continuing the treatment of patients who can be expected to receive therapeutic effects, and in addition, can also be used as a marker for circumventing the progression of cancer and increase in adverse effects associated with the continuous administration of an anti-cancer agent which cannot be expected to have drug efficacy.

Examples of the control level include cut-off values of PR. Examples of the cut-off values include $2.949 \times 10^{-2}$ ≤ for 4OVAL, 7.9605 ≤ for ALA, $1.367 \times 10^{-1}$ ≤ for BENZA, $6.609 \times 10^{-1}$ ≤ for CREAT, $1.233 \times 10^{-2}$ ≤ for CSSG, 4.9765 ≤ for LYS, and $4.548 \times 10^{-2}$ ≤ for SARCO.

In order to determine sensitivity to the targeted anti-cancer agent, specifically, determine whether or not to have PR after treatment with the anti-cancer agent, using 4OVAL, BENZA and LYS, the amounts, for example, concentrations, of 4OVAL, BENZA and LYS in a biological sample derived from a cancer patient may be measured at a stage after implementation of two cycles of treatment with the anti-cancer agent, and the predetermined numeric values according to the measurement results may be assigned to the expression (6):

$$p = \frac{1}{1+e^{(1.5237-(4OVAL)-(BENZA)-(LYS))}} \qquad (6)$$

wherein 4OVAL represents 1.2359 when a measurement result about 4OVAL is equal to or more than a cut-off value, and represents -1.2359 when the measurement result is less than the cut-off value; BENZA represents 1.1105 when a measurement result about BENZA is equal to or more than a cut-off value, and represents -1.1105 when the measurement result is less than the cut-off value; and LYS represents 0.8767 when a measurement result about LYS is equal to or more than a cut-off value, and represents -0.8767 when the measurement result is less than the cut-off value.

The cut-off value of each substance is $2.949 \times 10^{-2}$ for 4OVAL, $1.367 \times 10^{-1}$ for BENZA, and 4.9765 for LYS.

p calculated according to the expression (6) represents a probability that the targeted cancer patient exhibits tumor shrinkage by treatment with the targeted anti-cancer agent and thus has PR. When p exceeds 0.5, cancer in the cancer patient can be determined to be sensitive to the targeted anti-cancer agent, i.e., the cancer patient can be determined to have PR after treatment with the targeted anti-cancer agent. Therefore, these markers for determining anti-cancer agent sensitivity can be used as markers for predicting PR for aggressively continuing the treatment of patients who can be expected to receive therapeutic effects. On the other hand, when p is 0.5 or less, cancer in the cancer patient can be determined to be not sensitive to the targeted anti-cancer agent, i.e., the cancer patient can be determined to have no PR after treatment with the targeted anti-cancer agent. If the cancer patient has no sensitivity to the targeted anti-cancer agent, the control of tumor with the anti-cancer agent cannot be expected. In the case of performing or continuing the administration of such an anti-cancer agent which cannot be expected to have drug efficacy, there is a fear on the progression of cancer or increase in adverse effects. Thus, the marker for determining anti-cancer agent sensitivity according to the present invention can be used as a marker for aggressively continuing the treatment of patients who can be expected to receive therapeutic effects, and in addition, can also be used as a marker for circumventing the progression of cancer and increase in adverse effects associated with the continuous administration of an anti-cancer agent which cannot be expected to have drug efficacy.

In order to determine sensitivity to the targeted anti-cancer agent, specifically, determine whether or not to have PD after treatment with the anti-cancer agent, using one or more molecules selected from the group consisting of 3IND, 4OVAL, 5A4CR, ALA, CSSG, GABB and TMNO, the amount, for example, concentration, of one or more molecules selected from the group consisting of 3IND, 4OVAL, 5A4CR, ALA, CSSG, GABB and TMNO in a biological sample derived from a cancer patient may be measured at a stage after implementation of two cycles of treatment with the anti-cancer agent. When the concentration of one or more molecules selected from the group consisting of 5A4CR and GABB has a concentration confirmed to be higher than the predetermined control level, cancer in the cancer patient can be determined to be not sensitive to the targeted anti-cancer agent, i.e., the cancer patient can be determined to have PD after treatment with the targeted anti-cancer agent. When the concentration of one or more molecules selected from the group consisting of 3IND, 4OVAL, ALA, CSSG and TMNO has a concentration confirmed to be lower than the predetermined control level, cancer in the cancer patient can be determined to be not sensitive to the targeted anti-cancer agent, i.e., the cancer patient can be determined to have PD after treatment with the targeted anti-cancer agent. Accordingly, these markers for determining anti-cancer agent sensitivity can be used as markers for predicting PD for circumventing the continuation of the treatment of patients who cannot be expected to receive therapeutic effects, and giving priority to another treatment. On the other hand, when the concentration of one or more molecules selected from the group consisting of 5A4CR and GABB has a concentration confirmed to be lower than the predetermined control level, or when the concentration of one or more molecules selected from the group consisting of 3IND, 4OVAL, ALA, CSSG and TMNO has a concentration confirmed to be higher than the predetermined control level, cancer in the cancer patient can be determined to be sensitive to the targeted anti-cancer agent, i.e., the cancer patient can be determined to have no PD after treatment with the targeted anti-cancer agent. If the cancer patient has sensitivity to the targeted anti-cancer agent, the therapeutic effects of the anti-cancer agent, such as the control of tumor or the suppression of disease progression, can be expected. Thus, the marker for determining anti-cancer agent sensitivity according to the present invention can be used as a marker for circumventing the continuation of the treatment of patients who cannot be expected to receive therapeutic effects, and giving priority to another treatment, and in addition, can also be used as a marker for continuing the treatment of patients who can be expected to receive therapeutic effects.

Examples of the control level include cut-off values of PD. Examples of the cut-off values include $< 6.129 \times 10^{-2}$ for 3IND, $< 1.346 \times 10^{-2}$ for 4OVAL, $2.052 \times 10^{-2} \leq$ for 5A4CR, $< 7.3693$ for ALA, $< 1.273 \times 10^{-2}$ for CSSG, $5.117 \times 10^{-2} \leq$ for GABB, and $< 2.689 \times 10^{-1}$ for TMNO.

In order to determine sensitivity to the targeted anti-cancer agent, specifically, determine whether or not to have PD after treatment with the anti-cancer agent, using 3IND, 5A4CR, CSSG, GABB and TMNO, the amounts, for example, concentrations, of 3IND, 5A4CR, CSSG, GABB and TMNO in a biological sample derived from a cancer patient may be measured at a stage after implementation of two cycles of treatment with the anti-cancer agent, and the predetermined numeric values according to the measurement results may be assigned to the expression (7):

$$p = \frac{1}{1+e^{(2.4054+(3IND)+(5A4CR)+(CSSG)+(GABB)+(TMNO))}} \qquad (7)$$

wherein 3IND represents 1.4853 when a measurement result about 3IND is equal to or more than a cut-off value, and represents -1.4853 when the measurement result is less than the cut-off value; 5A4CR represents -1.0356 when a measurement result about 5A4CR is equal to or more than a cut-off value, and represents 1.0356 when the measurement result is less than the cut-off value; CSSG represents 1.1004 when a measurement result about CSSG is equal to or more than a cut-off value, and represents -1.1004 when the measurement result is less than the cut-off value; GABB represents -1.2343 when a measurement result about GABB is equal to or more than a cut-off value, and represents 1.2343 when the measurement result is less than the cut-off value; and TMNO represents 0.9992 when a measurement result about TMNO is equal to or more than a cut-off value, and represents -0.9992 when the measurement result is less than the cut-off value.

The cut-off value of each substance is $6.129 \times 10^{-2}$ for 3IND, $2.052 \times 10^{-2}$ for 5A4CR, $1.273 \times 10^{-2}$ for CSSG, $5.117 \times 10^{-2}$ for GABB, and $2.689 \times 10^{-1}$ for TMNO.

p calculated according to the expression (7) represents a probability that the targeted cancer patient does not respond to the targeted anti-cancer agent at all and thus has PD. When p exceeds 0.5, cancer in the cancer patient can be determined to be not sensitive to the targeted anti-cancer agent, i.e., the cancer patient can be determined to have PD after treatment with the targeted anti-cancer agent. Therefore, these markers for determining anti-cancer agent sensitivity can be used as markers for predicting PD for circumventing the continuation of the treatment of patients who cannot be expected to receive therapeutic effects, and giving priority to another treatment. On the other hand, when p is 0.5 or less, cancer in the cancer patient can be determined to be sensitive to the targeted anti-cancer agent, i.e., the cancer patient can be determined to have no PD after treatment with the targeted anti-cancer agent. If the cancer patient has sensitivity to the targeted anti-cancer agent, the therapeutic effects of the anti-cancer agent, such as the control of tumor or the suppression of disease progression, can be expected. Thus, the marker for determining anti-cancer agent sensitivity according to the present invention can be used as a marker for circumventing the continuation of the treatment of patients who cannot be expected to receive therapeutic effects, and giving priority to another treatment, and in addition, can also be used as a marker for continuing the treatment of patients who can be expected to receive therapeutic effects.

One or more molecules selected from the group consisting of 1MNA, 2H4MP, 3IND, 3MHIS, 5A4CR, ASP, CHCA, CSSG, GABA, HIPA, HYPX, MUCA, N8ASR and TAUR can be used as a marker for predicting prognosis for treatment with the targeted anti-cancer agent. Among these markers for predicting prognosis, 3IND, 5A4CR, CSSG and N8ASR which also serve as markers for predicting PR and/or PD are preferred. In order to predict prognosis for treatment with the targeted anti-cancer agent using one or more molecules selected from the group consisting of 1MNA, 2H4MP, 3IND, 3MHIS, ASP, CHCA, CSSG, GABA, HIPA, HYPX and N8ASR, the amount, for example, concentration, of one or more molecules selected from the group consisting of 1MNA, 2H4MP, 3IND, 3MHIS, ASP, CHCA, CSSG, GABA, HIPA, HYPX and N8ASR in a biological sample derived from a cancer patient may be measured after implementation of one cycle of treatment with the anti-cancer agent. A higher concentration of one or more molecules selected from the group consisting of 2H4MP, 3IND, 3MHIS, CHCA, CSSG, GABA and HIPA is indicative of better prognosis. For example, when the concentration has a concentration confirmed to be higher than the predetermined control level, good prognosis can be predicted as compared with when the concentration has a concentration confirmed to be lower than the predetermined control level. On the other hand, a lower concentration of one or more molecules selected from the group consisting of 1MNA, ASP, HYPX and N8ASR is indicative of better prognosis. For example, when the concentration has a concentration confirmed to be lower than the predetermined control level, good prognosis can be predicted as compared with when the concentration has a concentration confirmed to be higher than the predetermined control level. In order to predict prognosis for treatment with the targeted anti-cancer agent using one or more molecules selected from the group consisting of 2H4MP, 3IND, 5A4CR, GABA, MUCA and TAUR, the amount, for example, concentration, of one or more molecules selected from the group consisting of 2H4MP, 3IND, 5A4CR, GABA, MUCA and TAUR in a biological sample derived from a cancer patient may be measured after implementation of two cycles treatment with the anti-cancer agent. A higher concentration of one or more molecules selected from the group consisting of 2H4MP, 3IND, GABA and MUCA is indicative of better prognosis. For example, when the concentration has a concentration confirmed to be higher than the predetermined control level, good prognosis can be predicted as compared with when the concentration has a concentration confirmed to be lower than the predetermined control level. On the other hand, a lower concentration of one or more molecules selected from the group consisting of 5A4CR and TAUR is indicative of better prognosis. For example, when the concentration has a concentration confirmed to be lower than the predetermined control level, good prognosis can be predicted as compared with when the concentration has a concentration confirmed to be higher than the predetermined control level. The prediction of prognosis can be indicated by the length of a progression-free survival (PFS), an overall survival (OS), a disease-free survival (DFS), or the like, and is preferably indicated by OS, particularly preferably residual OS after implementation of one cycle or two cycles of treatment with the anti-cancer agent.

Examples of the control level include cut-off values of OS. Examples of the cut-off values include 0 for 1MNA, $3.126 \times 10^{-3}$ for 2H4MP after implementation of one cycle of treatment with the anti-cancer agent and $5.242 \times 10^{-3}$ for 2H4MP after implementation of two cycles of treatment with the anti-cancer agent, $1.050 \times 10^{-1}$ for 3IND after implementation of one cycle of treatment with the anti-cancer agent and $3.820 \times 10^{-2}$ for 3IND after implementation of two cycles of treatment with the anti-cancer agent, $1.031 \times 10^{-1}$ for 3MHIS, 0 for 5A4CR, $1.433 \times 10^{-1}$ for ASP, $1.069 \times 10^{-2}$ for CHCA, $9.367 \times 10^{-3}$ for CSSG, $3.624 \times 10^{-2}$ for GABA after implementation of one cycle of treatment with the anti-cancer agent and $4.354 \times 10^{-2}$ for GABA after implementation of two cycles of treatment with the anti-cancer agent, $3.884 \times 10^{-2}$ for HIPA, $5.846 \times 10^{-2}$ for HYPX, $1.025 \times 10^{-2}$ for MUCA, $1.122 \times 10^{-2}$ for N8ASR, and $4.661 \times 10^{-1}$ for TAUR.

For carrying out the method of the present invention for determining anti-cancer agent sensitivity or a total tumor size before the start of treatment with the anti-cancer agent, it is preferred to use a kit comprising a protocol for measuring one or more molecules selected from the group consisting of 5A4CR, ALA, ASP, CYS, CSSG, GLC3P, HIS, ILE, LEU, LYS, METSF, N6TLY, N6ALY, OCTA, SER, TUCA, THR, TRP, TYR and VAL in a specimen. For carrying out the method of the present invention for predicting prognosis before the start of treatment with the anti-cancer agent, it is preferred to use a kit comprising a protocol for measuring one or more molecules selected from the group consisting of 3IND, ALA, ASP, CITR, CREAT, CSSG, GABA, GUAA, HIS, HYPRO, METSF, N6TLY, N8ASR and SER in a specimen. The kit comprises a reagent for measuring these metabolite substances, and a protocol (a method for using the measurement reagent, and criteria for determining the presence or absence of anti-cancer agent sensitivity, etc.). The criteria include standard concentrations of these metabolite substances, their concentrations which are confirmed to be high, their concentrations which are confirmed to be low, factors which influence measurement results, the degree of the influence, etc. These concentrations can be set for each targeted anti-cancer agent. Determination or prediction can be performed as described above using the criteria.

For carrying out the method of the present invention for determining anti-cancer agent sensitivity in an early stage after the start of treatment with the anti-cancer agent, it is preferred to use a kit comprising a protocol for measuring one or more molecules selected from the group consisting of 3IND, 4OVAL, 5A4CR, ALA, BENZA, CREAT, CSSG, DECNA, GABB, GLC3P, HYPTA, LYS, METSF, N8ASR, QUINA, SARCO, TMNO and VAL in a specimen. For carrying out the method of the present invention for predicting prognosis in an early stage after the start of treatment with the anti-cancer agent, it is preferred to use a kit comprising a protocol for measuring one or more molecules selected from the group consisting of 1MNA, 2H4MP, 3IND, 3MHIS, 5A4CR, ASP, CHCA, CSSG, GABA, HIPA, HYPX, MUCA, N8ASR and TAUR in a specimen. The kit comprises a reagent for measuring these metabolite substances, and a protocol (a method for using the measurement reagent, and criteria for determining the presence or absence of anti-cancer agent sensitivity, etc.). The criteria include standard concentrations of these metabolite substances, their concentrations which are confirmed to be high, their concentrations which are confirmed to be low, factors which influence measurement results, the degree of the influence, etc. These concentrations can be set for each targeted anti-cancer agent. Determination or prediction can be performed as described above using the criteria.

An anti-cancer agent sensitivity-enhancing agent can be selected through screening by employing, as an index, expression variation of one or more molecules selected from the group consisting of 5A4CR, ALA, ASP, CYS, CSSG, GLC3P, HIS, ILE, LEU, LYS, METSF, N6TLY, N6ALY, OCTA, SER, TUCA, THR, TRP, TYR, VAL, 3IND, CITR, CREAT, GABA, GUAA, HYPRO and N8ASR in a biological sample derived from a cancer cell line or a cancer-bearing animal in the presence of the anti-cancer agent.

Specifically, a sensitivity-enhancing agent for the anti-cancer agent can be selected through screening by adding or administering the anti-cancer agent and a test substance to a cancer cell line or a cancer-bearing animal, and measuring a concentration of one or more molecules selected from the group consisting of 5A4CR, ALA, ASP, CYS, CSSG, GLC3P, HIS, ILE, LEU, LYS, METSF, N6TLY, N6ALY, OCTA, SER, TUCA, THR, TRP, TYR, VAL, 3IND, CITR, CREAT, GABA, GUAA, HYPRO and N8ASR in a biological sample derived from the cancer cell line or the cancer-bearing animal; and selecting a test substance enhancing the sensitivity of the cancer cell line or the cancer-bearing animal to the anti-cancer agent on the basis of variation in the concentration.

For example, as for one or more molecules selected from the group consisting of ALA, CYS, CSSG, GLC3P, HIS, ILE, LEU, LYS, METSF, N6TLY, OCTA, SER, THR, TRP, TYR, VAL, 3IND, CITR, CREAT, GABA, GUAA and HYPRO, the anti-cancer agent sensitivity-enhancing agent can be selected through screening by employing, as an index, expression variation of the metabolite substances, specifically, increase in their concentrations, in the presence of the anti-cancer agent. Specifically, substances which increase the concentrations of these metabolite substances *in vitro* or *in vivo* enhance anti-cancer agent sensitivity. For example, substances which increase the concentrations of these metabolite substances *in vitro* in the presence of the anti-cancer agent in each cancer cell line are substances enhancing the sensitivity of the cancer cell line to the anti-cancer agent (anti-cancer agent sensitivity-enhancing agents). Also, substances which increase the concentrations of these metabolite substances *in vivo* before or after administration of the anti-cancer agent to the cancer-bearing animal are substances enhancing the sensitivity of the cancer-bearing animal to the anti-cancer agent (anti-cancer agent sensitivity-enhancing agents).

For example, as for one or more molecules selected from the group consisting of 5A4CR, ASP, N6ALY, TUCA and N8ASR, the anti-cancer agent sensitivity-enhancing agent can be selected through screening by employing, as an index, expression variation of the metabolite substances, specifically, decrease in their concentrations, in the presence of the anti-cancer agent. Specifically, substances which decrease the concentrations of these metabolite substances *in vitro* or *in vivo* enhance anti-cancer agent sensitivity. For example, substances which decrease the concentrations of these metabolite substances *in vitro* in the presence of the anti-cancer agent in each cancer cell line are substances enhancing the sensitivity of the cancer cell line to the anti-cancer agent (anti-cancer agent sensitivity-enhancing agents). Also, substances which decrease the concentrations of these metabolite substances *in vivo* before or after administration of the anti-cancer agent to the cancer-bearing animal are substances enhancing the sensitivity of the cancer-bearing animal to the anti-cancer agent (anti-cancer agent sensitivity-enhancing agents).

Concomitant use of the anti-cancer agent sensitivity-enhancing agent thus obtained with the anti-cancer agent whose sensitivity is to be enhanced drastically improves the therapeutic effects of the anti-cancer agent. A form for the combination of the anti-cancer agent sensitivity-enhancing agent and the anti-cancer agent whose sensitivity is to be enhanced may be one composition comprising both of these components or may be a combination of respective separate preparations. These components may be administered through different administration routes. In this context, the targeted anti-cancer agent used is an anti-cancer agent including irinotecan or SN-38 or a salt thereof, fluorouracil or a salt thereof, and levofolinate or a salt thereof. Examples of an additional anti-cancer agent for use in combination with this anti-cancer agent include, but are not particularly limited to, oxaliplatin, cyclophosphamide, ifosfamide, thiotepa, melphalan, busulfan, nimustine, ranimustine, dacarbazine, procarbazine, temozolomide, cisplatin, carboplatin, nedaplatin, methotrexate, pemetrexed, tegafur/uracil, doxifluridine, tegafur/gimeracil/oteracil, capecitabine, cytarabine, enocitabine, gemcitabine, 6-mercaptopurine, fludarabine, pentostatin, cladribine, hydroxyurea, doxorubicin, epirubicin, daunorubicin, idarubicine, pirarubicin, mitoxantrone, amrubicin, actinomycin D, bleomycine, pepleomycin, mitomycin C, aclarubicin, zinostatin, vincristine, vindesine, vinblastine, vinorelbine, paclitaxel, docetaxel, nogitecan, topotecan, etoposide, prednisolone, dexamethasone, tamoxifen, toremifene, medroxyprogesterone, anastrozole, exemestane, letrozole, rituximab, imatinib, gefitinib, gemtuzumab ozogamicin, bortezomib, erlotinib, cetuximab, bevacizumab, sunitinib, sorafenib, dasatinib, panitumumab, ramucirumab, aflibercept, asparaginase, tretinoin, arsenic trioxide, and salts thereof, and active metabolites thereof. Among them, an anti-angiogenic drug such as cetuximab or bevacizumab, or oxaliplatin is preferred, an anti-angiogenic drug is more preferred, and bevacizumab is particularly preferred.

Examples

Next, the present invention will be described in more detail with reference to Examples. However, the present invention is not limited by these examples by any means.

Example 1

(1) Method

(a) Reagent

Methanol for LC/MS (manufactured by Wako Pure Chemical Industries, Ltd.), chloroform for HPLC (manufactured by Wako Pure Chemical Industries, Ltd.), and reverse osmosis water (Direct-Q UV, manufactured by Merck Millipore) were used in dissolution and sample preparation.

The internal standard solutions for LC/MS (cation) used were Internal Standard Solution Compound C1 (ISC1) and Internal Standard Solution Compound C2 (ISC2). ISC1 contains 10 mM L-methionine sulfone (manufactured by Human Metabolome Technologies America Inc.) in an aqueous solution. ISC2 contains 10 mM L-arginine-$^{13}C_6$ hydrochloride (manufactured by Sigma-Aldrich Co. LLC), L-asparagine-$^{15}N_2$ monohydrate (manufactured by Cambridge Isotope Laboratories, Inc.), β-alanine-$^{13}C_3$,$^{15}N$ (manufactured by Sigma-Aldrich Co. LLC), and tubercidin (manufactured by Sigma-Aldrich Co. LLC) in an aqueous solution.

The internal standard solutions for LC/MS (anion) used were Internal Standard Solution Compound A1 (ISA1) and Internal Standard Solution compound A2 (ISA2). ISA1 contains 10 mM D-camphor-10-sulfonic acid sodium salt (manufactured by Human Metabolome Technologies America Inc.) in an aqueous solution. ISA2 contains 10 mM chloranilic acid (manufactured by Tokyo Chemical Industry Co., Ltd.) in an aqueous solution.

These internal standard solutions were used for standardizing signal intensity and adjusting a migration time. Also, ISC1 and ISA1 were used for calculating the relative concentration of each metabolite.

(b) Clinical sample

(b-1) Patient background

Serum samples were prospectively collected from patients registered in the phase II trial of FOLFIRI + bevacizumab therapy, as advanced colorectal cancer (ACRC) patients histologically confirmed to be refractory or intolerant to FOLFOX therapy. Among 89 patients registered in this trial, 82 patients on which anti-tumor effects were determinable on the basis of Response Evaluation Criteria in Solid Tumors Guideline (RECIST) 1.0 by the independent external review board of this trial were to be analyzed for metabolites for predicting effects. Criteria for registration (eligibility) in this trial are as follows.

- Age at the time of registration is equal to or over 20 years,
- Performance status (PS) of Eastern Corporative Oncology Group (ECOG) is 0 or 1,
- Histopathologically confirmed to have colorectal cancer,
- Incurable and unresectable advanced or recurrent case,
- Underwent FOLFOX or the like as pretreatment and became refractory or intolerant thereto,
- Pretreatment with CPT-11 is absent,
- Found to belong to a wild-type group or a heterozygous group by the genetic test of UGT1A1*28 and UGT1A1*6,
- Having a predicted survival period of 3 months or longer,
- Having no severe dysfunction in major organs, and
- A written informed consent with patient's own signature and date was obtained before registration in this trial as to enrollment in tests including a gene polymorphism test and proteome or metabolome analysis.

The detailed patient backgrounds and clinical trials in this trial are described in Suenaga M, et. al., BMC Cancer. (2015) 15: 176.

(b-2) Treatment of patient

All the patients underwent bolus administration of bevacizumab (BV) at 5 mg/kg, irinotecan (CPT-11) at 180 mg/m$^2$, levofolinate (l-LV) at 200 mg/m$^2$, and 5-FU at 400 mg/m$^2$ and subsequent intravenous administration of 5-FU at 2400 mg/m$^2$. This treatment was repeated every 2 weeks.

Up to 24 cycles were continued as study treatment unless there were disease progression, appearance of an adverse event due to which further study treatment must be discontinued, doctor's judgment, patient's rejection of continuation of study treatment, shift to curative or resectional surgery of tumor, etc.

(b-3) Evaluation of anti-tumor effect

The anti-tumor effects were evaluated on the basis of Response Evaluation Criteria in Solid Tumors Guideline (RECIST) 1.0 by the independent external review board.

A total tumor size before treatment was measured using an image taken with a computer topography or a magnetic resonance imaging within 1 month before registration. A total tumor size after the start of treatment was measured using images repetitively taken every 8 weeks in the same way as that before treatment.

(b-4) Collection of sample

A blood sample was collected within 2 weeks before the start of chemotherapy and 2 weeks after implementation of chemotherapy of each treatment cycle from the start of chemotherapy to the 8th treatment cycle, and then collected before next CPT-11 administration of a treatment cycle where diagnostic imaging was conducted.

The collected blood specimen was left at room temperature for 15 minutes for coagulation and then centrifuged at 3,000 rpm at 4°C for 30 minutes. Then, serum was transferred in equal amounts to four polypropylene tubes and immediately frozen with liquid nitrogen. All of these procedures were completed within 1 hour from blood collection. The serum sample was stored at -80°C until analysis.

(b-5) Preparation of sample

Sample preparation was performed in accordance with the previously reported method (J Proteome Res. 2003 Sep-Oct; 2 (5): 488-94; Metabolomics. 2010 Mar; 6 (1): 78-95; and Metabolomics. 2013 Apr; 9 (2): 444-453). The serum sample was thawed on ice, and 200 $\mu$L of the serum, the internal standard (10 $\mu$M ISA1 or ISC1), 2,000 $\mu$L of chloroform and 800 $\mu$L of reverse osmosis water were placed in a centrifugal tube containing 1,800 $\mu$L of methanol and mixed therewith. After vortexing, the mixture was centrifuged at 4,600 g at 4°C for 5 minutes. Then, 1,500 $\mu$L of the upper layer was transferred to a 5 kDa filter (manufactured by Merck Millipore) for the removal of proteins and centrifugally filtered at 9,100 g at 4°C for 2 to 4 hours. The filtrate was dried in a vacuum centrifuge. Immediately before CE-TOF MS analysis, the dried filtrate was dissolved in 50 $\mu$L of reverse osmosis water containing ISC2 or ISA2 (final concentration: 0.1 mM) on ice, placed in an analysis vial, centrifuged at 1,000 g at 4°C for 10 minutes, and subjected to analysis.

(c) Measurement of metabolite substance in sample by CE-TOF MS

All the samples were measured in duplicate. Metabolite substances having a mass number of 1,000 or smaller were comprehensively measured under cation measurement conditions using CE-Q-TOF MS or under anion measurement conditions using CE-TOF MS.

(c-1) Cationic metabolite substance measurement conditions

1) Measurement equipment

Agilent 6530 Accurate-Mass Q-TOF MS system equipped with Agilent 7100 CE system (manufactured by Agilent Technologies, Inc.) was used in the measurement of cationic metabolite substances. The capillary used was a fused silica capillary (inner diameter: 50 $\mu$m, total length: 80 cm) of catalogue number (Cat. No.) H3305-2002 from Human Metabolome Technologies America Inc. (HMT). The buffer solution used was a buffer solution of HMT Cat. No. 3301-1001. The measurement was performed at an applied voltage of +27 kV at a capillary temperature of 20°C. The sample was injected at 50 mbar over 10 seconds by use of the pressure method.

2) Analysis conditions for time-of-flight mass spectrometer (Q-TOF MS)

A cation mode was used. The ionization voltage was set to 4 kV, the fragmentor voltage was set to 80 V, the skimmer voltage was set to 50 V, and the octRFV voltage was set to 650 V. The dry gas used was nitrogen, and its temperature and pressure were set to 300°C and 5 psig, respectively. The sheath solution used was a sheath solution of HMT Cat. No. H3301-1020. The reference masses were set to m/z 65.059706 and m/z 622.08963.

(c-2) Anionic metabolite substance measurement conditions

1) Measurement equipment

Agilent 6210 TOF system equipped with Agilent 1600 CE system (manufactured by Agilent Technologies, Inc.) was used in the measurement of anionic metabolite substances. The capillary and its temperature used had the same settings as those for the anion. The buffer solution used was a buffer solution of HMT Cat. No. 3302-1021. The applied voltage was 30 kV. The sample was injected at 50 mbar over 25 seconds by use of the pressure method.

2) Analysis conditions for time-of-flight mass spectrometer (TOF MS)

A new anion mode was used. The ionization voltage was set to 3.5 kV, the fragmentor voltage was set to 125 V, the skimmer voltage was set to 50 V, and the octRFV voltage was set to 175 V. The same dry gas and sheath solution as those for the cation were used under the same conditions thereas. The reference masses were set to m/z 51.013854 and m/z 680.035541.

(c-3) Data processing

In order to obtain information on m/z, migration times (MT), and peaks including peak regions, raw data on peaks found by CE-Q-TOF MS or CE-TOF MS was processed using Master Hands automatic integration software version 2.0 (manufactured by Keio University). All the peaks were found with the software, and noise was removed to generate data matrix including the annotation and relative peak areas of metabolites. The peaks were annotated with metabolite names estimated from the HMT metabolite database on the basis of m/z obtained from CE and MT obtained from TOF MS. The conditions of MT, m/z, and the minimum S/N ratio for annotating anion peaks were set to 1.5 minutes, 50 ppm, and 20, respectively, while those for cations were set to 0.5 minutes, 50 ppm, and 20, respectively.

The relative concentration of each annotated metabolite was calculated by dividing the peak area of the metabolite by the area of ISC1 (cation) or ISA1 (anion).

In the CE-Q-TOF MS and CE-TOF MS analysis, anti-tumor effects on each individual patient were masked for analyzers.

A list of the processed peaks was output for further statistical analysis. In the statistical analysis, the relative concentrations of each annotated metabolite measured in duplicate were averaged.

(d) Statistical analysis

For clinical and metabolomics data processing and statistical analysis, JMP 64-Bit Edition version 12 (manufactured by SAS Institute Inc.) in Microsoft Windows 7 was used.

In this trial, 89 patients were registered, and 82 patients on which effects were determinable as a result of diagnostic imaging by the independent external review board were to be analyzed. Data on metabolites obtained from 82 serum samples before the start of treatment of this trial were used.

In this trial, results of diagnostic imaging by radiodiagnosticians were adopted according to the RECIST criteria. As a result, 9 patients exhibited partial response (PR), 49 patients exhibited stable disease (SD), and 24 patients exhibited progressive disease (PD), in terms of effects during the study treatment period. The PR patients are patients in which evident tumor shrinkage was observed, the SD patients are patients in which the control of tumor was observed, though no evident tumor shrinkage was seen, and the PD patients are patients in which treatment with the anti-cancer agent was ineffective and failed to control tumor. In general, PR and SD patients together are regarded as patients in which tumor has been successfully controlled.

For the purpose of searching for a marker for determining anti-cancer agent sensitivity which permitted determination of PR or PD, ordinal logistic analysis was conducted by using PR, SD and PD as objective variables and the concentrations of metabolites as explanatory variables. Also, nominal logistic analysis was conducted by using PR and the other factor (SD or PD) or PD and the other factor (PR or SD) as objective variables. Metabolites which were significant in the whole model and also in parameter estimates both in the ordinal logistic analysis and in the nominal logistic analysis were used as markers for determining anti-cancer agent sensitivity which permitted determination of PR or PD. In order to further determine PR or PD with higher accuracy, variables (metabolites) were selected by the step-up method with Bayesian information criterion (BIC) as an index in the step wise method. Multivariate nominal logistic regression analysis was conducted using the metabolites thus selected. In order to evaluate candidate substances for their prediction performance, sensitivity, specificity and accuracy were calculated using receiver-operator characteristics (ROC) and confusion matrix. A survival curve and a treatment period were estimated by use of the Kaplan-Meier method, and the log-rank test was used for difference in the curve. The performance of prediction of an overall survival by the metabolites was analyzed using COX proportional hazard model. In all the cases, $p < 0.05$ was regarded as being statistically significant.

(2) Results

(a) Substance serving as marker for determining anti-cancer agent sensitivity

(a-1) Marker for predicting PR

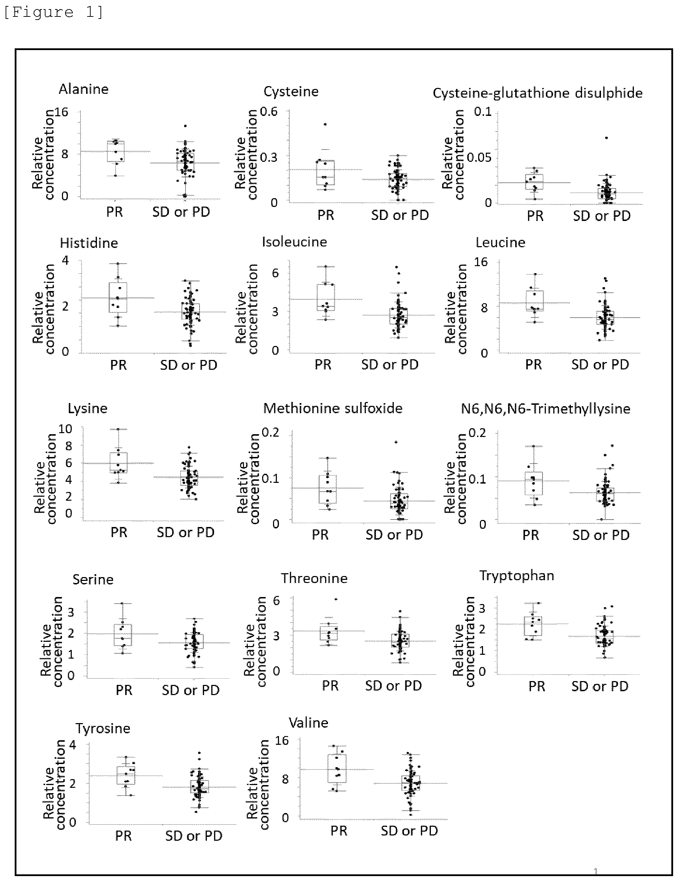

As a result of comprehensively conducting logistic analysis on 480 annotated metabolites in serum samples obtained before treatment of this trial, 14 substances, i.e., ALA, CYS, CSSG, HIS, ILE, LEU, LYS, METSF, N6TLY, SER, THR, TRP, TYR, and VAL, were found, as shown in Table 1, as substances which significantly differed in concentration between the PR group and the other group (SD or PD group), suggesting that these 14 substances serve as markers for determining PR. The relative concentration distribution of each substance in the PR group and in the other group (SD or PD group) is shown in Figure 1. All the substances exhibited a higher level in the PR group than in the other group (SD or PD group).

[Table 1]

| Metabolites | Ordinal Logistic Model PR vs. SD vs. PD | | Nominal Logistic Model PR vs. SD or PD | |
| --- | --- | --- | --- | --- |
| | p value (Whole Model) | p value (Parameter estimates) | p value (Whole Model) | p value (Parameter estimates) |
| ALA | 0.0003 | 0.0008 | 0.0113 | 0.0253 |
| CYS | 0.0296 | 0.0293 | 0.0242 | 0.0356 |
| CSSG | 0.0004 | 0.0016 | 0.0113 | 0.0254 |
| HIS | 0.0003 | 0.0006 | 0.0062 | 0.0107 |
| ILE | 0.0003 | 0.0006 | 0.0039 | 0.0053 |
| LEU | 0.0001 | 0.0003 | 0.003 | 0.0045 |
| LYS | 0.0019 | 0.0023 | 0.0025 | 0.0064 |
| METSF | 0.0217 | 0.0239 | 0.0179 | 0.0207 |
| N6TLY | 0.0012 | 0.002 | 0.0168 | 0.0167 |
| SER | 0.0067 | 0.0071 | 0.0225 | 0.0304 |
| THR | 0.0003 | 0.0006 | 0.0112 | 0.0171 |
| TRP | 0.0094 | 0.0095 | 0.0022 | 0.0047 |
| TYR | 0.0338 | 0.0289 | 0.0039 | 0.0068 |
| VAL | 0.0081 | 0.0098 | 0.0033 | 0.007 |

*In the table, "Ordinal Logistic Model" depicts results of ordinal logistic analysis by using PR, SD and PD as objective variables and the concentrations of the metabolites as explanatory variables, and "Nominal Logistic Model" depicts results of nominal logistic analysis by using PR and the other factor (SD or PD) as objective variables.

(a-2) Marker for predicting PD

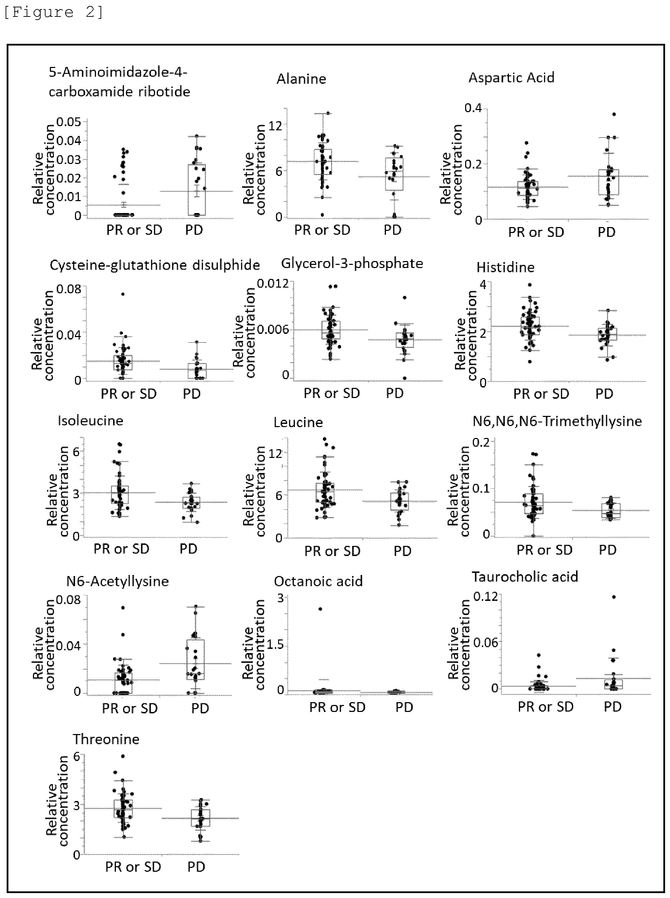

In the same way as in (a-1), 13 substances, i.e., 5A4CR, ALA, ASP, CSSG, GLC3P, HIS, ILE, LEU, N6TLY, N6ALY, OCTA, TUCA and THR, were found, as shown in Table 2, as substances which significantly differed in concentration between the PD group and the other group (PR or SD group), suggesting that these 13 substances serve as markers for determining PD. The relative concentration distribution of each substance in the PD group and in the other group (PR or SD group) is shown in Figure 2. 5A4CR, ASP, N6ALY and TUCA exhibited a higher level in the PD group, and the other substances exhibited a lower level in the PD group.

[Table 2]

| Metabolites | Ordinal Logistic Model PR vs. SD vs. PD | | Nominal Logistic Model PR or SD vs. PD | |
|---|---|---|---|---|
| | p value (Whole Model) | p value (Parameter estimates) | p value (Whole Model) | p value (Parameter estimates) |
| 5A4CR | 0.0146 | 0.0167 | 0.0210 | 0.0217 |
| ALA | 0.0003 | 0.0008 | 0.0024 | 0.0049 |
| ASP | 0.0045 | 0.0082 | 0.0073 | 0.0127 |
| CSSG | 0.0004 | 0.0016 | 0.0022 | 0.0081 |
| GLC3P | 0.0048 | 0.0065 | 0.0050 | 0.0119 |
| HIS | 0.0003 | 0.0006 | 0.0046 | 0.0098 |
| ILE | 0.0003 | 0.0006 | 0.0042 | 0.0127 |
| LEU | 0.0001 | 0.0003 | 0.0023 | 0.0082 |
| N6TLY | 0.0012 | 0.002 | 0.0071 | 0.0205 |
| N6ALY | 0.0016 | 0.0039 | 0.0009 | 0.0030 |
| OCTA | 0.0014 | 0.0197 | 0.0190 | 0.0405 |
| TUCA | 0.0117 | 0.0464 | 0.0061 | 0.0317 |
| THR | 0.0003 | 0.0006 | 0.0018 | 0.0052 |

*In the table, "Ordinal Logistic Model" depicts results of ordinal logistic analysis by using PR, SD and PD as objective variables and the concentrations of the metabolites as explanatory variables, and "Nominal Logistic Model" depicts results of nominal logistic analysis by using PD and the other factor (PR or SD) as objective variables.

A ROC curve was determined as to each individual substance of the markers for predicting PR or PD to determine a cut-off value of PR or PD. ROC AUC, sensitivity, specificity, and accuracy as the performance of prediction of PR or PD by each substance were determined on the basis of the cut-off value. As a result, as shown in Table 3, CSSG or LYS alone had ROC AUC as high as 0.79 and high sensitivity as PR prediction performance, and ALA had higher specificity and accuracy than those of the other substances. As shown in Table 4, ROC AUC as PD prediction performance was the highest in CSSG, whereas sensitivity was high in HIS or N6TLY.

[Table 3]

| PR Predict Metabolites | Cut-off | ROC AUC | Sensitivity (%) | Specificity (%) | Accuracy (%) |
|---|---|---|---|---|---|
| ALA | $9.957 \leqq$ | 0.75 | 55.6 | 94.5 | 90.2 |
| CYS | $2.444 \times 10^{-1} \leqq$ | 0.63 | 33.3 | 91.8 | 85.4 |
| CSSG | $1.430 \times 10^{-2} \leqq$ | 0.79 | 88.9 | 69.9 | 72.0 |
| HIS | $2.2409 \leqq$ | 0.74 | 77.8 | 71.2 | 72.0 |
| ILE | $2.997 \leqq$ | 0.70 | 77.8 | 63.0 | 64.6 |
| LEU | $7.437 \leqq$ | 0.74 | 66.7 | 82.2 | 80.5 |
| LYS | $4.945 \leqq$ | 0.79 | 88.9 | 68.5 | 70.7 |
| METSF | $6.658 \times 10^{-2} \leqq$ | 0.72 | 66.7 | 78.1 | 76.8 |
| N6TLY | $8.401 \times 10^{-2} \leqq$ | 0.76 | 66.7 | 84.9 | 82.9 |
| SER | $2.200 \leqq$ | 0.63 | 33.3 | 93.2 | 86.6 |
| THR | $2.753 \leqq$ | 0.72 | 77.8 | 65.8 | 67.1 |
| TRP | $2.165 \leqq$ | 0.77 | 66.7 | 86.3 | 84.1 |
| TYR | $2.084 \leqq$ | 0.76 | 77.8 | 74.0 | 74.4 |
| VAL | $8.317 \leqq$ | 0.76 | 77.8 | 74.0 | 74.4 |

[Table 4]

| PD Predict Metabolites | Cut-off | ROC AUC | Sensitivity (%) | Specificity (%) | Accuracy (%) |
|---|---|---|---|---|---|
| 5A4CR | $1.421 \times 10^{-2} \leq$ | 0.61 | 41.7 | 81.0 | 69.5 |
| ALA | $< 6.494$ | 0.65 | 66.7 | 63.8 | 64.6 |
| ASP | $0.1433 \leq$ | 0.68 | 54.2 | 81.0 | 73.2 |
| CSSG | $< 8.630 \times 10^{-3}$ | 0.69 | 66.7 | 70.7 | 69.5 |
| GCL3P | $< 6.009 \times 10^{-3}$ | 0.66 | 87.5 | 44.8 | 57.3 |
| HIS | $< 2.366$ | 0.67 | 95.8 | 37.9 | 54.9 |
| ILE | $< 2.748$ | 0.67 | 75.0 | 58.6 | 63.4 |
| LEU | $< 6.413$ | 0.66 | 79.2 | 53.4 | 61.0 |
| N6TLY | $< 8.030 \times 10^{-2}$ | 0.63 | 95.8 | 31.0 | 50.0 |
| N6ALY | $2.915 \times 10^{-2} \leq$ | 0.65 | 33.3 | 96.6 | 78.0 |
| OCAT | $< 6.767 \times 10^{-2}$ | 0.62 | 50.0 | 74.1 | 67.1 |
| TUCA | $2.380 \times 10^{-3} \leq$ | 0.64 | 66.7 | 62.1 | 63.4 |
| THR | $< 2.137$ | 0.67 | 54.2 | 79.3 | 72.0 |

(b) Calculation of anti-cancer agent sensitivity prediction model

Variables (metabolites) as to the 14 markers for predicting PR shown in (a-1) and the 13 markers for predicting PD shown in (a-2) were selected by the step-up method with Bayesian information criterion (BIC) as an index in the step wise method. Multivariate nominal logistic models were prepared using the selected variables to calculate a PR prediction model and a PD prediction model. The results are shown in Table 5.

[Table 5]

| | Metabolites | Cut-off value | Parameter | | probability |
|---|---|---|---|---|---|
| | | | < cut-off | cut-off ≤ | |
| PR prediction model | Alanine (ALA) | 9.957 | 0 | 2.5043 | PR probability = 1/(1+Exp( 4.2504 - Parameter :ALA - Parameter :CSSG)) |
| | Cysteine-glutathione disulphide (CSSG) | $1.430 \times 10^{-2}$ | 0 | 2.4626 | |
| PD prediction model | Aspartic Acid (ASP) | 0.1433 | 1.0820 | - 1.0820 | PD probability = 1/(1+Exp(0.8105 + Parameter :ASP + Parameter :HIS + Parameter :N6ALY + Parameter :TUCA)) |
| | Histidine (HIS) | 2.366 | - 1.7717 | 1.7717 | |
| | N6-Acetyllysine (N6ALY) | $2.915 \times 10^{-2}$ | 1.5499 | - 1.5499 | |
| | Taurocholic acid (TUCA) | $2.380 \times 10^{-3}$ | 0.7905 | - 0.7905 | |

The PR prediction model is as shown in the following expression (1):

$$p = \frac{1}{1+e^{(4.2504-(ALA)-(CSSG))}} \qquad (1)$$

wherein ALA represents 2.5043 when a measurement result about ALA is equal to or more than a cut-off value, and represents 0 when the measurement result is less than the cut-off value; and CSSG represents 2.4626 when a measurement result about CSSG is equal to or more than a cut-off value, and represents 0 when the measurement result is less than the cut-off value.

The model is an expression for determining whether or not a patient has PR after this treatment. The p value represents a probability that the patient has PR after this treatment. When the p value is 0.5 or more, it is determined that PR can be expected.

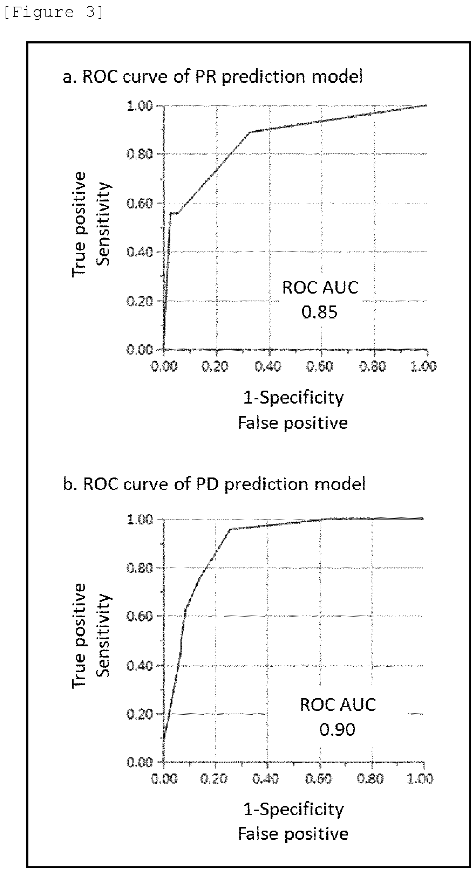

AUC of the ROC curve of this PR prediction model was 0.85 (Figure 3a). The PR predictive value, sensitivity, specificity, and accuracy thereof were 71.4%, 55.6%, 97.3%, and 92.7%, respectively, as shown in Table 6.

The PD prediction model is as shown in the following expression (2):

$$p = \frac{1}{1+e^{(0.8105+(ASP)+(HIS)+(N6ALY)+(TUCA))}} \qquad (2)$$

wherein ASP represents -1.0820 when a measurement result about ASP is equal to or more than a cut-off value, and represents 1.0820 when the measurement result is less than the cut-off value; HIS represents 1.7717 when a measurement result about HIS is equal to or more than a cut-off value, and represents -1.7717 when the measurement result is less than the cut-off value; N6ALY represents -1.5499 when a measurement result about N6ALY is equal to or more than a cut-off value, and represents 1.5499 when the measurement result is less than the cut-off value; and TUCA represents -0.7905 when a measurement result about TUCA is equal to or more than a cut-off value, and represents 0.7905 when the measurement result is less than the cut-off value.

The model is an expression for determining whether or not a patient has PD after this treatment. The p value represents a probability that the patient has PD after this treatment. When the p value is 0.5 or more, it is determined that PD appears.

AUC of the ROC curve of this PD prediction model was 0.90 (Figure 3b). The PD predictive value, sensitivity, specificity, and accuracy thereof were 75.0%, 62.5%, 91.4%, and 82.9%, respectively, as shown in Table 6.

[Table 6]

| | | Predicted | | | | Predictive value (%) | Sensitivity (%) | Specificity (%) | Accuracy (%) |
|---|---|---|---|---|---|---|---|---|---|
| | | PR | SD | PD | Total | | | | |
| Actual | PR | 5 | 4 | 0 | 9 | 71.4 | 55.6 | 97.3 | 92.7 |
| | SD | 2 | 42 | 5 | 49 | | | | |
| | PD | 0 | 9 | 15 | 24 | 75.0 | 62.5 | 91.4 | 82.9 |
| | Total | 7 | 55 | 20 | 82 | | | | |

Among patients determined to have PR by the PR prediction model, there were no patients who actually had PD. In addition, among patients determined to have PD by the PD prediction model, there were no patients who actually had PR. This fact also showed high accuracy of these prediction models.

(c) OS prediction performance of PR prediction model and PD prediction model

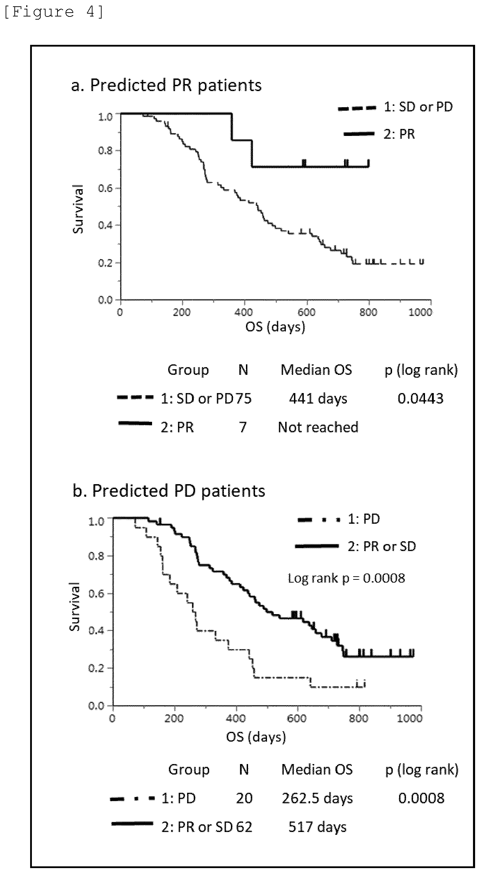

On the basis of the PR prediction model of (b), patients were classified into a PR group (group determined to have PR by the PR prediction model) and the other group (group determined to have no PR by the PR prediction model (SD or PD group)), and a Kaplan-Meier curve was drawn. The results are shown in Figure 4a. The prognosis of the PR group (median: incalculable) had a significantly (p = 0.0443) longer survival period than that of the SD or PD group (median: 441 days).

On the basis of the PD prediction model of (b), patients were classified into a PD group (group determined to have PD by the PD prediction model) and the other group (group determined to have no PD by the PD prediction model (PR or SD group)), and a Kaplan-Meier curve was drawn. The results are shown in Figure 4b. The prognosis of the PD group (median: 262.5 days) had a significantly (p = 0.0008) shorter survival period than that of the PR or SD group (median: 517 days).

These results demonstrate the usefulness of the PR prediction model and the PD prediction model.

(d) Analysis of OS using COX proportional hazard model

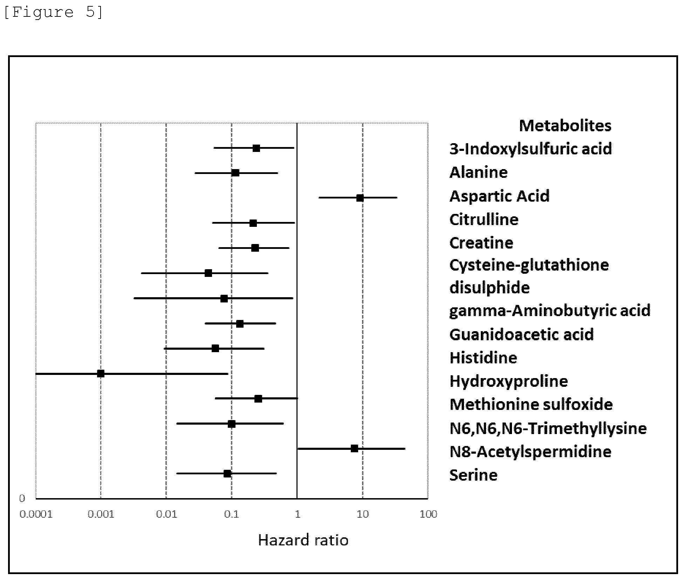

Substances for predicting prognosis were analyzed using proportional hazard model from blood metabolite concentrations before the start of treatment and the overall survival of each patient. As a result, the hazard ratios of substances with significance and their 95% confidence intervals are shown in Figure 5. It was found that the higher the blood concentrations of 3IND, ALA, CITR, CREAT, CSSG, GABA, GUAA, HIS, HYPRO, METSF, N6TLY and SER, the longer the survival period while the higher the blood concentrations of ASP and N8ASR, the shorter the survival period. ALA, CSSG, HIS, METSF, N6TLY and SER overlapped between these substances significant using the COX proportional hazard model and the markers for predicting PR in (a-1). These overlapping substances for predicting prognosis had behavior consistent with the results of (a-1), as shown in Table 7, demonstrating that these substances are particularly useful as markers for predicting prognosis.

[Table 7]

| Substance for predicting prognosis | Behavior in (a-1) | Behavior in (d) |
|---|---|---|
| ALA | Blood concentration was higher in the PR group than in the SD or PD group. | The higher the blood concentration, the longer the survival period. |
| CSSG | Blood concentration was higher in the PR group than in the SD or PD group. | The higher the blood concentration, the longer the survival period. |
| HIS | Blood concentration was higher in the PR group than in the SD or PD group. | The higher the blood concentration, the longer the survival period. |
| METSF | Blood concentration was higher in the PR group than in the SD or PD group. | The higher the blood concentration, the longer the survival period. |
| N6TLY | Blood concentration was higher in the PR group than in the SD or PD group. | The higher the blood concentration, the longer the survival period. |
| SER | Blood concentration was higher in the PR group than in the SD or PD group. | The higher the blood concentration, the longer the survival period. |

Likewise, ALA, ASP, CSSG, HIS and N6TLY overlapped between the substances significant using the COX proportional hazard model and the markers for predicting PD in (a-2). These overlapping substances for predicting prognosis had behavior consistent with the results of (a-2), as shown in Table 8, demonstrating that these substances are particularly useful as markers for predicting prognosis.

[Table 8]

| Substance for predicting prognosis | Behavior in (a-2) | Behavior in (d) |
|---|---|---|
| ALA | Blood concentration was higher in the PR or SD group than in the PD group. | The higher the blood concentration, the longer the survival period. |
| ASP | Blood concentration was higher in the PD group than in the PR or SD group. | The higher the blood concentration, the shorter the survival period. |
| CSSG | Blood concentration was higher in the PR or SD group than in the PD group. | The higher the blood concentration, the longer the survival period. |
| HIS | Blood concentration was higher in the PR or SD group than in the PD group. | The higher the blood concentration, the longer the survival period. |
| N6TLY | Blood concentration was higher in the PR or SD group than in the PD group. | The higher the blood concentration, the longer the survival period. |

(e) Comparison of OS in grouping using cut-off value

As for CSSG, HIS and N6TLY having the same cut-off value of OS as the cut-off value of PR prediction among the substances found to be particularly useful as markers for predicting prognosis in (d), patients were grouped using the cut-off value, and the usefulness of the markers was further verified by the comparison of OS.

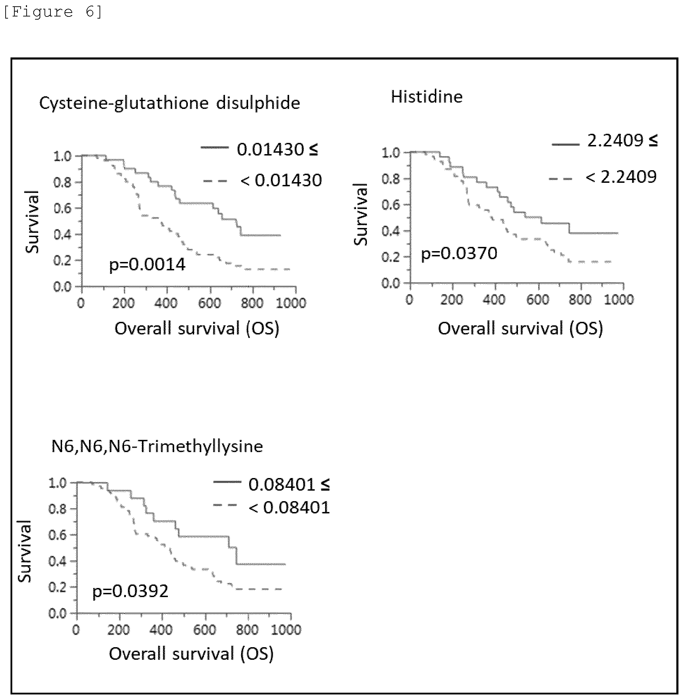

As for CSSG, when OS was compared between a group of $1.430 \times 10^{-2}$ (cut-off value of CSSG) or more and a group of less than $1.430 \times 10^{-2}$, the group of $1.430 \times 10^{-2}$ or more exhibited significantly longer OS ($p = 0.0014$) (Figure 6).

As for HIS, when OS was compared between a group of 2.2409 (cut-off value of HIS) or more and a group of less than 2.2409, the group of 2.2409 or more exhibited significantly longer OS ($p = 0.0370$) (Figure 6).

As for N6TLY, when OS was compared between a group of $8.401 \times 10^{-2}$ (cut-off value of N6TLY) or more and a group of less than $8.401 \times 10^{-2}$, the group of $8.401 \times 10^{-2}$ or more exhibited significantly longer OS ($p = 0.0392$) (Figure 6).

These results further demonstrated the usefulness of CSSG, HIS and N6TLY as markers for predicting prognosis.

(f) Correlation between total tumor size and CSSG before start of secondary treatment

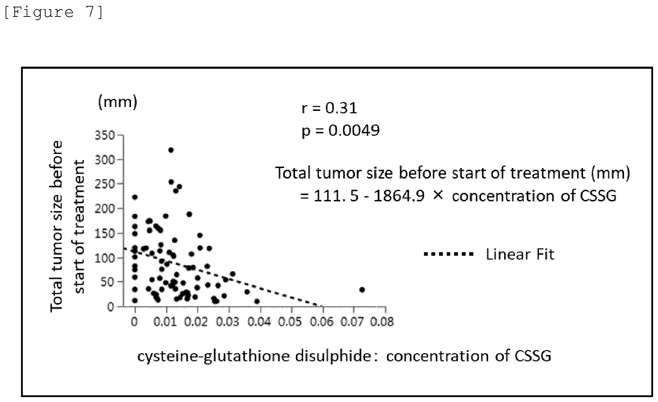

As a result of confirming the correlation between a total tumor size and the concentration of CSSG before the start of secondary treatment in patients who were refractory or intolerant to combination therapy of FOLFOX and bevacizumab as primary therapy, significant correlation was seen, as shown in Figure 7. The regression equation was total tumor size = 111.5 - 1864.9 $\times$ (CSSG) (correlation coefficient: r = 0.31 and p value: p = 0.0049; (CSSG) represents the concentration of CSSG). From these results, it was found that the total tumor size of a cancer patient refractory or intolerant to combination therapy of FOLFOX and bevacizumab can be determined by measuring the amount of CSSG in a biological sample derived from the cancer patient.

Example 2

(1) Method

Analysis was conducted in accordance with the method of Example 1 except that data on metabolites obtained from 82 and 77 serum samples in an early stage after the start of treatment (cycles 1 and 2, respectively) of this trial was used.

(2) Results

(a) Substance serving as marker for determining anti-cancer agent sensitivity

(a-1) Marker for predicting PR - 1

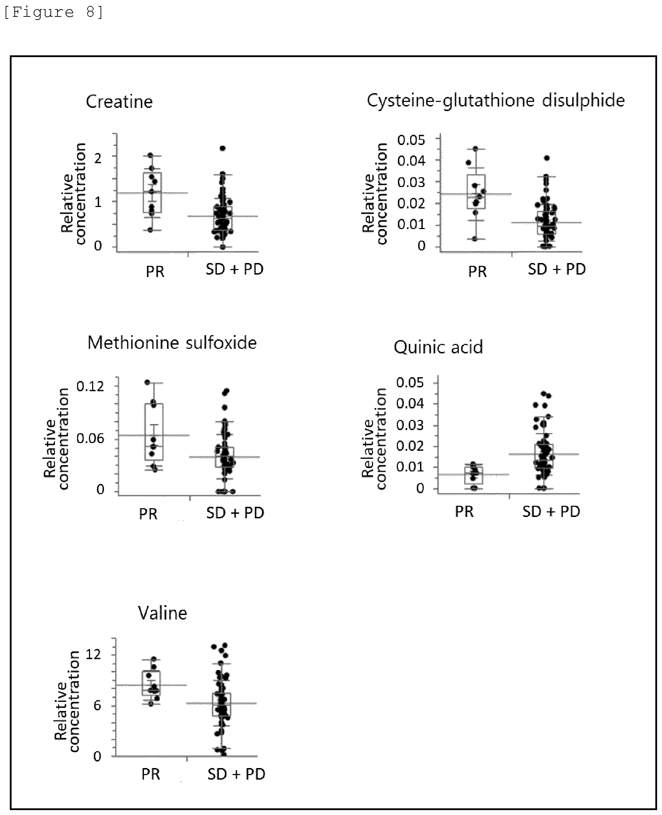

As a result of comprehensively conducting logistic analysis on metabolites detected in 25% or more of specimens of all patients among 480 annotated metabolites in serum samples obtained in an early stage after the start of treatment (cycle 1) of this trial, 5 substances, i.e., CREAT, CSSG, METSF, QUINA, and VAL, were found, as shown in Table 9, as substances which significantly differed in concentration between the PR group and the other group (SD or PD group), suggesting that these 5 substances serve as markers for determining PR. The relative concentration distribution of each substance in the PR group and in the other group (SD or PD group) is shown in Figure 8. QUINA exhibited a lower level in the PR group, and the other substances exhibited a higher level in the PR group.

[Table 9]

| Metabolites | Ordinal Logistic Model PR vs. SD vs. PD | | Nominal Logistic Model PR vs. SD or PD | |
| --- | --- | --- | --- | --- |
| | p value (Whole Model) | p value (Parameter estimates) | p value (Whole Model) | p value (Parameter estimates) |
| CREAT | 0.0323 | 0.0332 | 0.0018 | 0.0035 |
| CSSG | <.0001 | 0.0001 | 0.0004 | 0.0016 |
| METSF | 0.0419 | 0.0459 | 0.0153 | 0.0175 |
| QUINA | 0.0245 | 0.0275 | 0.0004 | 0.0056 |
| VAL | 0.0123 | 0.0159 | 0.021 | 0.0258 |
| *In the table, "Ordinal Logistic Model" depicts results of ordinal logistic analysis by using PR, SD and PD as objective variables and the concentrations of the metabolites as explanatory variables, and "Nominal Logistic Model" depicts results of nominal logistic analysis by using PR and the other factor (SD or PD) as objective variables. | | | | |

(a-2) Marker for predicting PD - 1

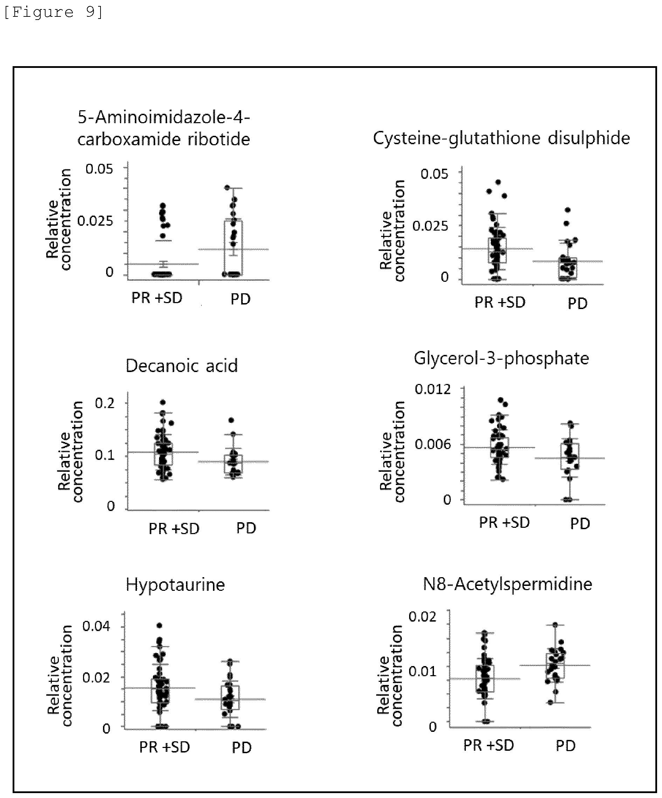

In the same way as in (a-1), 6 substances, i.e., 5A4CR, CSSG, DECNA, GLC3P, HYPTA, and N8ASR, were found, as shown in Table 10, as substances which significantly differed in concentration between the PD group and the other group (PR or SD group), suggesting that these 6 substances serve as markers for determining PD. The relative concentration distribution of each substance in the PD group and in the other group (PR or SD group) is shown in Figure 9. 5A4CR and N8ASR exhibited a higher level in the PD group, and the other substances exhibited a lower level in the PD group.

[Table 10]

| Metabolites | Ordinal Logistic Model PR vs. SD. vs. PD | | Nominal Logistic Model PR or SD vs. PD | |
|---|---|---|---|---|
| | p value (Whole Model) | p value (Parameter estimates) | p value (Whole Model) | p value (Parameter estimates) |
| 5A4CR | 0.0458 | 0.0438 | 0.0236 | 0.63111 |
| CSSG | <.0001 | 0.0001 | 0.008 | 0.0178 |
| DECNA | 0.0365 | 0.0418 | 0.0132 | 0.0259 |
| GLC3P | 0.0194 | 0.0256 | 0.0144 | 0.0229 |
| HYPTA | 0.0147 | 0.0162 | 0.0315 | 0.0417 |
| N8ASR | 0.0035 | 0.0042 | 0.0047 | 0.0089 |
| *In the table, "Ordinal Logistic Model" depicts results of ordinal logistic analysis by using PR, SD and PD as objective variables and the concentrations of the metabolites as explanatory variables, and "Nominal Logistic Model" depicts results of nominal logistic analysis by using PD and the other factor (PR or SD) as objective variables. | | | | |

(a-3) Marker for predicting PR - 2

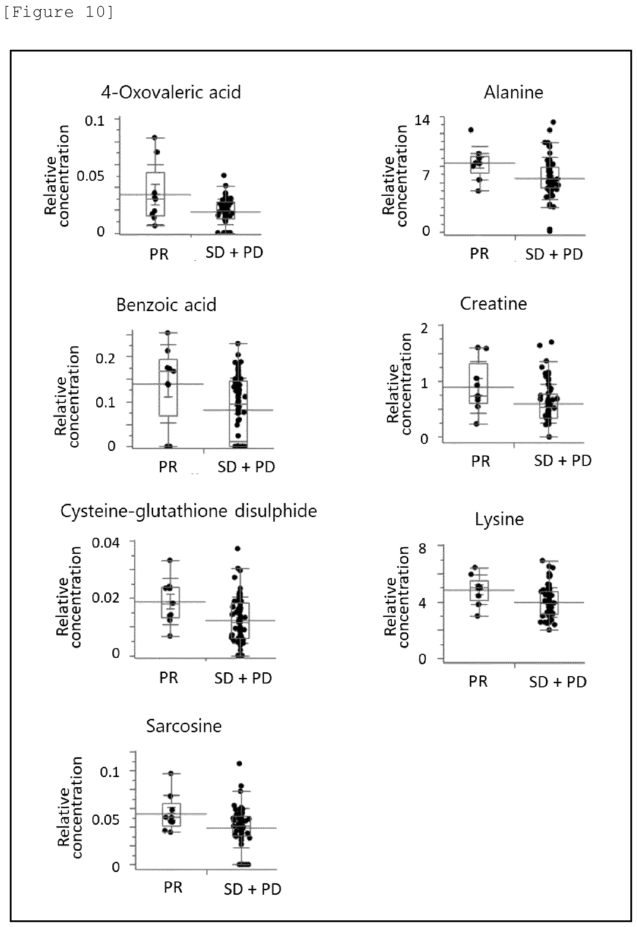

As a result of comprehensively conducting logistic analysis on metabolites detected in 25% or more of specimens of all patients among 480 annotated metabolites in serum samples obtained in an early stage after the start of treatment (cycle 2) of this trial, 7 substances, i.e., 4OVAL, ALA, BENZA, CREAT, CSSG, LYS, and SARCO, were found, as shown in Table 11, as substances which significantly differed in concentration between the PR group and the other group (SD or PD group), suggesting that these 7 substances serve as markers for determining PR. The relative concentration distribution of each substance in the PR group and in the other group (SD or PD group) is shown in Figure 10. All the substances exhibited a higher level in the PR group.

[Table 11]

| Metabolites | Ordinal Logistic Model PH vs. SD vs. PD | | Nominal Logistic Model PR vs. SD or PD | |
|---|---|---|---|---|
| | p value (Whole Model) | p value (Parameter estimates) | p value (Whole Model) | p value (Parameter estimates) |
| 4OVAL | 0.0015 | 0.0023 | 0.0073 | 0.0159 |
| ALA | 0.0083 | 0.011 | 0.0344 | 0.0439 |
| BENZA | 0.0117 | 0.0122 | 0.0246 | 0.0411 |
| CREAT | 0.0419 | 0.0464 | 0.0341 | 0.0326 |
| CSSG | 0.0054 | 0.0066 | 0.0302 | 0.0353 |
| LYS | 0.0233 | 0.0234 | 0.0264 | 0.0316 |
| SARCO | 0.0167 | 0.0197 | 0.0419 | 0.0514 |
| *In the table, "Ordinal Logistic Model" depicts results of ordinal logistic analysis by using PR, SD and PD as objective variables and the concentrations of the metabolites as explanatory variables, and "Nominal Logistic Model" depicts results of nominal logistic analysis by using PR and the other factor (SD or PD) as objective variables. | | | | |

(a-4) Marker for predicting PD - 2

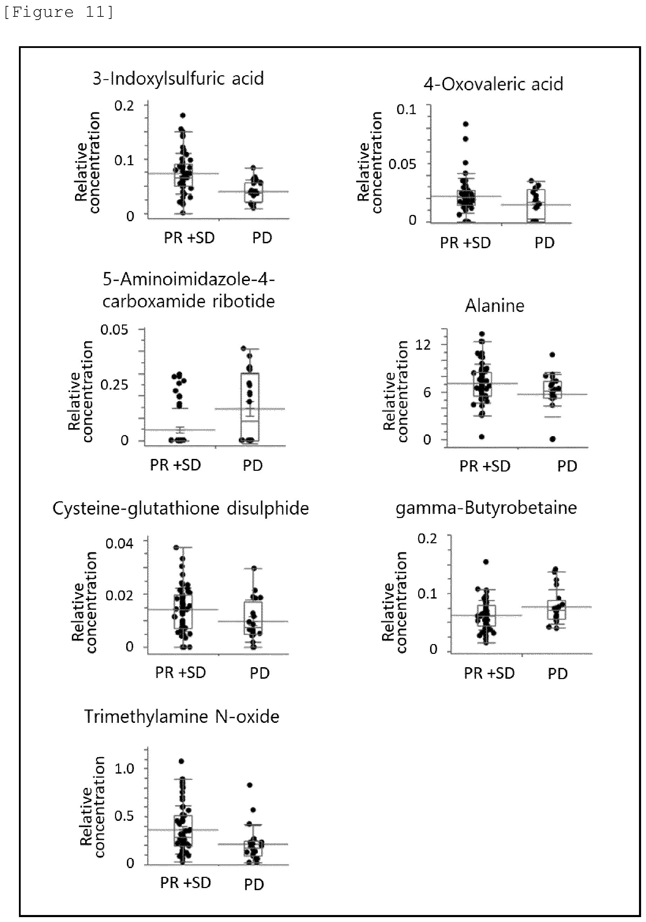

In the same way as in (a-3), 7 substances, i.e., 3IND, 4OVAL, 5A4CR, ALA, CSSG, GABB, and TMNO, were found, as shown in Table 12, as substances which significantly differed in concentration between the PD group and the other group (PR or SD group), suggesting that these 7 substances serve as markers for determining PD. The relative concentration distribution of each substance in the PD group and in the other group (PR or SD group) is shown in Figure 11. 5A4CR and GABB exhibited a higher level in the PD group, and the other substances exhibited a lower level in the PD group.

[Table 12]

| Metabolites | Ordinal Logistic Model PR vs. SD. vs. PD | | Nominal PR or Logistic Model SD vs. PD | |
|---|---|---|---|---|
| | p value (Whole Model) | p value (Parameter estimates) | p value (Whole Model) | p value (Parameter esti mates) |
| 3IND | 0.0059 | 0.0087 | <.0001 | 0.0011 |
| 4OVAL | 0.0015 | 0.0023 | 0.0343 | 0.0513 |
| 5A4CR | 0.0061 | 0.0073 | 0.004 | 0.0049 |
| ALA | 0.0083 | 0.011 | 0.0364 | 0.045 |
| CSSG | 0.0054 | 0.0066 | 0.0302 | 0.0425 |
| GABB | 0.014 | 0.0166 | 0.0399 | 0.0453 |
| TMNO | 0.0252 | 0.0303 | 0.0097 | 0.0273 |

*In the table, "Ordinal Logistic Model" depicts results of ordinal logistic analysis by using PR, SD and PD as objective variables and the concentrations of the metabolites as explanatory variables, and "Nominal Logistic Model" depicts results of nominal logistic analysis by using PD and the other factor (PR or SD) as objective variables.

An ROC curve was determined as to each individual substance of the markers for predicting PR or PD to determine a cut-off value of PR or PD. ROC AUC, sensitivity, specificity, and accuracy as the performance of prediction of PR or PD by each substance were determined on the basis of the cut-off value. The results about the markers for predicting PR or PD in (a-1) and (a-2) are shown in Table 13. CREAT, CSSG, METSF, QUINA and VAL as the markers for predicting PR exhibited ROC AUC as high as 0.7 or more as PR prediction performance. ROC AUC as PD prediction performance was the highest in N8ASR as the marker for predicting PD.

[Table 13]

| PR Predict Metabolites | Cut-off | ROC AUC | Sensitivity (%) | Specificity (%) | Accuracy (%) |
|---|---|---|---|---|---|
| CREAT | $1.2163 \leqq$ | 0.78 | 55.6 | 93.2 | 89.0 |
| CSSG | $1.965 \times 10^{-2} \leqq$ | 0.83 | 77.8 | 87.7 | 86.6 |
| METSF | $5.060 \times 10^{-2} \leqq$ | 0.70 | 66.7 | 75.3 | 74.4 |
| QUINA | $< 1.150 \times 10^{-2}$ | 0.84 | 100.0 | 61.6 | 65.9 |
| VAL | $7.6718 \leqq$ | 0.78 | 77.8 | 78.1 | 78.0 |

| PD Predict Metabolites | Cut-off | ROC AUC | Sensitivity (%) | Specificity (%) | Accuracy (%) |
|---|---|---|---|---|---|
| 5A4CR | $1.413 \times 10^{-2} \leqq$ | 0.63 | 81.0 | 45.8 | 70.7 |
| CSSG | $< 1.030 \times 10^{-2}$ | 0.69 | 62.1 | 79.2 | 67.1 |
| DECNA | $< 1.080 \times 10^{-1}$ | 0.67 | 48.3 | 91.7 | 61.0 |
| GLC3P | $< 4.586 \times 10^{-1}$ | 0.64 | 75.9 | 50.0 | 68.3 |
| HYPTA | $< 1.240 \times 10^{-2}$ | 0.66 | 69.0 | 66.7 | 68.3 |
| N8ASR | $1.122 \times 10^{-2} \leqq$ | 0.70 | 87.9 | 45.8 | 75.6 |

The results about the markers for predicting PR or PD in (a-3) and (a-4) are shown in Table 14. ALA, BENZA, CREAT, CSSG and LYS as the markers for predicting PR exhibited ROC AUC as high as 0.7 or more as PR prediction performance. ROC AUC as PD prediction performance was the highest in 3IND as the marker for predicting PD.

[Table 14]

| PR Predict Metabolites | Cut-off | ROC AUC | Sensitivity (%) | Specificity (%) | Accuracy (%) |
|---|---|---|---|---|---|
| 4OVAL | $2.949 \times 10^{-2} \leqq$ | 0.67 | 55.6 | 85.3 | 81.8 |
| ALA | $7.9605 \leqq$ | 0.75 | 77.8 | 76.5 | 76.6 |
| BENZA | $1.367 \times 10^{-1} \leqq$ | 0.72 | 77.8 | 72.1 | 72.7 |
| CREAT | $6.609 \times 10^{-1} \leqq$ | 0.71 | 77.8 | 66.2 | 67.5 |
| CSSG | $1.233 \times 10^{-2} \leqq$ | 0.73 | 88.9 | 52.9 | 57.1 |
| LYS | $4.9765 \leqq$ | 0.74 | 66.7 | 83.8 | 81.8 |
| SARCO | $4.548 \times 10^{-2} \leqq$ | 0.68 | 77.8 | 57.4 | 59.7 |

| PD Predict Metabolites | Cut-off | ROC AUC | Sensitivity (%) | Specificity (%) | Accuracy (%) |
|---|---|---|---|---|---|
| 3IND | $< 6.129 \times 10^{-2}$ | 0.79 | 63.2 | 90.0 | 70.1 |
| 4OVAL | $< 1.346 \times 10^{-2}$ | 0.62 | 80.7 | 45.0 | 71.4 |
| 5A4CR | $2.052 \times 10^{-2} \leqq$ | 0.67 | 87.7 | 45.0 | 76.6 |
| ALA | $< 7.3693$ | 0.62 | 47.4 | 80.0 | 55.8 |
| CSSG | $< 1.273 \times 10^{-2}$ | 0.67 | 57.9 | 75.0 | 62.3 |
| GABB | $5.117 \times 10^{-2} \leqq$ | 0.63 | 33.3 | 90.0 | 48.1 |
| TMNO | $< 2.689 \times 10^{-1}$ | 0.71 | 56.1 | 85.0 | 63.6 |

(b-1) Calculation of anti-cancer agent sensitivity prediction model - 1

Variables (metabolites) as to the 5 markers for predicting PR at cycle 1 shown in (a-1) and the 6 markers for predicting PD at cycle 1 shown in (a-2) were selected by the step-up method with Bayesian information criterion (BIC) as an index in the step wise method. Multivariate nominal logistic models were prepared using the selected variables to calculate a PR prediction model and a PD prediction model. The results are shown in Table 15.

[Table 15]

| | | Metabolites | PR or PD Cut-off value | Parameter | | probability |
|---|---|---|---|---|---|---|
| | | | | < Cut off | Cut off ≦ | |
| Cycle 1 | PR prediction model | CREAT | 1.2163 | - 1.2906 | 1.2906 | PR probability = 1/[1 + exp( 9.0171 - Parameter : CREAT - Parameter : CSSG - Parameter : QUINA)] |
| | | CSSG | $1.965 \times 10^{-2}$ | - 1.7703 | 1.7703 | |
| | | QUINA | $1.150 \times 10^{-2}$ | 8.6990 | - 8.6990 | |
| | PD prediction model | 5A4CR | $1.413 \times 10^{-2}$ | 0.9300 | - 0.9300 | PD probability = 1/[1 + exp ( 0.8232 + Parameter : 5A4CR + Parameter : CSSG + Parameter : DECNA + Parameter : HYPTA + Parameter : N8ASR)] |
| | | CSSG | $1.030 \times 10^{-2}$ | - 1.2325 | 1.2325 | |
| | | DECNA | $1.080 \times 10^{-1}$ | - 1.3052 | 1.3052 | |
| | | HYPTA | $1.240 \times 10^{-2}$ | - 0.8020 | 0.8020 | |
| | | N8ASR | $1.122 \times 10^{-2}$ | 1.4363 | - 1.4363 | |

The PR prediction model is as shown in the following expression (4):

$$p = \frac{1}{1+e^{(9.0171-(CREAT)-(CSSG)-(QUINA))}} \qquad (4)$$

wherein CREAT represents 1.2906 when a measurement result about CREAT is equal to or more than a cut-off value, and represents -1.2906 when the measurement result is less than the cut-off value; CSSG represents 1.7703 when a measurement result about CSSG is equal to or more than a cut-off value, and represents -1.7703 when the measurement result is less than the cut-off value; and QUINA represents -8.6990 when a measurement result about QUINA is equal to or more than a cut-off value, and represents 8.6990 when the measurement result is less than the cut-off value.

The model is an expression for determining whether or not a patient has PR after continuing the second or later cycles of treatment. The p value represents a probability that the patient has PR after continuing the second or later cycles of treatment. When the p value exceeds 0.5, it is determined that PR appears.

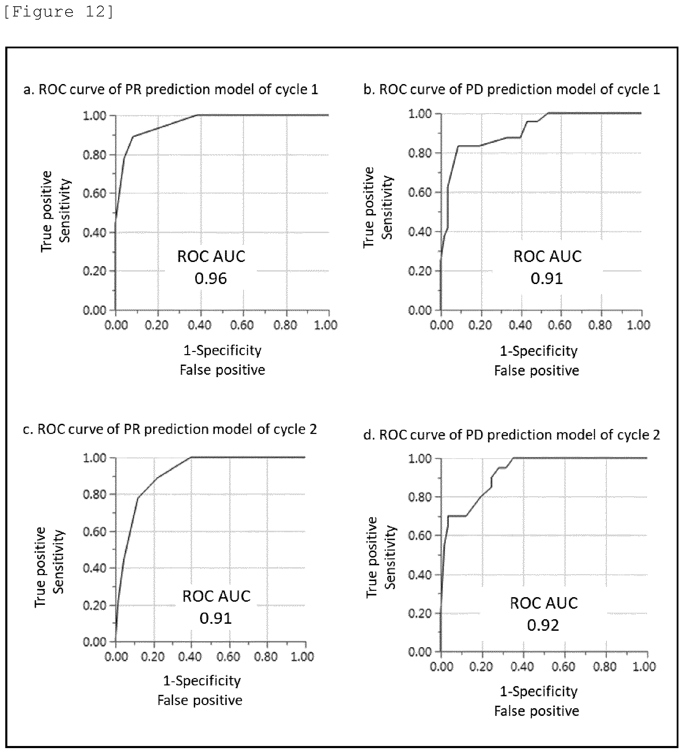

AUC of the ROC curve of this PR prediction model was 0.96 (Figure 12a). The PR predictive value, sensitivity, specificity, and accuracy thereof were 70.0%, 77.8%, 95.9%, and 93.9%, respectively, as shown in Table 16.

The PD prediction model is as shown in the following expression (5):

$$p = \frac{1}{1+e^{(0.8232+(5A4CR)+(CSSG)+(DECNA)+(HYPTA)+(N8ASR))}} \qquad (5)$$

wherein 5A4CR represents -0.9300 when a measurement result about 5A4CR is equal to or more than a cut-off value, and represents 0.9300 when the measurement result is less than the cut-off value; CSSG represents 1.2325 when a measurement result about CSSG is equal to or more than a cut-off value, and represents -1.2325 when the measurement result is less than the cut-off value; DECNA represents 1.3052 when a measurement result about DECNA is equal to or more than a cut-off value, and represents -1.3052 when the measurement result is less than the cut-off value; HYPTA represents 0.8020 when a measurement result about HYPTA is equal to or more than a cut-off value, and represents -0.8020 when the measurement result is less than the cut-off value; and N8ASR represents - 1.4363 when a measurement result about N8ASR is equal to or more than a cut-off value, and represents 1.4363 when the measurement result is less than the cut-off value.

The model is an expression for determining whether or not a patient has PD after continuing the second or later cycles of treatment. The p value represents a probability that the patient has PD after continuing the second or later cycles of treatment. When the p value exceeds 0.5, it is determined that PD appears.

AUC of the ROC curve of this PD prediction model was 0.91 (Figure 12b). The PD predictive value, sensitivity, specificity, and accuracy thereof were 80.0%, 91.4%, 83.3%, and 89.0%, respectively, as shown in Table 16.

[Table 16]

| | | Predicted | | | | Predictive value (%) | Sensitivity (%) | Specificity (%) | Accuracy (%) |
|---|---|---|---|---|---|---|---|---|---|
| | | PR | SD | PD | Total | | | | |
| Cycle 1 Actual | PR | 7 | 2 | | 9 | 70.0 | 77.8 | 95.9 | 93.9 |
| | SD | 3 | 41 | 5 | 49 | | | | |
| | PD | | 4 | 20 | 24 | 80.0 | 91.4 | 83.3 | 89.0 |
| | Total | 10 | 47 | 25 | 82 | | | | |

Among patients determined to have PR by the PR prediction model, there were no patients who actually had PD. In addition, among patients determined to have PD by the PD prediction model, there were no patients who actually had PR. This fact also showed high accuracy of these prediction models.

(b-2) Calculation of anti-cancer agent sensitivity prediction model - 2

Variables (metabolites) as to the 7 markers for predicting PR at cycle 2 shown in (a-3) and the 7 markers for predicting PD at cycle 2 shown in (a-4) were selected by the step-up method with Bayesian information criterion (BIC) as an index in the step wise method. Multivariate nominal logistic models were prepared using the selected variables to calculate a PR prediction model and a PD prediction model. The results are shown in Table 17.

[Table 17]

| | | Metabolites | PR or PD Cut-off value | Parameter | | probability |
|---|---|---|---|---|---|---|
| | | | | < Cut off | Cut off $\leqq$ | |
| Cycle 2 | PR prediction model | 4OVAL | $2.949 \times 10^{-2}$ | - 1.2359 | 1.2359 | PR probability = 1/[1 + exp( 1.5237 - Parameter : 4OVAL - Parameter : BENZA - Parameter : LYS)] |
| | | BENZA | $1.367 \times 10^{-1}$ | - 1.1105 | 1.1105 | |
| | | LYS | 4.9765 | - 0.8767 | 0.8767 | |
| | PD prediction model | 3IND | $6.129 \times 10^{-2}$ | - 1.4853 | 1.4853 | PD probability = 1/[1 + exp ( 2.4054 + Parameter : 3IND + Parameter : 5A4CR + Parameter : CSSG + Parameter : GABB + Parameter : TMNO)] |
| | | 5A4CR | $2.052 \times 10^{-2}$ | 1.0356 | - 1.0356 | |
| | | CSSG | $1.273 \times 10^{-2}$ | - 1.1004 | 1.1004 | |
| | | GABB | $5.117 \times 10^{-2}$ | 1.2343 | - 1.2343 | |
| | | TMNO | $2.689 \times 10^{-1}$ | - 0.9992 | 0.9992 | |

The PR prediction model is as shown in the following expression (6):

$$p = \frac{1}{1+e^{(1.5237-(4OVAL)-(BENZA)-(LYS))}} \qquad (6)$$

wherein 4OVAL represents 1.2359 when a measurement result about 4OVAL is equal to or more than a cut-off value, and represents -1.2359 when the measurement result is less than the cut-off value; BENZA represents 1.1105 when a measurement result about BENZA is equal to or more than a cut-off value, and represents -1.1105 when the measurement result is less than the cut-off value; and LYS represents 0.8767 when a measurement result about LYS is equal to or more than a cut-off value, and represents -0.8767 when the measurement result is less than the cut-off value.

The model is an expression for determining whether or not a patient has PR after continuing the third or later cycles of treatment. The p value represents a probability that the patient has PR after continuing the third or later cycles of treatment. When the p value exceeds 0.5, it is determined that PR appears.

AUC of the ROC curve of this PR prediction model was 0.91 (Figure 12c). The PR predictive value, sensitivity, specificity, and accuracy thereof were 66.7%, 22.2%, 98.5%, and 89.6%, respectively, as shown in Table 18.

The PD prediction model is as shown in the following expression (7):

$$p = \frac{1}{1+e^{(2.4054+(3IND)+(5A4CR)+(CSSG)+(GABB)+(TMNO))}} \qquad (7)$$

wherein 3IND represents 1.4853 when a measurement result about 3IND is equal to or more than a cut-off value, and represents -1.4853 when the measurement result is less than the cut-off value; 5A4CR represents -1.0356 when a measurement result about 5A4CR is equal to or more than a cut-off value, and represents 1.0356 when the measurement result is less than the cut-off value; CSSG represents 1.1004 when a measurement result about CSSG is equal to or more than a cut-off value, and represents -1.1004 when the measurement result is less than the cut-off value; GABB represents -1.2343 when a measurement result about GABB is equal to or more than a cut-off value, and represents 1.2343 when the measurement result is less than the cut-off value; and TMNO represents 0.9992 when a measurement result about TMNO is equal to or more than a cut-off value, and represents -0.9992 when the measurement result is less than the cut-off value.

The model is an expression for determining whether or not a patient has PD after continuing the third or later cycles of treatment. The p value represents a probability that the patient has PD after continuing the third or later cycles of treatment. When the p value exceeds 0.5, it is determined that PD appears.

AUC of the ROC curve of this PD prediction model was 0.92 (Figure 12d). The PD predictive value, sensitivity, specificity, and accuracy thereof were 82.4%, 94.7%, 70.0%, and 88.3%, respectively, as shown in Table 18.

[Table 18]

| | | Predicted | | | | Predictive value (%) | Sensitivity (%) | Specificity (%) | Accuracy (%) |
|---|---|---|---|---|---|---|---|---|---|
| | | PR | SD | PD | Total | | | | |
| Cycle 2 Actual | PR | 2 | 7 | | 9 | 66.7 | 22.2 | 98.5 | 89.6 |
| | SD | 1 | 44 | 3 | 48 | | | | |
| | PD | | 6 | 14 | 20 | 82.4 | 94.7 | 70.0 | 88.3 |
| | Total | 3 | 57 | 17 | 77 | | | | |

Among patients determined to have PR by the PR prediction model, there were no patients who actually had PD. In addition, among patients determined to have PD by the PD prediction model, there were no patients who actually had PR. This fact also showed high accuracy of these prediction models.

(c) Residual OS prediction performance of PR prediction model and PD prediction model

On the basis of the PR prediction models of (b), patients were classified into a PR group (group determined to have PR by the PR prediction models) and the other group (group determined to have no PR by the PR prediction models (SD or PD group)), and a Kaplan-Meier curve was drawn to conduct the log-rank test. The results are shown in Figures 13a and 13c. The prognosis of the PR group (median: incalculable) tended to have a longer survival period than that of the SD or PD group (median: 441 days at cycle 1 and 438 days at cycle 2). In this context, the survival period refers to a residual overall survival (OS) when the day of implementation of each treatment cycle was defined as day 0.

On the basis of the PD prediction models of (b), patients were classified into a PD group (group determined to have PD by the PD prediction models) and the other group (group determined to have no PD by the PD prediction models (PR or SD group)), and a Kaplan-Meier curve was drawn to conduct the log-rank test. The results are shown in Figures 13b and 13d. The prognosis of the PD group (median: 267 days at cycle 1 and 262.5 days at cycle 2) had a significantly (p = 0.0001 at cycle 1 and p = 0.0033 at cycle 2) shorter survival period than that of the PR or SD group (median: 540 days at cycle 1 and 477 days at cycle 2).

These results demonstrate the usefulness of the PR prediction models and the PD prediction models.

(d) Analysis of residual OS using COX proportional hazard model

Substances for predicting prognosis were analyzed using proportional hazard model from blood metabolite concentrations in an early stage after the start of treatment (cycle 1 or 2) and the residual overall survival of each patient when the day of implementation of each treatment cycle was defined as day 0. As a result, the hazard ratios of substances with significance and their 95% confidence intervals are shown in Figure 14. It was found that the higher the blood concentrations of 2H4MP, 3IND, 3MHIS, CHCA, CSSG, GABA and HIPA, the longer the survival period after treatment of cycle 1 while the higher the blood concentrations of 1MNA, ASP, HYPX and N8ASR, the shorter the survival period after treatment of cycle 1. It was further found that the higher the blood concentrations of 2H4MP, 3IND, GABA and MUCA, the longer the survival period after treatment of cycle 2 while the higher the blood concentrations of 5A4CR and TAUR, the shorter the survival period after treatment of cycle 2. CSSG overlapped between these substances significant using the COX proportional hazard model and the markers for predicting PR in (a-1). CSSG had behavior consistent with the results of (a-1), as shown in Table 19, demonstrating that this substance is particularly useful as a marker for predicting prognosis.

[Table 19]

| Substance for predicting prognosis | Behavior in (a-1) | Behavior in (d) |
|---|---|---|
| CSSG | Blood concentration was higher in the PR group than in the SD or PD group. | The higher the blood concentration, the longer the survival period. |

Likewise, N8ASR overlapped between the substances significant using the COX proportional hazard model and the markers for predicting PD in (a-2). N8ASR had behavior consistent with the results of (a-2), as shown in Table 20, demonstrating that this substance is particularly useful as a marker for predicting prognosis.

[Table 20]

| Substance for predicting prognosis | Behavior in (a-2) | Behavior in (d) |
|---|---|---|
| N8ASR | Blood concentration was higher in the PD group than in the PR or SD group. | The higher the blood concentration, the shorter the survival period. |

Also, 3IND and 5A4CR overlapped between the substances significant using the COX proportional hazard model and the markers for predicting PD in (a-4). 3IND and 5A4CR had behavior consistent with the results of (a-4), as shown in Table 21, demonstrating that these substances are particularly useful as markers for predicting prognosis.

[Table 21]

| Substance for predicting prognosis | Behavior in (a-4) | Behavior in (d) |
|---|---|---|
| 3IND | Blood concentration was higher in the PR or SD group than in the PD group. | The higher the blood concentration, the longer the survival period. |

(continued)

| Substance for predicting prognosis | Behavior in (a-4) | Behavior in (d) |
|---|---|---|
| N8ASR | Blood concentration was higher in the PD group than in the PR or SD group | The higher the blood concentration, the shorter the survival period. |

**Claims**

1. A marker for determining sensitivity to an anti-cancer agent, the anti-cancer agent including irinotecan or SN-38 or a salt thereof, fluorouracil or a salt thereof, and levofolinate or a salt thereof, the marker comprising one or more molecules selected from the group consisting of 5-aminoimidazole-4-carboxamide ribotide (5A4CR), alanine (ALA), aspartic acid (ASP), cysteine (CYS), cysteine-glutathione disulphide (CSSG), glycerol-3-phosphate (GLC3P), histidine (HIS), isoleucine (ILE), leucine (LEU), lysine (LYS), methionine sulfoxide (METSF), N6,N6,N6-trimethyllysine (N6TLY), N6-acetyllysine (N6ALY), octanoic acid (OCTA), serine (SER), taurocholic acid (TUCA), threonine (THR), tryptophan (TRP), tyrosine (TYR) and valine (VAL).

2. The marker for determining anti-cancer agent sensitivity according to claim 1, wherein the anti-cancer agent further includes an anti-angiogenic drug.

3. The marker for determining anti-cancer agent sensitivity according to claim 2, wherein the anti-angiogenic drug is bevacizumab.

4. A method for determining sensitivity to an anti-cancer agent, the anti-cancer agent including irinotecan or SN-38 or a salt thereof, fluorouracil or a salt thereof, and levofolinate or a salt thereof, the method comprising measuring an amount of one or more molecules selected from the group consisting of 5A4CR, ALA, ASP, CYS, CSSG, GLC3P, HIS, ILE, LEU, LYS, METSF, N6TLY, N6ALY, OCTA, SER, TUCA, THR, TRP, TYR and VAL in a biological sample derived from a cancer patient.

5. The determination method according to claim 4, further comprising determining the sensitivity of the cancer patient to the anti-cancer agent by comparing a measurement result with a control level.

6. The determination method according to claim 4, wherein the amount of one or more molecules selected from the group consisting of ALA, CYS, CSSG, HIS, ILE, LEU, LYS, METSF, N6TLY, SER, THR, TRP, TYR and VAL is measured, and the method further comprises determining whether or not the cancer patient has PR after treatment with the anti-cancer agent, by comparing a measurement result with a cut-off value of PR, wherein the cut-off value is $9.957 \leq$ for ALA, $2.444 \times 10^{-1} \leq$ for CYS, $1.430 \times 10^{-2} \leq$ for CSSG, $2.2409 \leq$ for HIS, $2.997 \leq$ for ILE, $7.437 \leq$ for LEU, $4.945 \leq$ for LYS, $6.658 \times 10^{-2} \leq$ for METSF, $8.401 \times 10^{-2} \leq$ for N6TLY, $2.200 \leq$ for SER, $2.753 \leq$ for THR, $2.165 \leq$ for TRP, $2.084 \leq$ for TYR, and $8.317 \leq$ for VAL.

7. The determination method according to claim 4, wherein the amount of one or more molecules selected from the group consisting of 5A4CR, ALA, ASP, CSSG, GLC3P, HIS, ILE, LEU, N6TLY, N6ALY, OCTA, TUCA and THR is measured, and the method further comprises determining whether or not the cancer patient has PD after treatment with the anti-cancer agent, by comparing a measurement result with a cut-off value of PD, wherein the cut-off value is $1.421 \times 10^{-2} \leq$ for 5A4CR, $< 6.494$ for ALA, $0.1433 \leq$ for ASP, $< 8.630 \times 10^{-3}$ for CSSG, $< 6.009 \times 10^{-3}$ for GLC3P, $< 2.366$ for HIS, $< 2.748$ for ILE, $< 6.413$ for LEU, $< 8.030 \times 10^{-2}$ for N6TLY, $2.915 \times 10^{-2} \leq$ for N6ALY, $< 6.767 \times 10^{-2}$ for OCTA, $2.380 \times 10^{-3} \leq$ for TUCA, and $< 2.137$ for THR.

8. The determination method according to claim 4, further comprising determining whether or not the cancer patient has PR after treatment with the anti-cancer agent, by calculating a probability (p) of PR according to the following expression (1):

$$p = \frac{1}{1+e^{(4.2504-(ALA)-(CSSG))}} \qquad (1)$$

wherein ALA represents 2.5043 when a measurement result about ALA is equal to or more than a cut-off value, and represents 0 when the measurement result is less than the cut-off value; CSSG represents 2.4626 when a measurement result about CSSG is equal to or more than a cut-off value, and represents 0 when the measurement result is less than the cut-off value; and the cut-off value is 9.957 for ALA and $1.430 \times 10^{-2}$ for CSSG.

9. The determination method according to claim 4, further comprising determining whether or not the cancer patient has PD after treatment with the anti-cancer agent, by calculating a probability (p) of PD according to the following expression (2):

$$p = \frac{1}{1+e^{(0.8105+(ASP)+(HIS)+(N6ALY)+(TUCA))}} \qquad (2)$$

wherein ASP represents -1.0820 when a measurement result about ASP is equal to or more than a cut-off value, and represents 1.0820 when the measurement result is less than the cut-off value; HIS represents 1.7717 when a measurement result about HIS is equal to or more than a cut-off value, and represents -1.7717 when the measurement result is less than the cut-off value; N6ALY represents -1.5499 when a measurement result about N6ALY is equal to or more than a cut-off value, and represents 1.5499 when the measurement result is less than the cut-off value; TUCA represents -0.7905 when a measurement result about TUCA is equal to or more than a cut-off value, and represents 0.7905 when the measurement result is less than the cut-off value; and the cut-off value is 0.1433 for ASP, 2.366 for HIS, $2.915 \times 10^{-2}$ for N6ALY, and $2.380 \times 10^{-3}$ for TUCA.

10. The determination method according to any one of claims 4 to 9, wherein the biological sample is a biological sample derived from a cancer patient to whom the anti-cancer agent has been administered.

11. The determination method according to any one of claims 4 to 10, wherein the anti-cancer agent further includes an anti-angiogenic drug.

12. The determination method according to claim 11, wherein the anti-angiogenic drug is bevacizumab.

13. A method for determining a total tumor size of a cancer patient, comprising measuring an amount of CSSG in a biological sample derived from the cancer patient.

14. A marker for predicting prognosis for treatment with an anti-cancer agent, the anti-cancer agent including irinotecan or SN-38 or a salt thereof, fluorouracil or a salt thereof, and levofolinate or a salt thereof, the marker comprising one or more molecules selected from the group consisting of 3-indoxylsulfuric acid (3IND), ALA, ASP, citrulline (CITR), creatine (CREAT), CSSG, gamma-aminobutyric acid (GABA), guanidoacetic acid (GUAA), HIS, hydroxyproline (HYPRO), METSF, N6TLY, N8-acetylspermidine (N8ASR) and SER.

15. The marker for predicting prognosis according to claim 14, wherein the anti-cancer agent further includes an anti-angiogenic drug.

16. The marker for predicting prognosis according to claim 15, wherein the anti-angiogenic drug is bevacizumab.

17. A method for predicting prognosis for treatment with an anti-cancer agent, the anti-cancer agent including irinotecan or SN-38 or a salt thereof, fluorouracil or a salt thereof, and levofolinate or a salt thereof, the method comprising measuring an amount of one or more molecules selected from the group consisting of 3IND, ALA, ASP, CITR, CREAT, CSSG, GABA, GUAA, HIS, HYPRO, METSF, N6TLY, N8ASR and SER in a biological sample derived from a cancer patient.

18. The method for predicting prognosis according to claim 17, wherein the anti-cancer agent further includes an anti-angiogenic drug.

19. The method for predicting prognosis according to claim 18, wherein the anti-angiogenic drug is bevacizumab.

20. A kit for carrying out a determination method according to any one of claims 4 to 13, the kit comprising a protocol for measuring an amount of one or more molecules selected from the group consisting of 5A4CR, ALA, ASP, CYS, CSSG, GLC3P, HIS, ILE, LEU, LYS, METSF, N6TLY, N6ALY, OCTA, SER, TUCA, THR, TRP, TYR and VAL in a biological sample derived from a cancer patient.

21. A kit for carrying out a method for predicting prognosis according to any one of claims 17 to 19, the kit comprising a protocol for measuring an amount of one or more molecules selected from the group consisting of 3IND, ALA, ASP, CITR, CREAT, CSSG, GABA, GUAA, HIS, HYPRO, METSF, N6TLY, N8ASR and SER in a biological sample derived from a cancer patient.

22. A screening method for an anti-cancer agent sensitivity-enhancing agent, the method comprising employing, as an index, expression variation of one or more molecules selected from the group consisting of 5A4CR, ALA, ASP, CYS, CSSG, GLC3P, HIS, ILE, LEU, LYS, METSF, N6TLY, N6ALY, OCTA, SER, TUCA, THR, TRP, TYR, VAL, 3IND, CITR, CREAT, GABA, GUAA, HYPRO and N8ASR in a biological sample derived from a cancer cell line or a cancer-bearing animal in the presence of an anti-cancer agent including irinotecan or SN-38 or a salt thereof, fluorouracil or a salt thereof, and levofolinate or a salt thereof.

23. The screening method according to claim 22, wherein the anti-cancer agent further includes an anti-angiogenic drug.

24. The screening method according to claim 23, wherein the anti-angiogenic drug is bevacizumab.

25. A sensitivity-enhancing agent for an anti-cancer agent including irinotecan or SN-38 or a salt thereof, fluorouracil or a salt thereof, and levofolinate or a salt thereof, which is obtained by a method according to any one of claims 22 to 24.

26. A composition for cancer treatment, the composition comprising a sensitivity-enhancing agent according to claim 25 in combination with an anti-cancer agent including irinotecan or SN-38 or a salt thereof, fluorouracil or a salt thereof, and levofolinate or a salt thereof.

27. The composition for cancer treatment according to claim 26, wherein the anti-cancer agent further includes an anti-angiogenic drug.

28. The composition for cancer treatment according to claim 27, wherein the anti-angiogenic drug is bevacizumab.

29. A marker for determining sensitivity to an anti-cancer agent, the anti-cancer agent including irinotecan or SN-38 or a salt thereof, fluorouracil or a salt thereof, and levofolinate or a salt thereof, the marker comprising one or more molecules selected from the group consisting of 3IND, 4-oxavaleric acid (4OVAL), 5A4CR, ALA, benzoic acid (BENZA), CREAT, CSSG, decanoic acid (DECNA), gamma-butyrobetaine (GABB), GLC3P, hypotaurine (HYPTA), LYS, METSF, N8ASR, quinic acid (QUINA), sarcosine (SARCO), trimethylamine N-oxide (TMNO) and VAL.

30. The marker for determining anti-cancer agent sensitivity according to claim 29, wherein the anti-cancer agent further includes an anti-angiogenic drug.

31. The marker for determining anti-cancer agent sensitivity according to claim 30, wherein the anti-angiogenic drug is bevacizumab.

32. A method for determining sensitivity to an anti-cancer agent, the anti-cancer agent including irinotecan or SN-38 or a salt thereof, fluorouracil or a salt thereof, and levofolinate or a salt thereof, the method comprising measuring an amount of one or more molecules selected from the group consisting of 3IND, 4OVAL, 5A4CR, ALA, BENZA, CREAT, CSSG, DECNA, GABB, GLC3P, HYPTA, LYS, METSF, N8ASR, QUINA, SARCO, TMNO and VAL in a biological sample derived from a cancer patient who has received at least one cycle of treatment with the anti-cancer agent including irinotecan or SN-38 or a salt thereof, fluorouracil or a salt thereof, and levofolinate or a salt thereof.

33. The determination method according to claim 32, further comprising determining the sensitivity of the cancer patient to the anti-cancer agent by comparing a measurement result with a control level.

34. The determination method according to claim 32, wherein the cancer patient is a patient who has received one cycle of treatment with the anti-cancer agent, the amount of one or more molecules selected from the group consisting of CREAT, CSSG, METSF, QUINA and VAL is measured, and the method further comprises determining whether or not the cancer patient has PR after treatment with the anti-cancer agent, by comparing a measurement result with a cut-off value of PR, wherein the cut-off value is $1.2163 \leq$ for CREAT, $1.965 \times 10^{-2} \leq$ for CSSG, $5.060 \times 10^{-2}$

≤ for METSF, < 1.150 × 10⁻² for QUINA, and 7.6718 ≤ for VAL.

**35.** The determination method according to claim 32, wherein the cancer patient is a patient who has received one cycle of treatment with the anti-cancer agent, the amount of one or more molecules selected from the group consisting of 5A4CR, CSSG, DECNA, GLC3P, HYPTA and N8ASR is measured, and the method further comprises determining whether or not the cancer patient has PD after treatment with the anti-cancer agent, by comparing a measurement result with a cut-off value of PD, wherein the cut-off value is $1.413 \times 10^{-2} \leq$ for 5A4CR, $< 1.030 \times 10^{-2}$ for CSSG, $< 1.080 \times 10^{-1}$ for DECNA, $< 4.586 \times 10^{-1}$ for GLC3P, $< 1.240 \times 10^{-2}$ for HYPTA, and $1.122 \times 10^{-2} \leq$ for N8ASR.

**36.** The determination method according to claim 32, wherein the cancer patient is a patient who has received two cycles of treatment with the anti-cancer agent, the amount of one or more molecules selected from the group consisting of 4OVAL, ALA, BENZA, CREAT, CSSG, LYS and SARCO is measured, and the method further comprises determining whether or not the cancer patient has PR after treatment with the anti-cancer agent, by comparing a measurement result with a cut-off value of PR, wherein the cut-off value is $2.949 \times 10^{-2} \leq$ for 4OVAL, $7.9605 \leq$ for ALA, $1.367 \times 10^{-1} \leq$ for BENZA, $6.609 \times 10^{-1} \leq$ for CREAT, $1.233 \times 10^{-2} \leq$ for CSSG, $4.9765 \leq$ for LYS, and $4.548 \times 10^{-2} \leq$ for SARCO.

**37.** The determination method according to claim 32, wherein the cancer patient is a patient who has received two cycles of treatment with the anti-cancer agent, the amount of one or more molecules selected from the group consisting of 3IND, 4OVAL, 5A4CR, ALA, CSSG, GABB and TMNO is measured, and the method further comprises determining whether or not the cancer patient has PD after treatment with the anti-cancer agent, by comparing a measurement result with a cut-off value of PD, wherein the cut-off value is $< 6.129 \times 10^{-2}$ for 3IND, $< 1.346 \times 10^{-2}$ for 4OVAL, $2.052 \times 10^{-2} \leq$ for 5A4CR, $< 7.3693$ for ALA, $< 1.273 \times 10^{-2}$ for CSSG, $5.117 \times 10^{-2} \leq$ for GABB, and $< 2.689 \times 10^{-1}$ for TMNO.

**38.** The determination method according to claim 32, wherein the cancer patient is a patient who has received one cycle of treatment with the anti-cancer agent, and the method further comprises determining whether or not the cancer patient has PR after treatment with the anti-cancer agent, by calculating a probability (p) of PR according to the following expression (4):

$$p = \frac{1}{1+e^{(9.0171-(CREAT)-(CSSG)-(QUINA))}} \qquad (4)$$

wherein CREAT represents 1.2906 when a measurement result about CREAT is equal to or more than a cut-off value, and represents -1.2906 when the measurement result is less than the cut-off value; CSSG represents 1.7703 when a measurement result about CSSG is equal to or more than a cut-off value, and represents -1.7703 when the measurement result is less than the cut-off value; QUINA represents -8.6990 when a measurement result about QUINA is equal to or more than a cut-off value, and represents 8.6990 when the measurement result is less than the cut-off value; and the cut-off value is 1.2163 for CREAT, $1.965 \times 10^{-2}$ for CSSG, and $1.150 \times 10^{-2}$ for QUINA.

**39.** The determination method according to claim 32, wherein the cancer patient is a patient who has received one cycle of treatment with the anti-cancer agent, and the method further comprises determining whether or not the cancer patient has PD after treatment with the anti-cancer agent, by calculating a probability (p) of PD according to the following expression (5):

$$p = \frac{1}{1+e^{(0.8232+(5A4CR)+(CSSG)+(DECNA)+(HYPTA)+(N8ASR))}} \qquad (5)$$

wherein 5A4CR represents -0.9300 when a measurement result about 5A4CR is equal to or more than a cut-off value, and represents 0.9300 when the measurement result is less than the cut-off value; CSSG represents 1.2325 when a measurement result about CSSG is equal to or more than a cut-off value, and represents -1.2325 when the measurement result is less than the cut-off value; DECNA represents 1.3052 when a measurement result about DECNA is equal to or more than a cut-off value, and represents -1.3052 when the measurement result is less than the cut-off value; HYPTA represents 0.8020 when a measurement result about HYPTA is equal to or more than a cut-off value, and represents -0.8020 when the measurement result is less than the cut-off value; N8ASR represents -1.4363 when a measurement result about N8ASR is equal to or more than a cut-off value, and represents 1.4363 when the measurement result is less than the cut-off value; and the cut-off value is 1.413 × 10$^{-2}$ for 5A4CR, 1.030 × 10$^{-2}$ for CSSG, 1.080 × 10$^{-1}$ for DECNA, 1.240 × 10$^{-2}$ for HYPTA, and 1.122 × 10$^{-2}$ for N8ASR.

**40.** The determination method according to claim 32, wherein the cancer patient is a patient who has received two cycles of treatment with the anti-cancer agent, and the method further comprises determining whether or not the cancer patient has PR after treatment with the anti-cancer agent, by calculating a probability (p) of PR according to the following expression (6):

$$p = \frac{1}{1+e^{(1.5237-(4OVAL)-(BENZA)-(LYS))}} \qquad (6)$$

wherein 4OVAL represents 1.2359 when a measurement result about 4OVAL is equal to or more than a cut-off value, and represents -1.2359 when the measurement result is less than the cut-off value; BENZA represents 1.1105 when a measurement result about BENZA is equal to or more than a cut-off value, and represents -1.1105 when the measurement result is less than the cut-off value; LYS represents 0.8767 when a measurement result about LYS is equal to or more than a cut-off value, and represents - 0.8767 when the measurement result is less than the cut-off value; and the cut-off value is 2.949 × 10$^{-2}$ for 4OVAL, 1.367 × 10$^{-1}$ for BENZA, and 4.9765 for LYS.

**41.** The determination method according to claim 32, wherein the cancer patient is a patient who has received two cycles of treatment with the anti-cancer agent, and the method further comprises determining whether or not the cancer patient has PD after treatment with the anti-cancer agent, by calculating a probability (p) of PD according to the following expression (7):

$$p = \frac{1}{1+e^{(2.4054+(3IND)+(5A4CR)+(CSSG)+(GABB)+(TMNO))}} \qquad (7)$$

wherein 3IND represents 1.4853 when a measurement result about 3IND is equal to or more than a cut-off value, and represents -1.4853 when the measurement result is less than the cut-off value; 5A4CR represents -1.0356 when a measurement result about 5A4CR is equal to or more than a cut-off value, and represents 1.0356 when the measurement result is less than the cut-off value; CSSG represents 1.1004 when a measurement result about CSSG is equal to or more than a cut-off value, and represents -1.1004 when the measurement result is less than the cut-off value; GABB represents -1.2343 when a measurement result about GABB is equal to or more than a cut-off value, and represents 1.2343 when the measurement result is less than the cut-off value; TMNO represents 0.9992 when a measurement result about TMNO is equal to or more than a cut-off value, and represents -0.9992 when the measurement result is less than the cut-off value; and the cut-off value is 6.129 × 10$^{-2}$ for 3IND, 2.052 × 10$^{-2}$ for 5A4CR, 1.273 × 10$^{-2}$ for CSSG, 5.117 × 10$^{-2}$ for GABB, and 2.689 × 10$^{-1}$ for TMNO.

**42.** The determination method according to any one of claims 32 to 41, wherein the anti-cancer agent further includes an anti-angiogenic drug.

**43.** The determination method according to claim 42, wherein the anti-angiogenic drug is bevacizumab.

**44.** A marker for predicting prognosis for treatment with an anti-cancer agent, the anti-cancer agent including irinotecan or SN-38 or a salt thereof, fluorouracil or a salt thereof, and levofolinate or a salt thereof, the marker comprising one or more molecules selected from the group consisting of 1-methylnicotinamide (1MNA), 2-hydroxy-4-methylpentanoic acid (2H4MP), 3IND, 3-methylhistidine (3MHIS), 5A4CR, ASP, cyclohexanecarboxylic acid (CHCA), CSSG, GABA, hippuric acid (HIPA), hypoxanthine (HYPX), mucic acid (MUCA), N8ASR and taurine (TAUR).

**45.** The marker for predicting prognosis according to claim 44, wherein the anti-cancer agent further includes an anti- angiogenic drug.

**46.** The marker for predicting prognosis according to claim 45, wherein the anti-angiogenic drug is bevacizumab.

**47.** A method for predicting prognosis for treatment with an anti-cancer agent, the anti-cancer agent including irinotecan or SN-38 or a salt thereof, fluorouracil or a salt thereof, and levofolinate or a salt thereof, the method comprising measuring an amount of one or more molecules selected from the group consisting of 1MNA, 2H4MP, 3IND, 3MHIS, 5A4CR, ASP, CHCA, CSSG, GABA, HIPA, HYPX, MUCA, N8ASR and TAUR in a biological sample derived from a cancer patient who has received at least one cycle of treatment with the anti-cancer agent including irinotecan or SN-38 or a salt thereof, fluorouracil or a salt thereof, and levofolinate or a salt thereof.

48. The method for predicting prognosis according to claim 47, wherein the anti-cancer agent further includes an anti-angiogenic drug.

49. The method for predicting prognosis according to claim 48, wherein the anti-angiogenic drug is bevacizumab.

50. A kit for carrying out a determination method according to any one of claims 32 to 43, the kit comprising a protocol for measuring an amount of one or more molecules selected from the group consisting of 3IND, 4OVAL, 5A4CR, ALA, BENZA, CREAT, CSSG, DECNA, GABB, GLC3P, HYPTA, LYS, METSF, N8ASR, QUINA, SARCO, TMNO and VAL in a biological sample derived from a cancer patient who has received at least one cycle of treatment with the anti-cancer agent including irinotecan or SN-38 or a salt thereof, fluorouracil or a salt thereof, and levofolinate or a salt thereof.

51. A kit for carrying out a method for predicting prognosis according to any one of claims 47 to 49, the kit comprising a protocol for measuring an amount of one or more molecules selected from the group consisting of 1MNA, 2H4MP, 3IND, 3MHIS, 5A4CR, ASP, CHCA, CSSG, GABA, HIPA, HYPX, MUCA, N8ASR and TAUR in a biological sample derived from a cancer patient who has received at least one cycle of treatment with the anti-cancer agent including irinotecan or SN-38 or a salt thereof, fluorouracil or a salt thereof, and levofolinate or a salt thereof.

[Figure 1]

[Figure 2]

[Figure 3]

a. ROC curve of PR prediction model

b. ROC curve of PD prediction model

[Figure 4]

a. Predicted PR patients

| Group | N | Median OS | p (log rank) |
|---|---|---|---|
| 1: SD or PD | 75 | 441 days | 0.0443 |
| 2: PR | 7 | Not reached | |

b. Predicted PD patients

| Group | N | Median OS | p (log rank) |
|---|---|---|---|
| 1: PD | 20 | 262.5 days | 0.0008 |
| 2: PR or SD | 62 | 517 days | |

[Figure 5]

[Figure 6]

[Figure 7]

[Figure 8]

[Figure 9]

[Figure 10]

[Figure 11]

[Figure 12]

a. ROC curve of PR prediction model of cycle 1

True positive Sensitivity / 1-Specificity False positive

ROC AUC 0.96

b. ROC curve of PD prediction model of cycle 1

True positive Sensitivity / 1-Specificity False positive

ROC AUC 0.91

c. ROC curve of PR prediction model of cycle 2

True positive Sensitivity / 1-Specificity False positive

ROC AUC 0.91

d. ROC curve of PD prediction model of cycle 2

True positive Sensitivity / 1-Specificity False positive

ROC AUC 0.92

[Figure 13]

a. Predicted PR patients of Cycle 1

| Group | Median Time | Hazard ratio (95% CI) |
|---|---|---|
| —— PR | . | 0.3998 |
| ‑ ‑ ‑ ‑ SD + PD | 441 | 0.1209 - 0.9774 |

b. Predicted PD patients of Cycle 1

| Group | Median Time | Hazard ratio (95% CI) |
|---|---|---|
| —— PR+SD | 540 | 0.3641 |
| ‑ ‑ ‑ ‑ PD | 267 | 0.2137 - 0.6370 |

c. Predicted PR patients of Cycle 2

| Group | Median Time | Hazard比 (95% CI) |
|---|---|---|
| —— PR | . | 0.3135 |
| ‑ ‑ ‑ ‑ SD + PD | 438 | 0.01773- 1.4267 |

d. Predicted PD patients of Cycle 2

| Group | Median Time | Hazard比 (95% CI) |
|---|---|---|
| —— PR+SD | 477 | 0.4083 |
| ‑ ‑ ‑ ‑ PD | 262.5 | 0.2259 - 0.7828 |

[Figure 14]

(a) Residual OS Hazard ratio (cycle 1)

(b) Residual OS Hazard ratio (cycle 2)

## INTERNATIONAL SEARCH REPORT

| | International application No. |
|---|---|
| | PCT/JP2019/010963 |

A.  CLASSIFICATION OF SUBJECT MATTER
Int.Cl.  G01N33/68(2006.01)i, A61K31/4745(2006.01)i,
          A61K31/513(2006.01)i, A61K31/519(2006.01)i,
          A61K39/395(2006.01)i, A61K45/00(2006.01)i, A61P35/00(2006.01)i,
          A61P43/00(2006.01)i, G01N33/15(2006.01)i, G01N33/50(2006.01)i
According to International Patent Classification (IPC) or to both national classification and IPC

B.  FIELDS SEARCHED

Minimum documentation searched (classification system followed by classification symbols)
Int.Cl.  G01N33/68, A61K31/4745, A61K31/513, A61K31/519, A61K39/395,
          A61K45/00, A61P35/00, A61P43/00, G01N33/15, G01N33/50

Documentation searched other than minimum documentation to the extent that such documents are included in the fields searched
| | |
|---|---|
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)
JSTPlus/JMEDPlus/JST7580 (JDreamIII), CAplus/MEDLINE/BIOSIS (STN)

C.  DOCUMENTS CONSIDERED TO BE RELEVANT

| Category* | Citation of document, with indication, where appropriate, of the relevant passages | Relevant to claim No. |
|---|---|---|
| A | 硲彰一ほか，新しい遺伝子多型解析による大腸癌化学療法 (FOLFIRI)の効果と毒性予測システムの開発，日本消化器外科学会雑誌 01 July 2009, vol. 42, no. 7, pp. 955, PD-2-8, non-official translation (HAZAMA, Shoichi et al., "Effects of colorectal cancer chemotherapy (FOLFIRI) by new genetic polymorphism analysis, and development of toxic prediction system", The Japanese Journal of Gastroenterological Surgery) | 1-12, 20, 22-24, 29-43 (parts) |
| A | HARA, Masayasu et al., "High serum levels of inter leukin-6 in patients with advanced or metastatic colorectal cancer: the effect on the outcome and the response to chemotherapy plus bevacizumab", Surgery Today, April 2017, vol. 47, no. 4, pp. 483-489 | 1-12, 20, 22-24, 29-43 (parts) |

☒  Further documents are listed in the continuation of Box C.    ☐  See patent family annex.

| | |
|---|---|
| *  Special categories of cited documents: | "T"  later document published after the international filing date or priority date and not in conflict with the application but cited to understand the principle or theory underlying the invention |
| "A"  document defining the general state of the art which is not considered to be of particular relevance | |
| "E"  earlier application or patent but published on or after the international filing date | "X"  document of particular relevance; the claimed invention cannot be considered novel or cannot be considered to involve an inventive step when the document is taken alone |
| "L"  document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O"  document referring to an oral disclosure, use, exhibition or other means | |
| "P"  document published prior to the international filing date but later than the priority date claimed | "&"  document member of the same patent family |

| Date of the actual completion of the international search | Date of mailing of the international search report |
|---|---|
| 07 June 2019 (07.06.2019) | 18 June 2019 (18.06.2019) |

| Name and mailing address of the ISA/ | Authorized officer |
|---|---|
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

International application No.

PCT/JP2019/010963

C (Continuation).   DOCUMENTS CONSIDERED TO BE RELEVANT

| Category* | Citation of document, with indication, where appropriate, of the relevant passages | Relevant to claim No. |
|---|---|---|
| A | WO 2016/031816 A1 (KEIO GIJUKU) 03 March 2016, claims 1, 3 & US 2017/0248578 A1 & EP 3187878 A1 & CN 106605147 A | 1-12, 20, 22-24, 29-43 (parts) |
| A | WO 2009/096189 A1 (KEIO GIJUKU) 06 August 2009, claims 1, 4 & US 2011/0003842 A1 & EP 2237042 A1 & AU 2009208519 A & CA 2712966 A1 & CN 101932939 A | 1-12, 20, 22-24, 29-43 (parts) |

Form PCT/ISA/210 (continuation of second sheet) (January 2015)

## INTERNATIONAL SEARCH REPORT

| International application No. |
| --- |
| PCT/JP2019/010963 |

**Box No. II**    **Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☒ Claims Nos.: 25-28
   because they relate to parts of the international application that do not comply with the prescribed requirements to such an extent that no meaningful international search can be carried out, specifically:
   Claims 25-28 relate to an agonist sensitive to an anti-cancer agent and a composition for treating cancer, which is made in combination with said agonist and an anti-cancer agent, but the specification does not disclose a specific compound at all with respect to the agonist sensitive to an anti-cancer agent, and it cannot be assumed which one can be an agonist sensitive to an anti-cancer agent, and thus a meaningful international examination cannot be carried out.

3. ☐ Claims Nos.:
   because they are dependent claims and are not drafted in accordance with the second and third sentences of Rule 6.4(a).

**Box No. III**    **Observations where unity of invention is lacking (Continuation of item 3 of first sheet)**

This International Searching Authority found multiple inventions in this international application, as follows:
See extra sheet

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☒ No required additional search fees were timely paid by the applicant.    Consequently, this international search report is restricted to the invention first mentioned in the claims; it is covered by claims Nos.: Claims 1-12, 20, 22-24, 29-43 (parts thereof)

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

**INTERNATIONAL SEARCH REPORT**

International application No.

PCT/JP2019/010963

<Continuation of Box No. III>

The inventions in claims 1-51 are obviously divided into three individual invention groups of an invention with respect to a marker for determining sensitivity of an anti-cancer agent including: irinotecan, SN-38, or a salt thereof; fluorouracil or a salt thereof; and levofolinate or a salt thereof (hereinafter, referring to as "anti-cancer agent"), an invention with respect to a method for determining the total of tumor diameters of a cancer patient, and an invention with respect to a marker for predicting prognosis during treatment using an anti-cancer agent. In addition, the technical feature of a marker for determining sensitivity of an anti-cancer agent and a marker for predicting prognosis during treatment using an anti-cancer agent does not make a contribution over the prior art in light of the disclosures of documents 1 and 2, and thus cannot be considered a special technical feature. Therefore, the claims are classified into 56 inventions respectively having the following special technical features.

(Invention 1) Claims 1-12, 20, 22-24, and 29-43 (parts thereof)
Invention with respect to 5-Aminoimidazole-4-carboxamide ribotide (5A4CR) as a marker for determining sensitivity of an anti-cancer agent.
(Invention 2) Claims 1-12, 20, 22-24, and 29-43 (parts thereof)
Invention with respect to alanine (ALA) as a marker for determining sensitivity of an anti-cancer agent.
(Invention 3) Claims 1-12, 20, and 22-24 (parts thereof)
Invention with respect to aspartic acid (ASP) as a marker for determining sensitivity of an anti-cancer agent.
(Invention 4) Claims 1-12, 20, and 22-24 (parts thereof)
Invention with respect to cysteine (CYS) as a marker for determining sensitivity of an anti-cancer agent.
(Invention 5) Claims 1-12, 20, 22-24, and 29-43 (parts thereof)
Invention with respect to cysteine-glutathione disulphide (CSSG) as a marker for determining sensitivity of an anti-cancer agent.
(Invention 6) Claims 1-12, 20, and 22-24 (parts thereof)
Invention with respect to glycerol-3-phosphate (GLC 3P) as a marker for determining sensitivity of an anti-cancer agent.
(Invention 7) Claims 1-12, 20, and 22-24 (parts thereof)
Invention with respect to histidine (HIS) as a marker for determining sensitivity of an anti-cancer agent.
(Invention 8) Claims 1-12, 20, and 22-24 (parts thereof)
Invention with respect to isoleucine (ILE) as a marker for determining sensitivity of an anti-cancer agent.
(Invention 9) Claims 1-12, 20, and 22-24 (parts thereof)
Invention with respect to leucine (LEU) as a marker for determining sensitivity of an anti-cancer agent.
(Invention 10) Claims 1-12, 20, 22-24, and 29-43 (parts thereof)
Invention with respect to lysine (LYS) as a marker for determining sensitivity of an anti-cancer agent.
(Invention 11) Claims 1-12, 20, 22-24, and 29-43 (parts thereof)
Invention with respect to methionine sulfoxide (METSF) as a marker for determining sensitivity of an anti-cancer agent.
(Invention 12) Claims 1-12, 20, and 22-24 (parts thereof)
Invention with respect to N6, N6, N6-trimethyllysine (N6TLY) as a marker for determining sensitivity of an anti-cancer agent.
(Invention 13) Claims 1-12, 20, and 22-24 (parts thereof)
Invention with respect to N6-acetyllysine (N6ALY) as a marker for determining sensitivity of an anti-cancer agent.
(Invention 14) Claims 1-12, 20, and 22-24 (parts thereof)
Invention with respect to octanoic acid (OCTA) as a marker for determining sensitivity of an anti-cancer agent.

Form PCT/ISA/210 (extra sheet) (January 2015)

**INTERNATIONAL SEARCH REPORT**

International application No.

PCT/JP2019/010963

(Invention 15) Claims 1-12, 20, and 22-24 (parts thereof)
Invention with respect to serine (SER) as a marker for determining sensitivity of an anti-cancer agent.
(Invention 16) Claims 1-12, 20, and 22-24 (parts thereof)
Invention with respect to taurocholic acid (TUCA) as a marker for determining sensitivity of an anti-cancer agent.
(Invention 17) Claims 1-12, 20, and 22-24 (parts thereof)
Invention with respect to threonine (THR) as a marker for determining sensitivity of an anti-cancer agent.
(Invention 18) Claims 1-12, 20, and 22-24 (parts thereof)
Invention with respect to tryptophan (TRP) as a marker for determining sensitivity of an anti-cancer agent.
(Invention 19) Claims 1-12, 20, and 22-24 (parts thereof)
Invention with respect to tyrosine (TYR) as a marker for determining sensitivity of an anti-cancer agent.
(Invention 20) Claims 1-12, 20, 22-24, and 29-43 (parts thereof)
Invention with respect to valine (VAL) as a marker for determining sensitivity of an anti-cancer agent.

(Invention 21) Claims 13 and 20 (parts thereof)
Invention with respect to a method for determining the total of tumor diameters of a cancer patient.

(Invention 22) Claims 14-19, 21, 44-49, and 51 (parts thereof)
Invention with respect to 3-indoxylsulfuric acid (3IND) as a marker for predicting prognosis during treatment using an anti-cancer agent.
(Invention 23) Claims 14-19 and 21 (parts thereof)
Invention with respect to ALA as a marker for predicting prognosis during treatment using an anti-cancer agent.
(Invention 24) Claims 14-19, 21, 44-49, and 51 (parts thereof)
Invention with respect to ASP as a marker for predicting prognosis during treatment using an anti-cancer agent.
(Invention 25) Claims 14-19 and 21 (parts thereof)
Invention with respect to citrulline (CITR) as a marker for predicting prognosis during treatment using an anti-cancer agent.
(Invention 26) Claims 14-19 and 21 (parts thereof)
Invention with respect to creatine (CREAT) as a marker for predicting prognosis during treatment using an anti-cancer agent.
(Invention 27) Claims 14-19, 21, 44-49, and 51 (parts thereof)
Invention with respect to CSSG as a marker for predicting prognosis during treatment using an anti-cancer agent.
(Invention 28) Claims 14-19, 21, 44-49, and 51 (parts thereof)
Invention with respect to gamma-aminobutyric acid (GABA) as a marker for predicting prognosis during treatment using an anti-cancer agent.
(Invention 29) Claims 14-19 and 21 (parts thereof)
Invention with respect to guanidoacetic acid (GUAA) as a marker for predicting prognosis during treatment using an anti-cancer agent.
(Invention 30) Claims 14-19 and 21 (parts thereof)
Invention with respect to HIS as a marker for predicting prognosis during treatment using an anti-cancer agent.
(Invention 31) Claims 14-19 and 21 (parts thereof)
Invention with respect to hydroxyproline (HYPRO) as a marker for predicting prognosis during treatment using an anti-cancer agent.
(Invention 32) Claims 14-19 and 21 (parts thereof)
Invention with respect to METSF as a marker for predicting prognosis during treatment using an anti-cancer agent.
(Invention 33) Claims 14-19 and 21 (parts thereof)
Invention with respect to N6TLY as a marker for predicting prognosis during treatment using an anti-cancer agent.

Form PCT/ISA/210 (extra sheet) (January 2015)

**INTERNATIONAL SEARCH REPORT**

International application No.

PCT/JP2019/010963

(Invention 34) Claims 14-19, 21, 44-49, and 51 (parts thereof)
Invention with respect to N8-acetylspermidine (N8ASR) as a marker for predicting prognosis during treatment using an anti-cancer agent.
(Invention 35) Claims 14-19 and 21 (parts thereof)
Invention with respect to SER as a marker for predicting prognosis during treatment using an anti-cancer agent.
(Invention 36) Claims 29-43 and 50 (parts thereof)
Invention with respect to 3IND as a marker for determining sensitivity of an anti-cancer agent.
(Invention 37) Claims 29-43 and 50 (parts thereof)
Invention with respect to 4-oxavaleric acid (4OVAL) as a marker for determining sensitivity of an anti-cancer agent.
(Invention 38) Claims 29-43 and 50 (parts thereof)
Invention with respect to benzoic acid (BENZA) as a marker for determining sensitivity of an anti-cancer agent.
(Invention 39) Claims 29-43 and 50 (parts thereof)
Invention with respect to CREAT as a marker for determining sensitivity of an anti-cancer agent.
(Invention 40) Claims 29-43 and 50 (parts thereof)
Invention with respect to decanoic acid (DECNA) as a marker for determining sensitivity of an anti-cancer agent.
(Invention 41) Claims 29-43 and 50 (parts thereof)
Invention with respect to gamma-butyrobetaine (GABB) as a marker for determining sensitivity of an anti-cancer agent.
(Invention 42) Claims 29-43 and 50 (parts thereof)
Invention with respect to GLC3P as a marker for determining sensitivity of an anti-cancer agent.
(Invention 43) Claims 29-43 and 50 (parts thereof)
Invention with respect to hypotaurine (HYPTA) as a marker for determining sensitivity of an anti-cancer agent.
(Invention 44) Claims 29-43 and 50 (parts thereof)
Invention with respect to N8ASR as a marker for determining sensitivity of an anti-cancer agent.
(Invention 45) Claims 29-43 and 50 (parts thereof)
Invention with respect to quinic acid (QUINA) as a marker for determining sensitivity of an anti-cancer agent.
(Invention 46) Claims 29-43 and 50 (parts thereof)
Invention with respect to sarcosine (SARCO) as a marker for determining sensitivity of an anti-cancer agent.
(Invention 47) Claims 29-43 and 50 (parts thereof)
Invention with respect to trimethylamine N-oxide (TMNO) as a marker for determining sensitivity of an anti-cancer agent.
(Invention 48) Claims 44-49 and 51 (parts thereof)
Invention with respect to 1-methylnicotinamide (1MNA) as a marker for predicting prognosis during treatment using an anti-cancer agent.
(Invention 49) Claims 44-49 and 51 (parts thereof)
Invention with respect to 2-hydroxy-4-methylpentanoic acid (2H4MP) as a marker for predicting prognosis during treatment using an anti-cancer agent.
(Invention 50) Claims 44-49 and 51 (parts thereof)
Invention with respect to 3-methylhistidine (3MHIS) as a marker for predicting prognosis during treatment using an anti-cancer agent.
(Invention 51) Claims 44-49 and 51 (parts thereof)
Invention with respect to 5A4CR as a marker for predicting prognosis during treatment using an anti-cancer agent.
(Invention 52) Claims 44-49 and 51 (parts thereof)
Invention with respect to cyclohexanecarboxylic acid (CHCA) as a marker for predicting prognosis during treatment using an anti-cancer agent.

Form PCT/ISA/210 (extra sheet) (January 2015)

**INTERNATIONAL SEARCH REPORT**

International application No.

PCT/JP2019/010963

(Invention 53) Claims 44-49 and 51 (parts thereof)
Invention with respect to hippuric acid (HIPA) as a marker for predicting prognosis during treatment using an anti-cancer agent.
(Invention 54) Claims 44-49 and 51 (parts thereof)
Invention with respect to hypoxanthine (HYPX) as a marker for predicting prognosis during treatment using an anti-cancer agent.
(Invention 55) Claims 44-49 and 51 (parts thereof)
Invention with respect to mucic acid (MUCA) as a marker for predicting prognosis during treatment using an anti-cancer agent.
(Invention 56) Claims 44-49 and 51 (parts thereof)
Invention with respect to taurine (TAUR) as a marker for predicting prognosis during treatment using an anti-cancer agent.

Claims 25-28 relate to an agonist sensitive to an anti-cancer agent and a composition for treating cancer, which is made in combination with said agonist and an anti-cancer agent, but the specification does not disclose a specific compound at all with respect to the agonist sensitive to an anti-cancer agent, and it cannot be assumed which one can be an agonist sensitive to an anti-cancer agent, and thus a meaningful international examination cannot be carried out.

Document 1: 硲彰一ほか，新しい遺伝子多型解析による大腸癌化学療法(FOLFIRI)の効果と毒性予測システムの開発， 日本消化器外科学会雑誌 01 July 2009, vol. 42, no. 7, pp. 955, PD-2-8, non-official translation (HAZAMA, Shoichi et al., "Effects of colorectal cancer chemotherapy (FOLFIRI) by new genetic polymorphism analysis, and development of toxic prediction system", The Japanese Journal of Gastroenterological Surgery)
Document 2: HARA, Masayasu et al., "High serum levels of interleukin-6 in patients with advanced or metastatic colorectal cancer: the effect on the outcome and the response to chemotherapy plus bevacizumab", Surgery Today, April 2017, vol. 47, no. 4, pp. 483-489

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009096189 A
- WO 2011052750 A
- WO 2012127984 A
- WO 2013125675 A

**Non-patent literature cited in the description**

- FOLFIRI Followed by FOLFOX6 or the Reverse Sequence. **TOURNIGAND C. et al.** Advanced Colorectal Cancer: A Randamized GERCOR Study. JCO, 2004, vol. 22, 229-37
- **SUENAGA M.** *BMC Cancer,* 2015, vol. 15, 176
- *J Proteome Res.,* September 2003, vol. 2 (5), 488-94
- *Metabolomics,* March 2010, vol. 6 (1), 78-95
- *Metabolomics,* April 2013, vol. 9 (2), 444-453